# EUROPÄISCHE PATENTSCHRIFT

(11) **EP 1 080 086 B1**
(45) Veröffentlichungstag und Bekanntmachung des Hinweises auf die Patenterteilung: **04.09.2002**
(21) Anmeldenummer: 99920603.0
(22) Anmeldetag: 07.04.1999
(51) Int. Cl.: C07D 401/04, C07D 213/78, C07D 213/84

(54) **DIHYDROPYRIMIDINE**
DIHYDROPYRIMIDINES
DIHYDROPYRIMIDINES

(30) Priorität: 18.04.1998 DE 19817264
(43) Veröffentlichungstag der Anmeldung: 07.03.2001
(73) Patentinhaber: BAYER AG, 51368 Leverkusen (DE)
(72) Erfinder: STÖLTING, Jörn, D-51061 Köln (DE); STOLTEFUSS, Jürgen, D-42781 Haan (DE); GOLDMANN, Siegfried, D-42327 Wuppertal (DE); KRÄMER, Thomas, D-42111 Wuppertal (DE); SCHLEMMER, Karl-Heinz, D-42113 Wuppertal (DE); NIEWÖHNER, Ulrich, D-42929 Wermelskirchen (DE); PAESSENS, Arnold, D-42781 Haan (DE); GRAEF, Erwin, D-42553 Velbert (DE); LOTTMANN, Stefan, D-42115 Wuppertal (DE); DERES, Karl, D-53489 Bad Bodendorf (DE); WEBER, Olaf, D-42489 Wülfrath (DE)
(86) Internationale Anmeldenummer: EP9902344
(87) Internationale Veröffentlichungsnummer: WO99054326

(56) Entgegenhaltungen:
- EP-A- 0 103 796
- EP-A- 0 169 712
- WO-A-90/11281
- WO-A-98/21199
- PATENT ABSTRACTS OF JAPAN vol. 10, no. 31 (C-327), 6. Februar 1986 (1986-02-06) & JP 60 185764 A (WAKO JUNYAKU KOGYO KK), 21. September 1985 (1985-09-21)

## Beschreibung

Die vorliegende Erfindung betrifft neue Dihydropyrimidin-Verbindungen, Verfahren zu ihrer Herstellung und ihre Verwendung als Arzneimittel, insbesondere zur Behandlung und Prophylaxe von Hepatitis B.

Aus der Publikation EP 103 796 A2 sind bereits Dihydropyrimidine mit einer kreislaufbeeinflussenden Wirkung bekannt.

Die vorliegende Erfindung betrifft jetzt neue Dihydropyrimidin- Verbindungen der allgemeinen Formel (I) 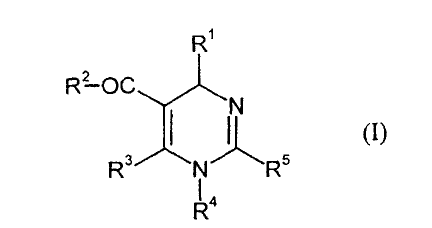 bzw. deren isomere Form (Ia) 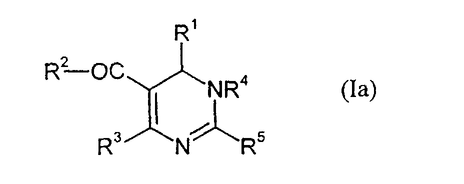 in welcher
- R¹: für Phenyl, Furyl, Thienyl, Triazolyl, Pyridyl, Cycloalkyl mit 3 bis 6 Kohlenstoffatomen oder für Reste der Formeln 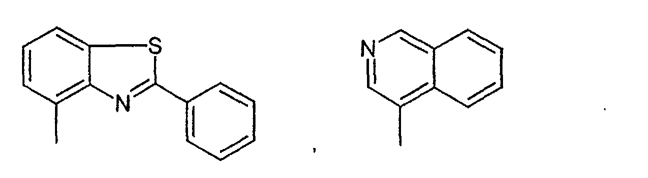 oder 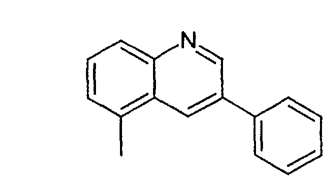 steht, wobei die oben aufgeführten Ringsysteme gegebenenfalls einfach oder mehrfach, gleich oder verschieden durch Substituenten, ausgewählt aus der Gruppe Halogen, Trifluormethyl, Nitro, Cyano, Trifluormethoxy, Carboxyl, Hydroxyl, (C₁-C₆)-Alkoxy, (C₁-C₆)-Alkoxycarbonyl und (C₁-C₆)-Alkyl substituiert sind, das seinerseits durch Aryl mit 6 bis 10 Kohlenstoffatomen oder Halogen substituiert sein kann,
und/oder die aufgeführten Ringsysteme gegebenenfalls durch Gruppen der Formeln -S-R⁶, NR⁷R⁸, CO-NR⁹R¹⁰, SO₂-CF₃, und -A-CH₂-R¹¹ substituiert sind,
worin
R⁶ Phenyl bedeutet, das gegebenenfalls durch Halogen substituiert ist,
R⁷, R⁸, R⁹ und R¹⁰ gleich oder verschieden sind und Wasserstoff, Phenyl, Hydroxy-substituiertes Phenyl, Hydroxy, (C₁-C₆)-Acyl oder (C₁-C₆)-Alkyl bedeuten, das seinerseits durch Hydroxy, (C₁-C₆)-Alkoxycarbonyl, Phenyl oder Hydroxy substituiertes Phenyl substituiert sein kann,
A einen Rest O, S, SO oder SO, bedeutet,
R¹¹ Phenyl bedeutet, das gegebenenfalls ein- bis mehrfach, gleich oder verschieden durch Substituenten, ausgewählt aus der Gruppe Halogen, Nitro, Trifluormethyl, (C₁-C₆)-Alkyl und (C₁-C₆)-Alkoxy substituiert ist,
- R²: für einen Rest der Formel -XR¹² oder -NR¹³R¹⁴ steht,
worin
X eine Bindung oder Sauerstoff bedeutet,
R¹² Wasserstoff, geradkettiges oder verzweigtes (C₁-C₆)-Alkoxycarbonyl oder einen geradkettigen, verzweigten oder cyclischen, gesättigten oder ungesättigten (C₁-C₈)-Kohlenwasserstoffrest bedeutet, der gegebenenfalls eine oder zwei gleiche oder verschiedene Heterokettenglieder aus der Gruppe O, CO, NH, -NH-(C₁-C₄)-Alkyl, -N-((C₁-C₄)-Alkyl)₂, S oder SO₂ enthält und der gegebenenfalls substituiert ist durch Halogen, Nitro, Cyano, Hydroxy, Aryl mit 6 bis 10 Kohlenstoffatomen oder Aralkyl mit 6 bis 10 Kohlenstoffatomen, Heteroaryl oder einer Gruppe der Formel -NR¹⁵R¹⁶,
worin
R¹⁵ und R¹⁶ gleich oder verschieden sind und Wasserstoff, Benzyl oder (C₁-C₆)-Alkyl bedeuten,
R¹³ und R¹⁴ gleich oder verschieden sind und Wasserstoff, (C₁-C₆)-Alkyl oder Cycloalkyl mit 3 bis 6 Kohlenstoffatomen bedeuten,
- R³: für Wasserstoff. Amino oder für einen Rest der Formel 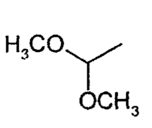 steht oder
für Formyl, Cyano, Trifluormethyl oder Pyridyl steht, oder
für einen geradkettigen, verzweigten oder cyclischen, gesättigten oder ungesättigten Kohlenwasserstoffrest mit bis zu 8 Kohlenstoffatomen steht, der gegebenenfalls ein- oder mehrfach, gleich oder verschieden durch Aryloxy mit 6 bis 10 Kohlenstoffatomen, Azido, Cyano, Hydroxy, Carboxyl, (C₁-C₆)-Alkoxycarbonyl, einen 5- bis 7-gliedrigen heterocyclischen Ring, (C₁-C₆)-Alkylthio oder (C₁-C₆)-Alkoxy substituiert ist, das seinerseits durch Azido oder Amino substituiert sein kann,
und/oder durch Triazolyl substituiert ist, das seinerseits bis zu 3-fach durch (C₁-C₆)-Alkoxycarbonyl substituiert sein kann,
und/oder durch Gruppen der Formeln -OSO₂-CH₃ oder (CO)ₐ-NR¹⁷R¹⁸ substituiert sein kann,
worin
a eine Zahl 0 oder 1 bedeutet,
R¹⁷ und R¹⁸ gleich oder verschieden sind und Wasserstoff oder Aryl, Aralkyl mit 6 bis 10 Kohlenstoffatomen bedeuten,
oder (C₁-C₆)-Alkyl bedeuten, das gegebenenfalls durch (C₁-C₆)-Alkoxycarbonyl, Hydroxyl, Phenyl oder Benzyl substituiert ist, wobei Phenyl oder Benzyl gegebenenfalls ein- oder mehrfach, gleich oder verschieden durch Hydroxy, Carboxyl, (C₁-C₆)-Alkyl oder (C₁-C₆)-Alkoxy substituiert sind,
oder (C₁-C₆)-Alkyl gegebenenfalls durch Gruppen der Formeln NH-CO-CH₃ oder NH-CO-CF₃ substituiert ist,
oder
R¹⁷ und R¹⁸ gemeinsam mit dem Stickstoffatom einen Morpholin-, Piperidinyl- oder Pyrrolidinylring bilden, oder
- R³: für Phenyl steht, das gegebenenfalls durch Methoxy substituiert ist,
oder
- R² und R³: gemeinsam einen Rest der Formel 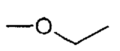 bilden,
- R⁴: für Wasserstoff, (C₁-C₄)-Alkyl, (C₂-C₄)-Alkenyl, Benzoyl oder für Acyl mit 2 bis 6 Kohlenstoffatomen steht,
- R⁵: für Pyridyl steht, das bis zu 3-fach gleich oder verschieden durch Halogen, Hydroxy, Cyano, Trifluormethyl, (C₁-C₆)-Alkoxy, (C₁-C₆)-Alkyl, (C₁-C₆)-Alkylthio, Carbalkoxy, (C₁-C₆)-Acyloxy, Amino, Nitro, Mono- oder (C₁-C₆)-Dialkylamino substituiert ist,
und deren Salze.

Cycloalkyl mit 3 bis 6 Kohlenstoffatomen bzw. (C₃-C₆)-Cycloalkyl steht im Rahmen der Erfindung für Cyclopropyl, Cyclopentyl, Cyclobutyl oder Cyclohexyl. Bevorzugt seien genannt: Cyclopentyl oder Cyclohexyl.

Aryl steht im allgemeinen für einen aromatischen Rest mit 6 bis 10 Kohlenstoffatomen. Bevorzugte Arylreste sind Phenyl und Naphthyl.

(C₁-C₆)-Acyl steht im Rahmen der Erfindung für einen geradkettigen oder verzweigten Acylrest mit 1 bis 6 Kohlenstoffatomen. Bevorzugt ist ein geradkettiger oder verzweigter Acylrest mit 1 bis 4 Kohlenstoffatomen. Besonders bevorzugte Acylreste sind Acetyl und Propionyl.

(C₁-C₆)-Alkyl steht im Rahmen der Erfindung für einen geradkettigen oder verzweigten Alkylrest mit 1 bis 6 Kohlenstoffatomen. Beispielsweise seien genannt: Methyl, Ethyl, Propyl, Isopropyl, tert.Butyl, n-Pentyl und n-Hexyl.

Bevorzugt ist ein geradkettiger oder verzweigter Alkylrest mit 1 bis 4 Kohlenstoffatomen.

(C₂-C₆)-Alkenyl steht im Rahmen der Erfindung für einen geradkettigen oder verzweigten Alkenylrest mit 2 bis 6 Kohlenstoffatomen. Bevorzugt ist ein geradkettiger oder verzweigter Alkenylrest mit 3 bis 5 Kohlenstoffatomen. Beispielsweise seien genannt: Ethenyl, Propenyl, Alkyl, n-Pentenyl und n-Hexenyl.

(C₁-C₆)-Alkoxy steht im Rahmen der Erfindung für einen geradkettigen oder verzweigten Alkoxyrest mit 1 bis 6 Kohlenstoffatomen. Bevorzugt ist ein geradkettiger oder verzweigter Alkoxyrest mit 1 bis 4 Kohlenstoffatomen. Beispielsweise seien genannt: Methoxy, Ethoxy und Propoxy.

(C₁-C₆)-Alkylthio steht im Rahmen der Erfindung für einen geradkettigen oder verzweigten Alkylthiorest mit 1 bis 6 Kohlenstoffatomen. Bevorzugt ist ein geradkettiger oder verzweigter Alkylthiorest mit 1 bis 4 Kohlenstoffatomen. Beispielsweise seien genannt: Methylthio, Ethylthio und Propylthio.

(C₁-C₆)-Alkoxycarbonyl steht im Rahmen der Erfindung für einen geradkettigen oder verzweigten Alkoxycarbonylrest mit 1 bis 6 Kohlenstoffatomen. Bevorzugt ist ein geradkettiger oder verzweigter Alkoxycarbonylrest mit 1 bis 4 Kohlenstoffatomen. Beispielsweise seien genannt: Methoxycarbonyl, Ethoxycarbonyl und Propoxycarbonyl.

Ein geradkettiger, verzweigter oder cyclischer, gesättigter oder ungesättigter (C₁-C₈)-Kohlenwasserstoffrest schließt beispielsweise die oben beschriebenen (C₁-C₆)-Alkyl, (C₂-C₆)-Alkenyl oder (C₃-C₆)-Cycloalkyl, bevorzugt (C₁-C₆)-Alkyl ein.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren und deren jeweilige Mischungen. Die Racemformen lassen sich ebenso wie die Diastereomeren in bekannter Weise in die stereoisomer einheitlichen Bestandteile trennen.

Die Verbindungen der vorliegenden Erfindung schließen die Isomere der allgemeinen Formeln (I) und (Ia) sowie Mischungen davon ein. Im Falle, daß R⁴ Wasserstoff ist, liegen die Isomeren (I) und (Ia) im tautomeren Gleichgewicht vor:

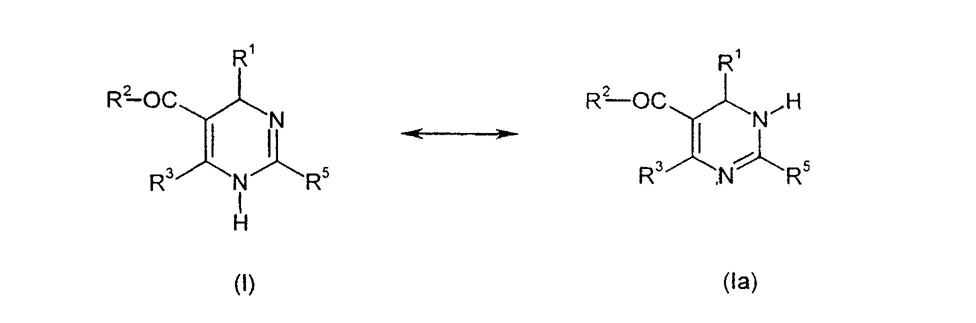

Die erfindungsgemäßen Stoffe können auch als Salze vorliegen. Im Rahmen der Erfindung sind physiologisch unbedenkliche Salze bevorzugt.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Bevorzugt sind erfindungsgemäße Verbindungen der allgemeinen Formeln (I) bzw. (Ia)
in welcher
- R¹: für Phenyl, Furyl, Thienyl, Pyridyl, Cyclopentyl oder Cyclohexyl oder für Reste der Formeln 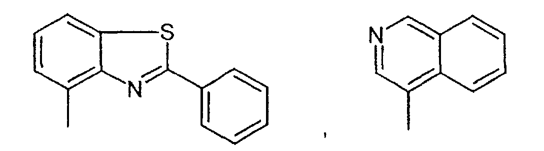 oder 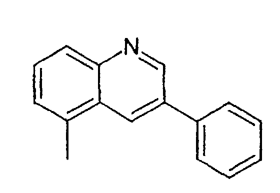 steht,
wobei die oben aufgeführten Ringsysteme gegebenenfalls ein- oder 2-fach gleich oder verschieden durch Substituenten, ausgewählt aus der Gruppe Halogen, Trifluormethyl, Nitro, SO₂-CF₃, Methyl, Cyano, Trifluormethoxy, Amino. Hydroxy, Carboxyl. Methoxycarbonyl und Resten der Formeln -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -S-pCl-C₆H₄ substituiert sind,
- R²: für einen Rest der Formel -XR¹² oder -NR¹³R¹⁴ steht,
worin
X eine Bindung oder ein Sauerstoffatom bedeutet,
R¹² Wasserstoff, (C₁-C₄)-Alkenyl, (C₁-C₄)-Alkoxycarbonyl oder (C₁-C₄)-Alkyl bedeutet, das gegebenenfalls durch Pyridyl, Cyano, Phenoxy, Benzyl oder durch einen Rest der Formel -NR¹⁵R¹⁶ substituiert sind,
worin
R¹⁵ und R¹⁶ gleich oder verschieden sind und Wasserstoff, Benzyl oder (C₁-C₄)-Alkyl bedeuten,
R¹³ und R¹⁴ gleich oder verschieden sind und Wasserstoff, (C₁-C₄)-Alkyl oder Cyclopropyl bedeuten,
- R³: für Wasserstoff, Amino oder einen Rest der Formel 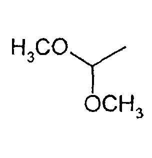 steht,
oder
für Formyl, Cyano, Trifluormethyl, Cyclopropyl oder Pyridyl steht, oder für (C₁-C₄)-Alkyl steht, das gegebenenfalls durch Halogen, (C₁-C₄)-Alkoxycarbonyl, Hydroxy oder durch Triazolyl substituiert ist, das seinerseits bis zu 3-fach durch (C₁-C₄)-Alkoxycarbonyl substituiert sein kann, und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
C₁-C₄-Alkyl bedeuten, das gegebenenfalls durch (C₁-C₄)-Alkoxycarbonyl, Hydroxyl, Phenyl oder Benzyl substituiert ist, wobei Phenyl oder Benzyl gegebenenfalls ein- oder mehrfach gleich oder verschieden durch Hydroxy, Carboxy, (C₁-C₄)-Alkyl oder (C₁-C₄)-Alkoxy substituiert sind,
und/oder (C₁-C₄)-Alkyl gegebenenfalls durch Reste der Formeln -NH-CO-CH₃ oder -NH-CO-CF₃ substituiert ist,
oder
R¹⁷ und R¹⁸ gemeinsam mit dem Stickstoffatom einen Morpholin-, Piperidinyl- oder Pyrrolidinylring bilden, oder
- R³: für Phenyl steht, das gegebenenfalls durch Methoxy substiuiert ist,
oder
- R² und R³: gemeinsam einen Rest der Formel 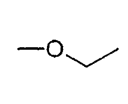 bilden,
- R⁴: für Wasserstoff, Methyl, Benzoyl oder Acetyl steht,
- R⁵: für Pyridyl steht, das bis zu 2-fach gleich oder verschieden durch Fluor, Chlor, Brom, (C₁-C₄)-Alkoxy oder (C₁-C₄)-Alkyl substituiert ist,
und deren Salze.

Besonders bevorzugt sind erfindungsgemäße Verbindungen der allgemeinen Formeln (I) bzw. (Ia),
in welcher
- R¹: für Phenyl, Furyl, Thienyl, Pyridyl, Cyclopentyl, Cyclohexyl oder für Reste der Formeln 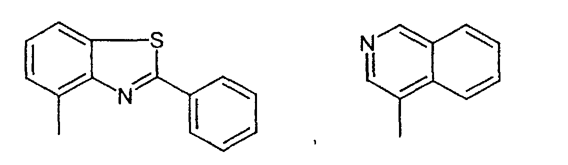 oder 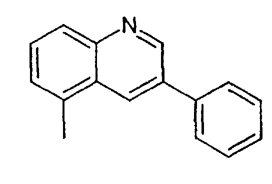 steht,
wobei die oben aufgeführten Ringsysteme gegebenenfalls bis zu 2-fach, gleich oder verschieden durch Substituenten, ausgewählt aus der Gruppe Fluor, Chlor, Brom, Jod, Hydroxy, Trifluormethyl, Nitro, SO₂-CF₃, Methyl, Cyano, Amino, Trifluormethoxy, Carboxyl, Methoxycarbonyl und Resten der Formeln -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -S-pCl-C₆H₄ substituiert sind,
- R²: für einen Rest der Formel -XR¹² oder -NR¹³R¹⁴ steht,
worin
X eine Bindung oder ein Sauerstoffatom bedeutet,
R¹² Wasserstoff, (C₁-C₃)-Alkenyl, (C₁-C₄)-Alkoxycarbonyl oder (C₁-C₄)-Alkyl bedeutet, das gegebenenfalls durch Pyridyl, Cyano, Phenoxy, Benzyl oder durch einen Rest der Formel -NR¹⁵R¹⁶ substituiert sind,
worin
R¹⁵ und R¹⁶ gleich oder verschieden sind und Wasserstoff oder Methyl bedeuten,
R¹³ und R¹⁴ gleich oder verschieden sind und Wasserstoff, (C₁-C₃)-Alkyl oder Cyclopropyl bedeuten,
- R³: für Wasserstoff, Amino oder für einen Rest der Formel 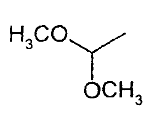 steht,
oder
für Formyl, Cyano, Trifluormethyl, Cyclopropyl oder Pyridyl steht, oder
für (C₁-C₄)-Alkyl steht, das gegebenenfalls durch Fluor, Chlor, (C₁-C₃)-Alkoxycarbonyl, Hydroxy oder durch Triazolyl substituiert ist, das seinerseits bis zu 3-fach durch (C₁-C₃)-Alkoxycarbonyl substituiert sein kann,
und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
(C₁-C₃)-Alkyl bedeuten, das gegebenenfalls durch (C₁-C₃)-Alkoxycarbonyl, Hydroxyl, Phenyl oder Benzyl substituiert ist, wobei Phenyl oder Benzyl gegebenenfalls ein- oder zweifach, gleich oder verschieden durch Hydroxy, Carboxy, (C₁-C₃)-Alkyl oder (C₁-C₃)-Alkoxy substituiert sind,
und/oder (C₁-C₄)-Alkyl gegebenenfalls durch Reste der Formeln -NH-CO-CH₃ oder -NH-CO-CF₃ substituiert ist,
oder
R¹⁷ und R¹⁸ gemeinsam mit dem Stickstoffatom einen Morpholin-, Piperidinyl- oder Pyrrolidinylring bilden, oder
- R³: für Phenyl steht, das gegebenenfalls durch Methoxy substiuiert ist,
oder
- R² und R³: gemeinsam einen Rest der Formel 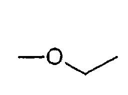 bilden,
- R⁴: für Wasserstoff, Methyl, Benzoyl oder Acetyl steht,
- R⁵: für Pyridyl steht, das bis zu 2-fach gleich oder verschieden durch Fluor. Chlor, (C₁-C₃)-Alkoxy oder (C₁-C₃)-Alkyl substituiert ist,
und deren Salze.

Ganz besonders bevorzugt sind erfindungsgemäße Verbindungen der allgemeinen Formeln (I) bzw. (Ia),
in welcher
- R¹: für Phenyl steht, das gegebenenfalls bis zu 2-fach gleich oder verschieden durch Fluor, Chlor, Brom, Jod, Methyl oder Nitro substituiert ist,
- R²: für -XR¹² steht, worin X für Sauerstoff und R¹² für geradkettiges oder verzweigtes Alkyl mit bis zu 4 Kohlenstoffatomen steht,
- R³: für Methyl, Ethyl oder Cyclopropyl steht,
oder
- R² und R³: gemeinsam einen Rest der Formel 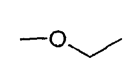 bilden,
- R⁴: für Wasserstoff oder Acetyl steht,
und
- R⁵: für Pyridyl steht, das bis zu zweifach, gleich oder verschieden durch Fluor oder Chlor substituiert ist,
und deren Salze.

Noch bevorzugter sind erfindungsgemäße Verbindungen der allgemeinen Formel (I) oder (Ia), worin R⁵ für 2-Pyridyl steht, das durch 1 bis 2 Fluoratome substituiert sein kann.

Ebenso ganz besonders bevorzugt sind erfindungsgemäße Verbindungen der allgemeinen Formeln (I) bzw. (Ia), die in der Tabelle A aufgeführt sind:

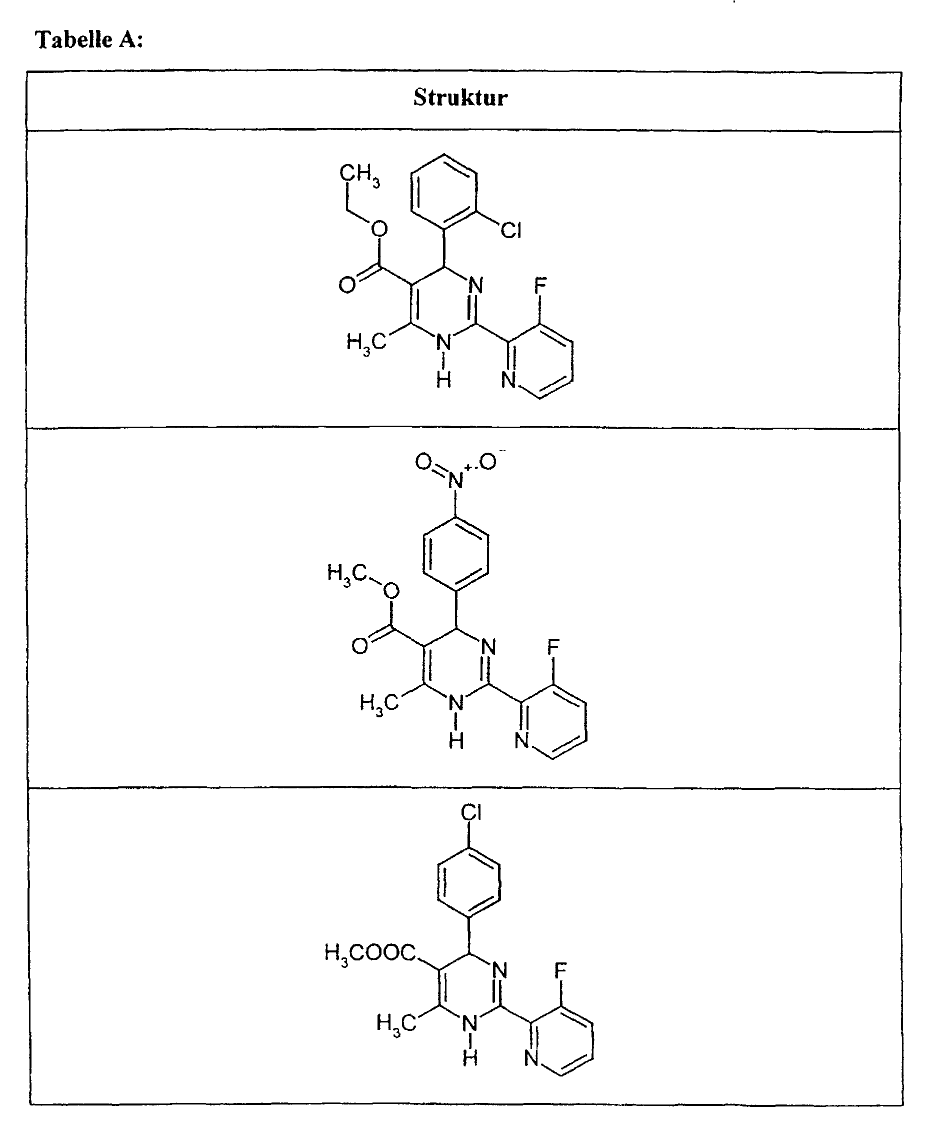

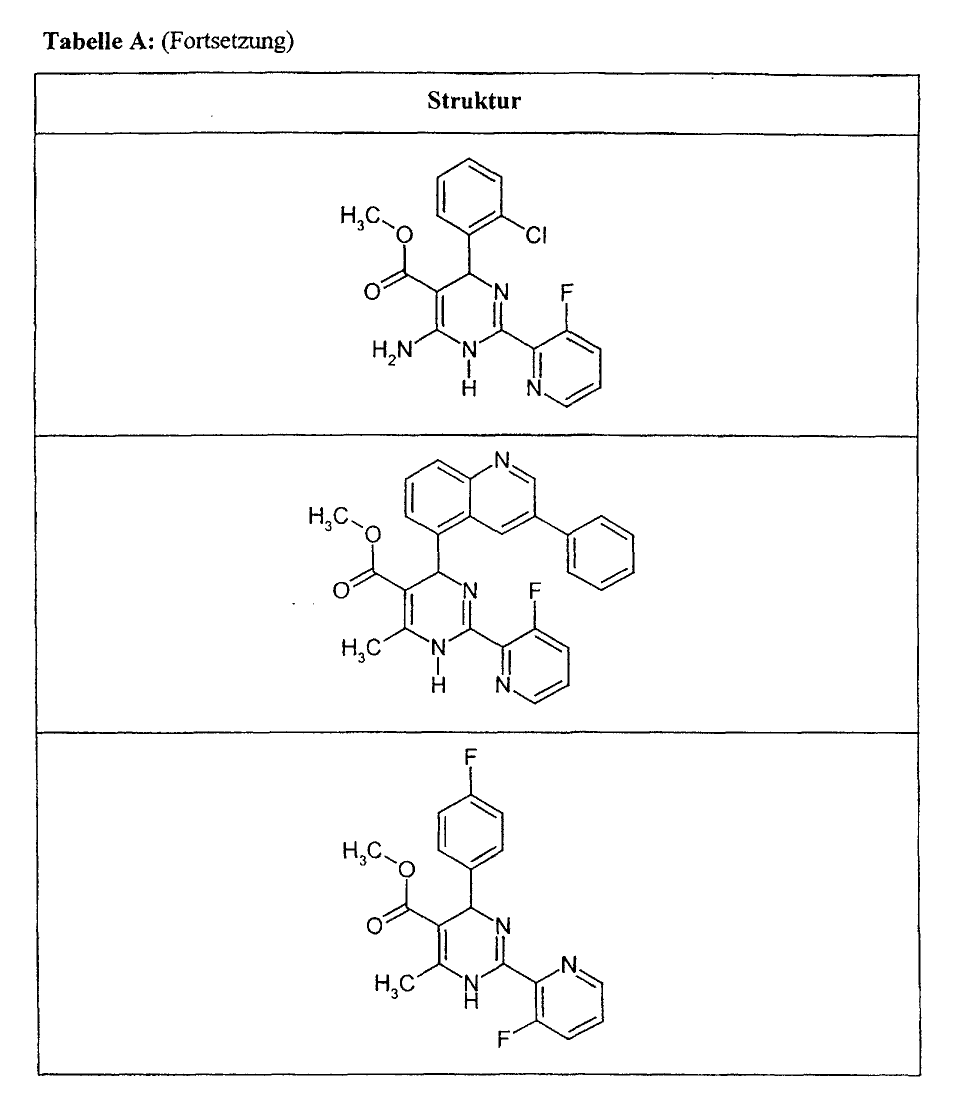

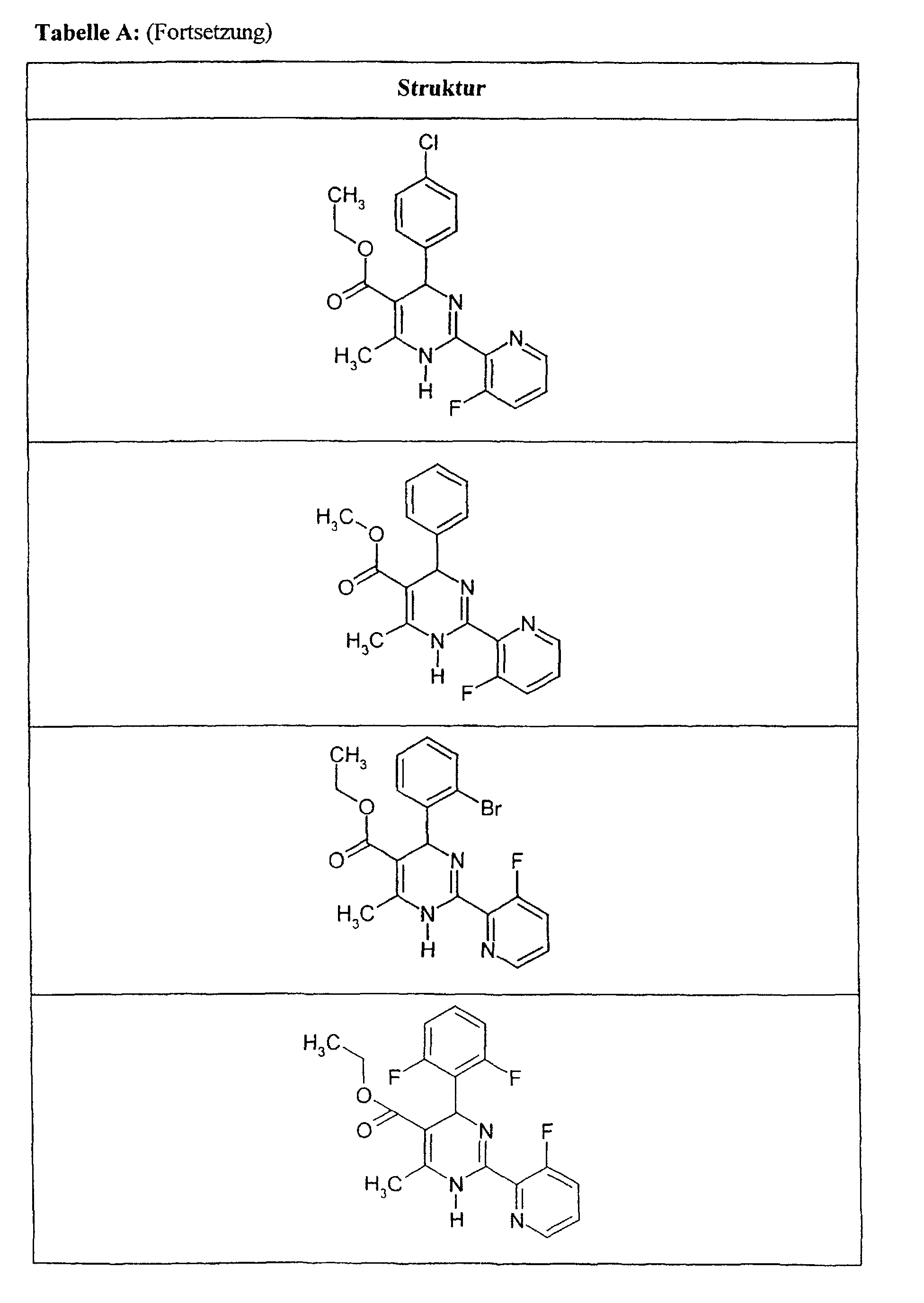

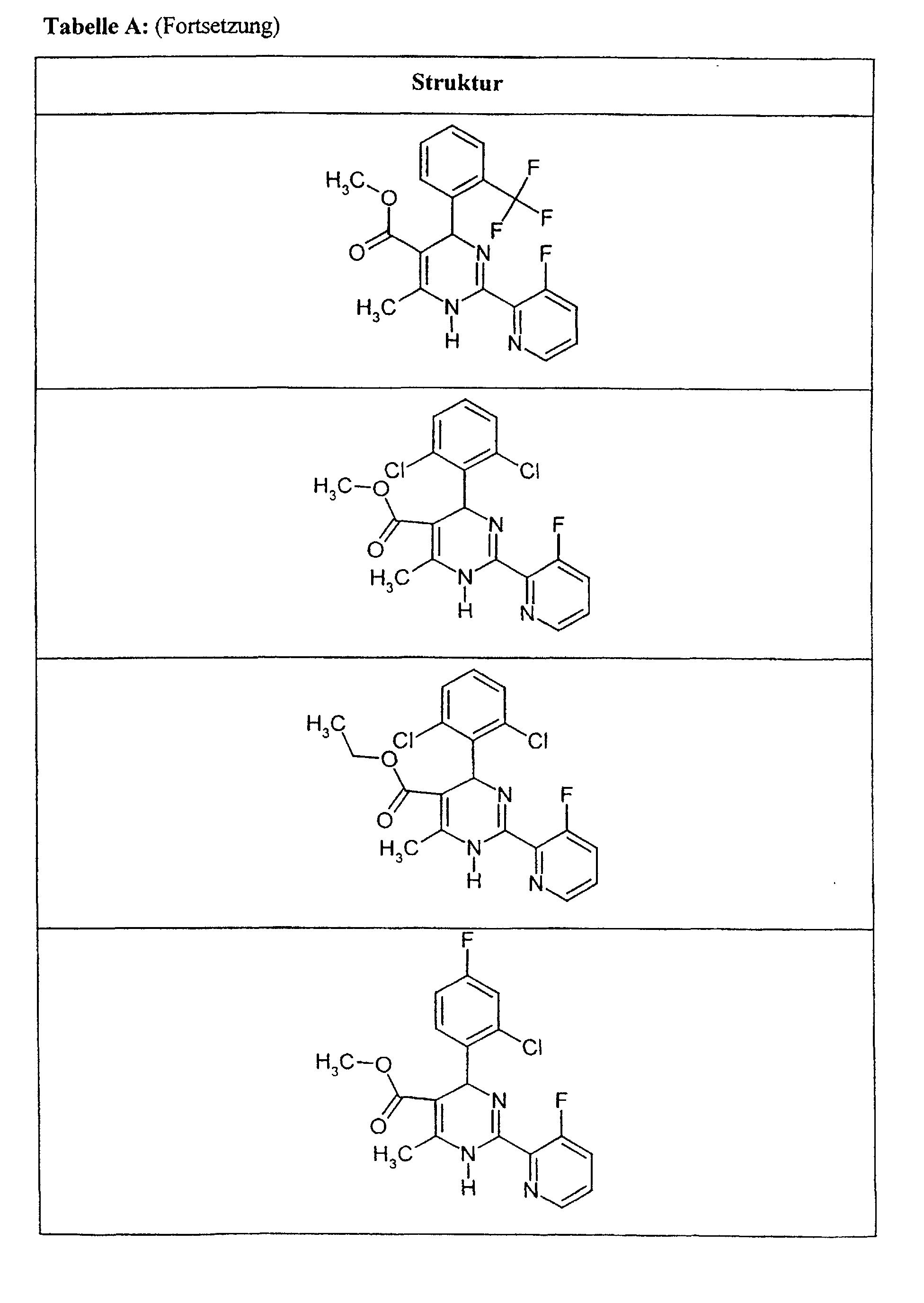

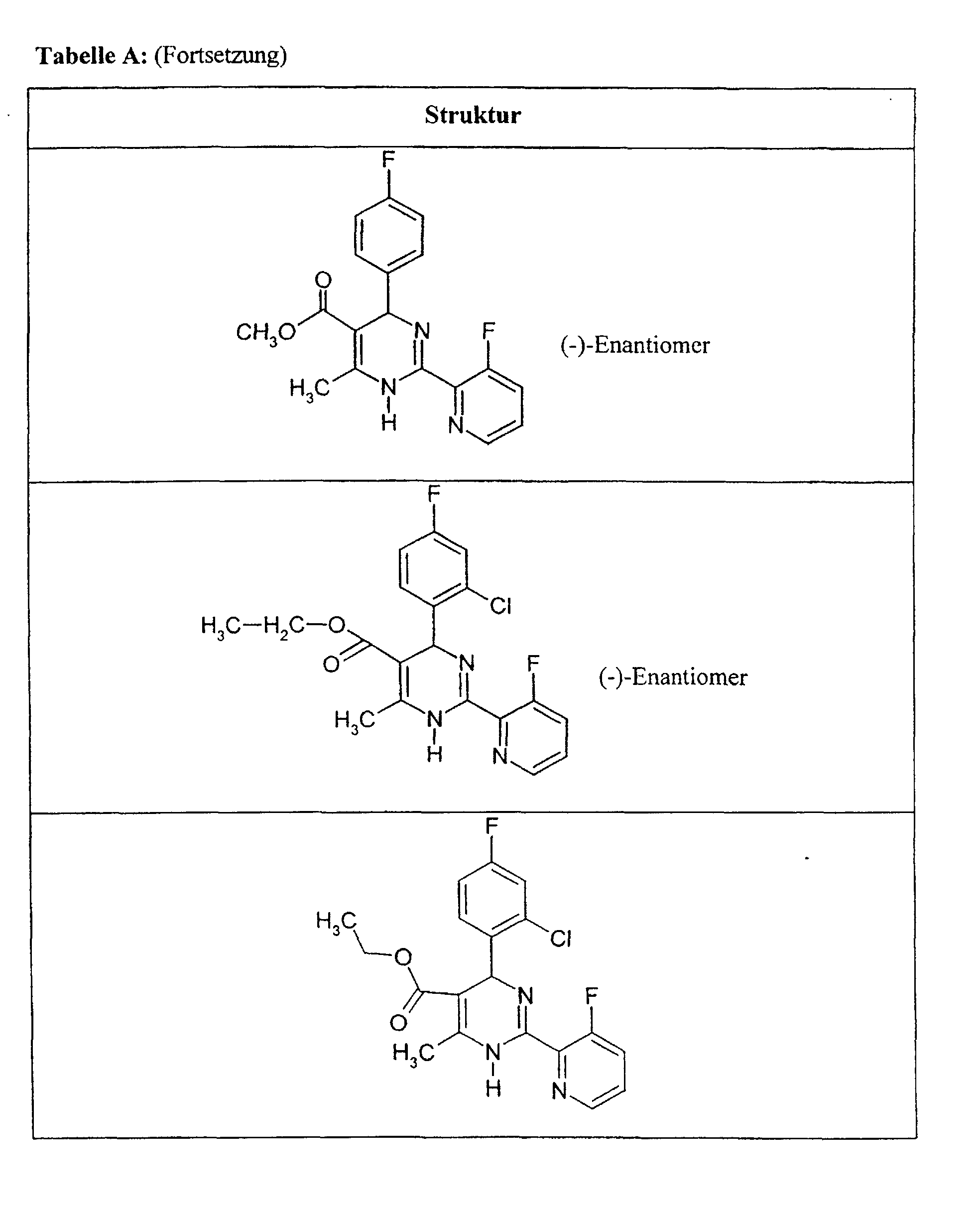

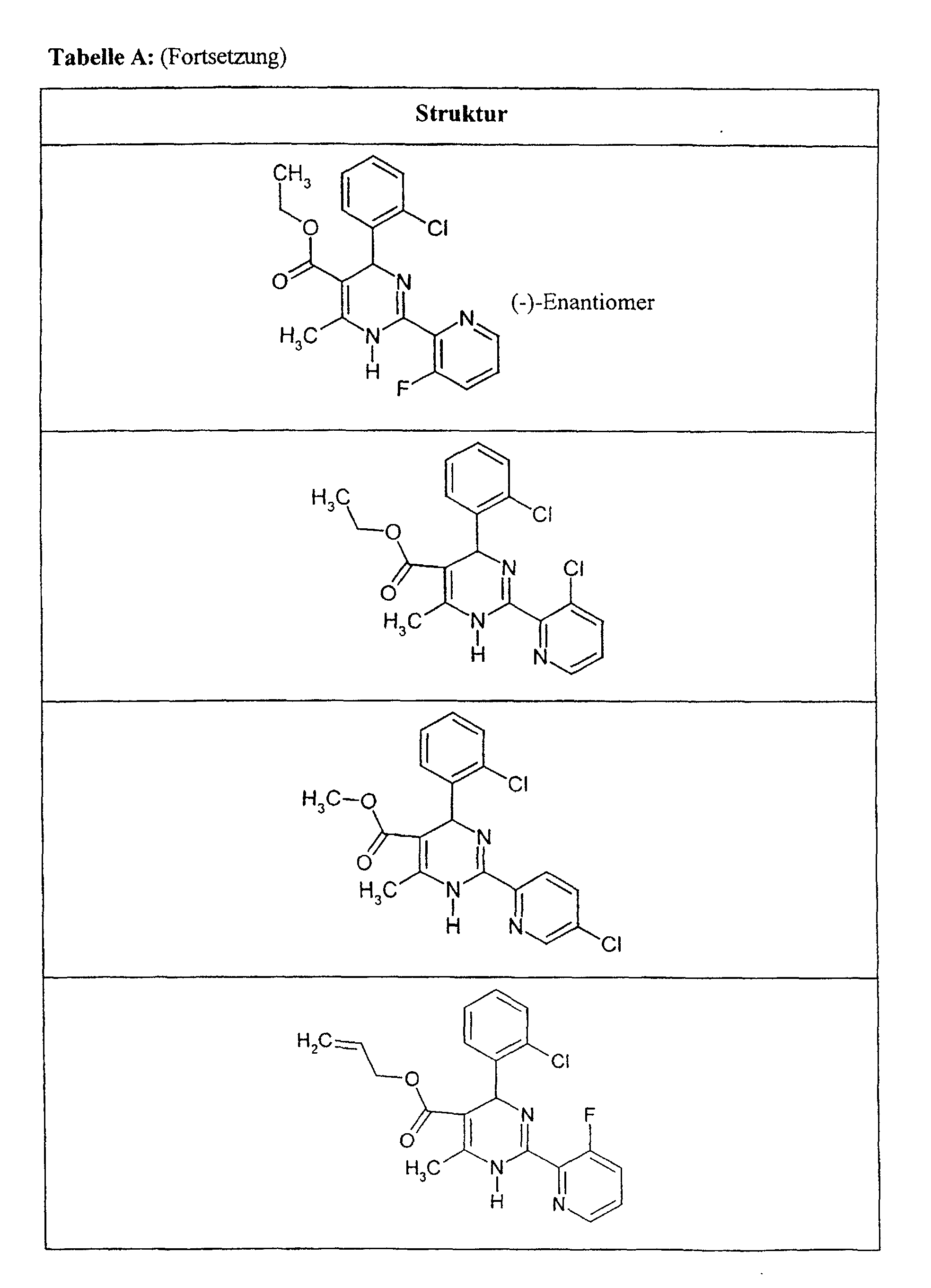

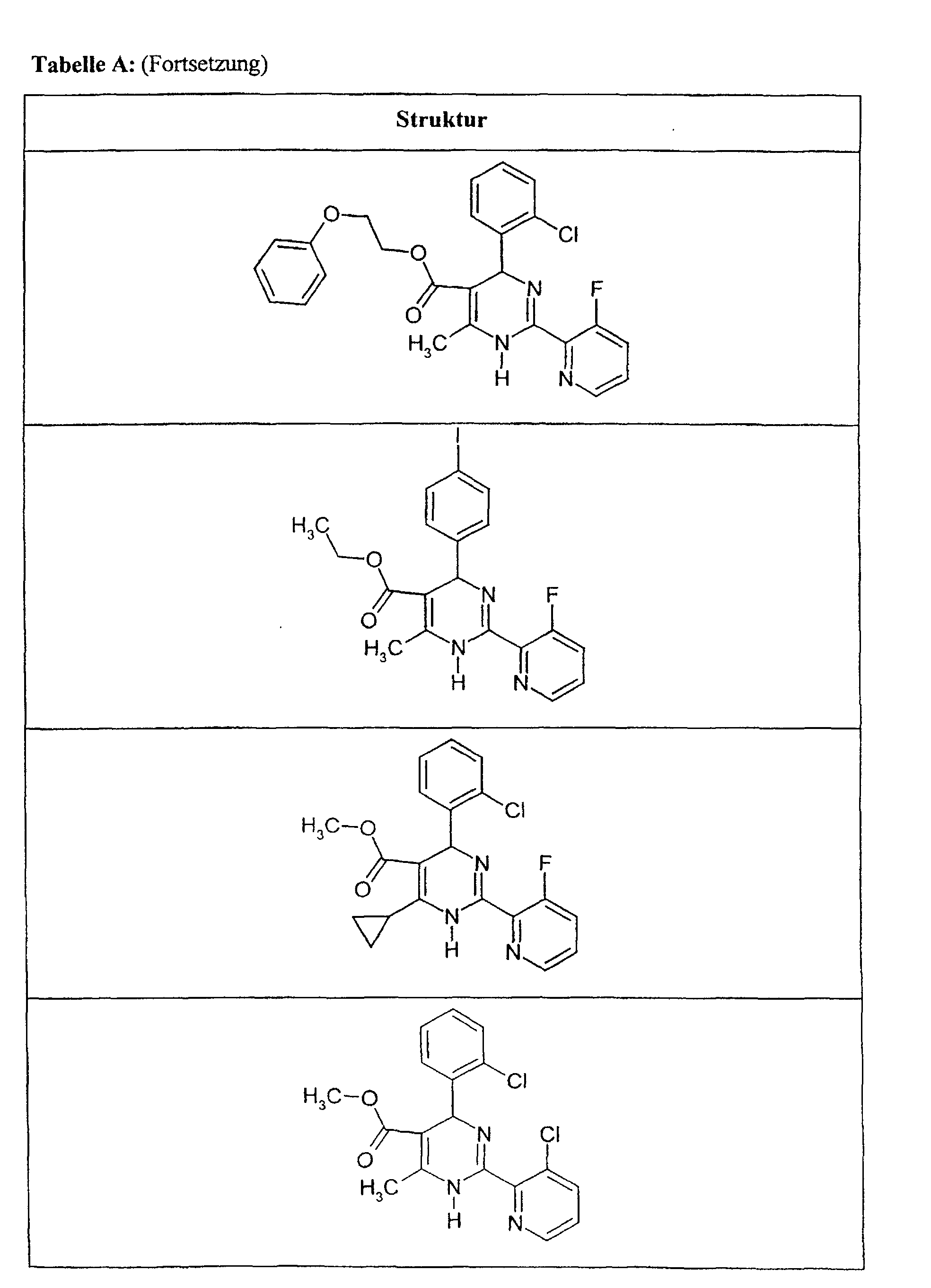

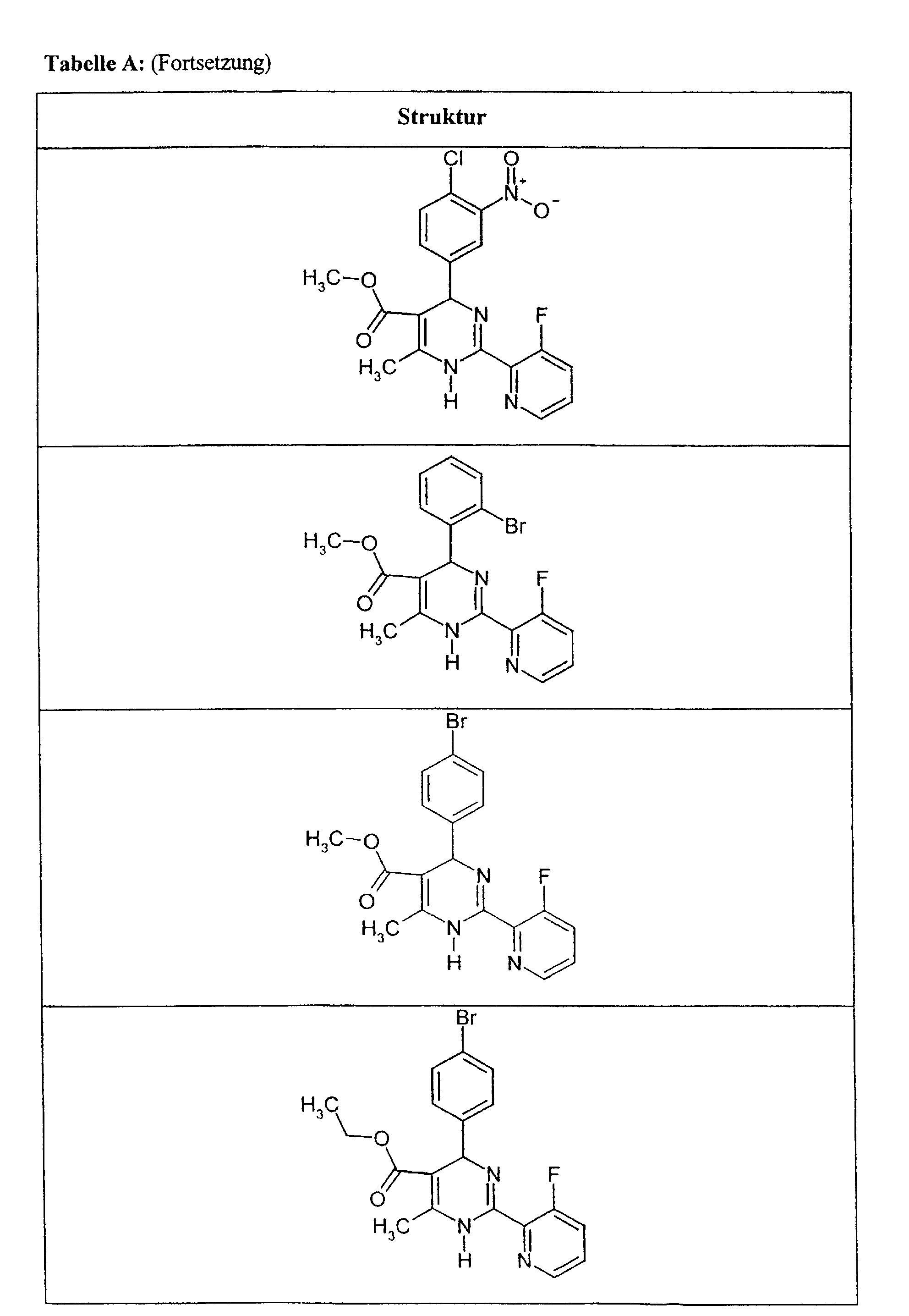

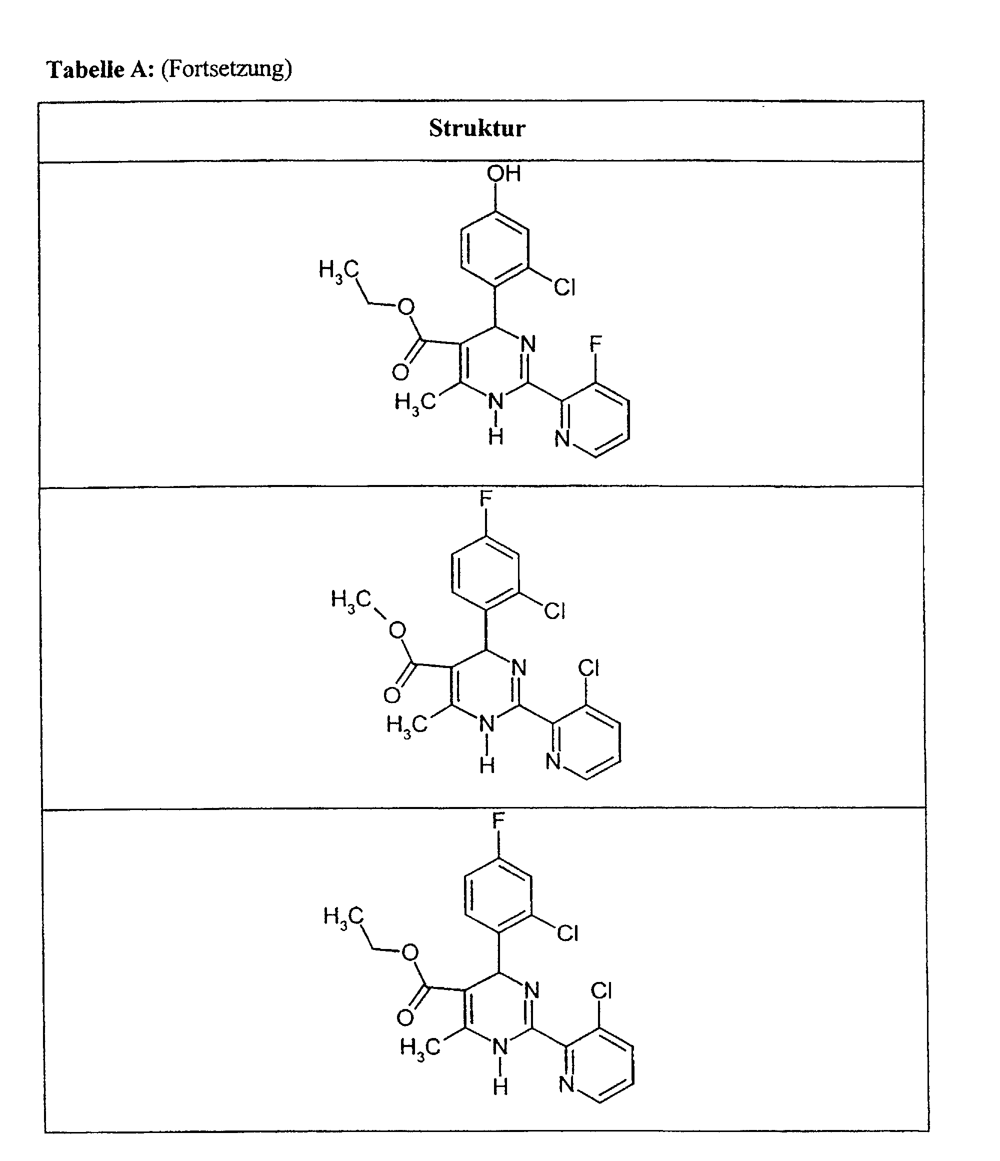

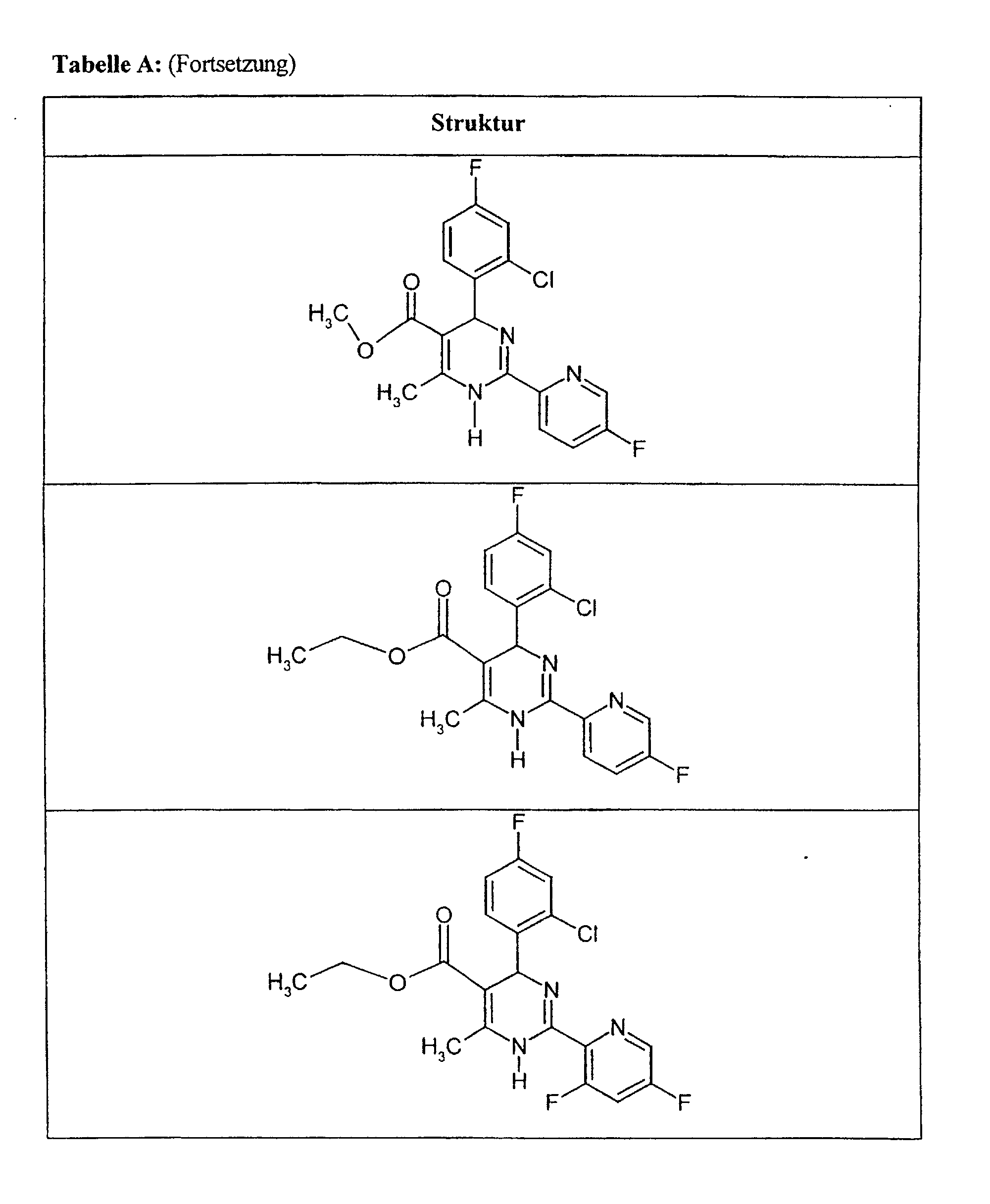

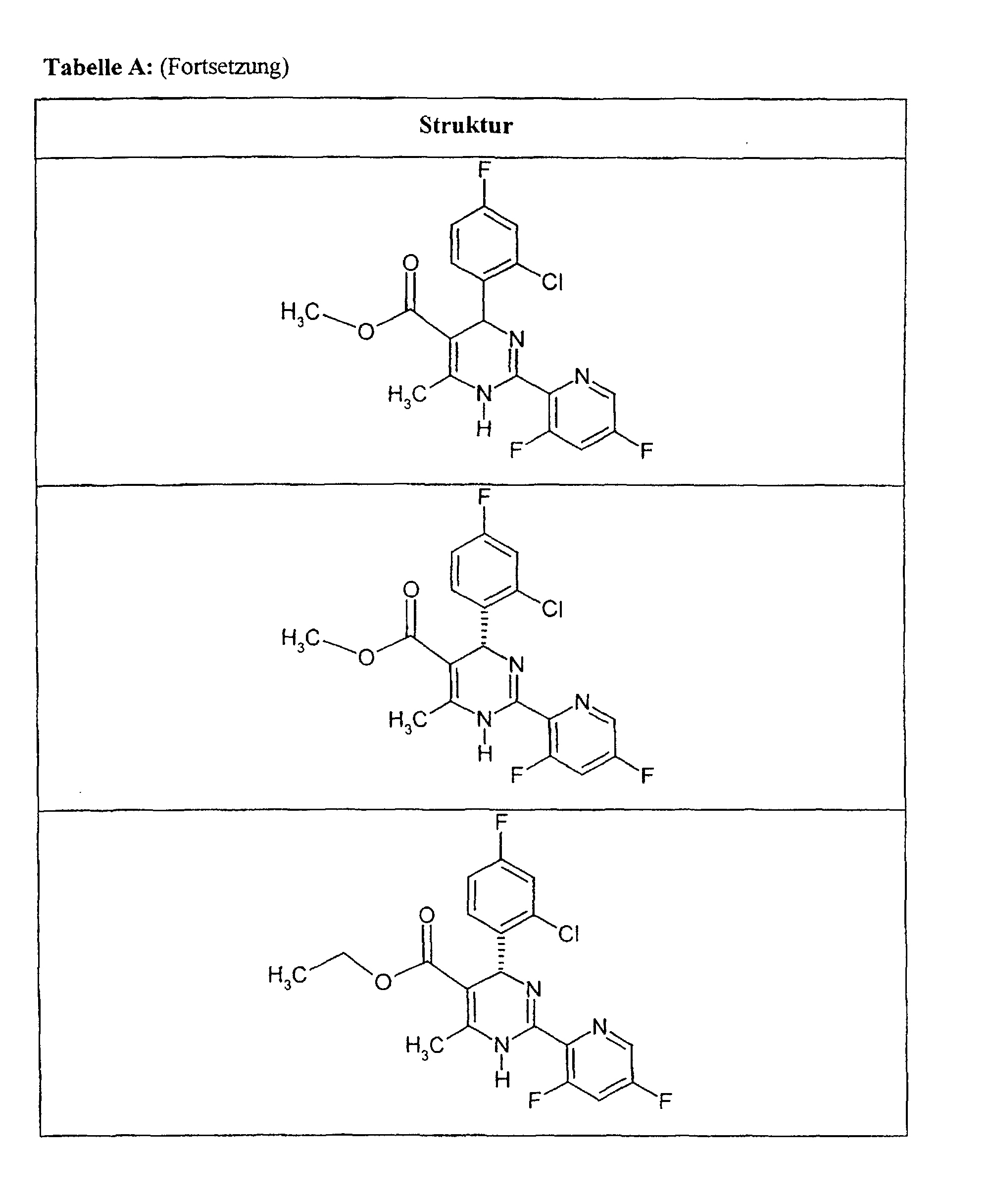

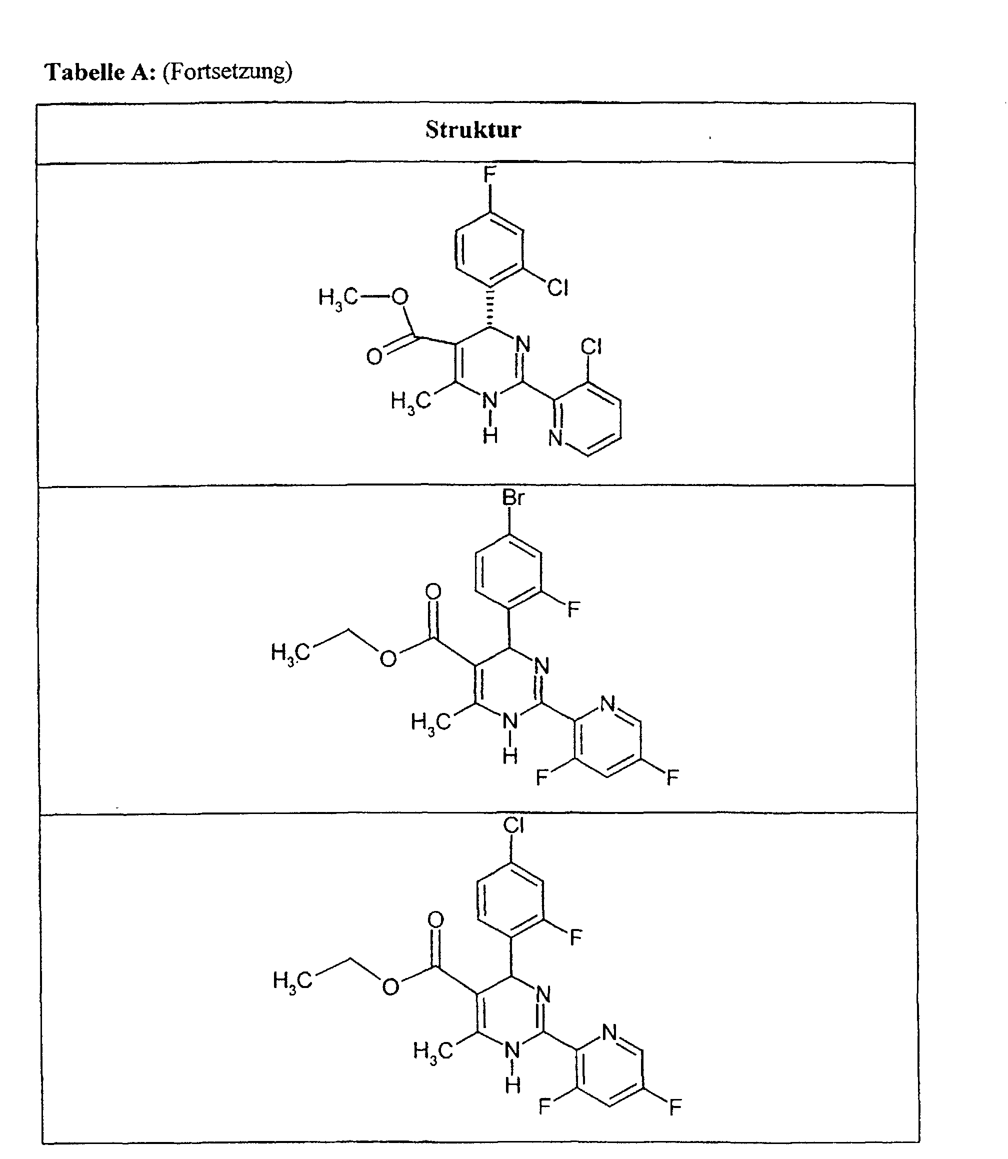

Ganz besonders bevorzugt sind die folgenden Verbindungen:

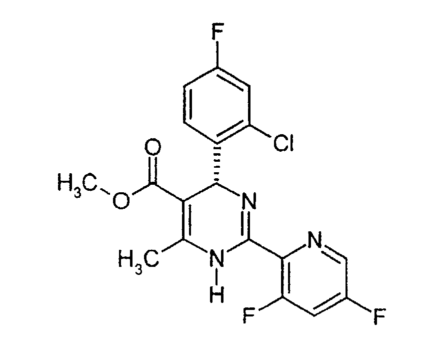

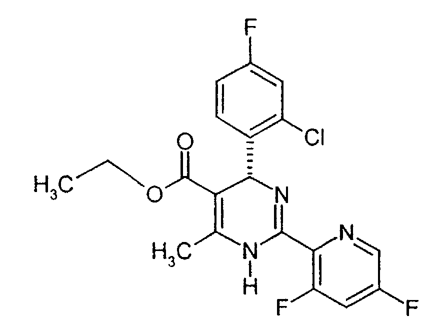

Die erfindungsgemäßen Verbindungen der allgemeinen Formel (I) können hergestellt werden,
indem man
[A] Aldehyde der allgemeinen Formel (II)

   R¹-CHO (II)

   in welcher
   - R¹: die oben angegebene Bedeutung hat,
   mit Amidinen oder deren Hydrochloriden der Formel (III) 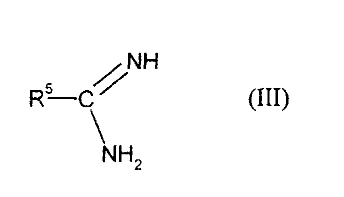 in welcher
   - R⁵: die oben angegebene Bedeutung hat,
   und Verbindungen der allgemeinen Formel (IV)

   R³-CO-CH₂-CO-R² (IV)

   in welcher
   - R² und R³: die oben angegebene Bedeutung haben,
   gegebenenfalls in Gegenwart inerter organischer Lösemittel mit oder ohne Basen- bzw. Säurezusatz umsetzt,
   oder
[B] Verbindungen der allgemeinen Formel (V) 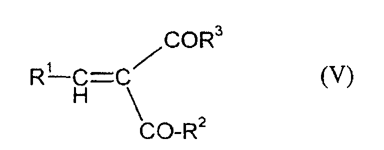 in welcher
   - R¹, R² und R³: die oben angegebene Bedeutung haben,
   mit Amidinen der allgemeinen Formel (III) 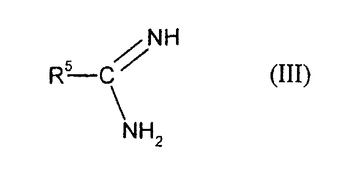 in welcher
   - R⁵: die oben angegebene Bedeutung hat,
   gegebenenfalls in Gegenwart inerter organischer Lösemittel bei Temperaturen zwischen 20°C und 150°C mit oder ohne Basen- oder Säurezusatz umsetzt,
   oder
[C] Aldehyde der allgemeinen Formel (II)

   R¹-CHO (II)

   in welcher
   - R¹: die oben angegebene Bedeutung hat,
   mit Verbindungen der allgemeinen Formel (VI) 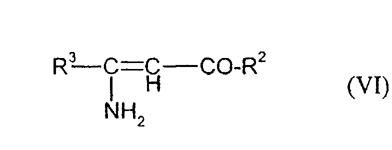 in welcher
   - R² und R³: die oben angegebene Bedeutung haben,
   und Amidinen der allgemeinen Formel (III) wie oben beschriehen umsetzt,
   oder
[D] Aldehyde der allgemeinen Formel (II) mit Verbindungen der allgemeinen Formel (IV) und Iminoether der allgemeinen Formel (VII) 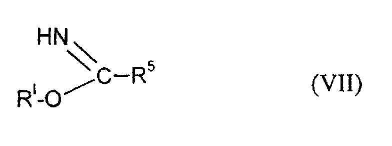 in welcher
   - R⁵: die oben angegebene Bedeutung hat,
   und
   - R¹: für (C₁-C₄)-Alkyl steht,
in Gegenwart von Ammoniumsalzen umsetzt.

Die erfindungsgemäßen Verfahren können durch folgende Formelschemata beispielhaft erläutert werden:

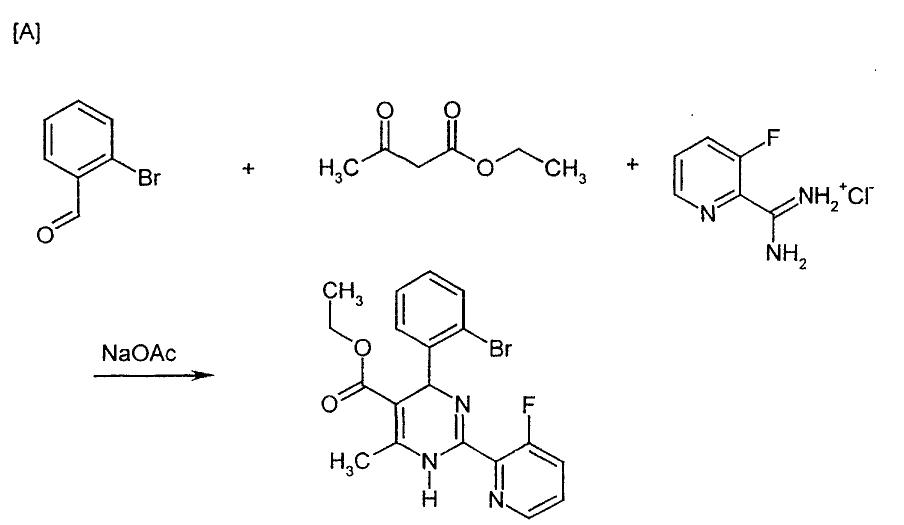

Für alle Verfahrensvarianten A, B, C und D kommen als Lösemittel alle inerten organischen Lösemittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösemittels.

Die Umsetzung kann bei Normaldruck, aber auch bei erhöhtem Druck durchgeführt werden. Im allgemeinen arbeitet man unter Normaldruck.

Die Umsetzung kann mit oder ohne Basen- bzw. Säurezusatz durchgeführt werden, es hat sich jedoch gezeigt, daß eine Umsetzung im Sinne der Erfindung vorzugsweise in Gegenwart von schwächeren Säuren wie z.B. Essigsäure oder Ameisensäure stattfindet.

Die als Ausgangsstoffe verwendeten Aldehyde der allgemeinen Formel (II) sind bekannt oder können nach literaturbekannten Methoden darstellt werden [vgl. T.D. Harris und G.P. Roth, J. Org. Chem. 44, 146 (1979), Deutsche Offenlegungsschrift 2 165 260, Juli 1972, Deutsche Offenlegungsschrift 2 401 665, Juli 1974, Mijano et al., Chem. Abstr. 59, (1963), 13 929 c, E. Adler und H.-D. Becker, Chem. Scand. 15, 849 (1961), E.P. Papadopoulos, M. Mardin und Ch. Issidoridis, J. Org. Chem. Soc. 78, 2543 (1956)).

Die als Ausgangsstoffe verwendeten Yliden-β-ketoester der Formel (V) können nach literaturbekannten Methoden dargestellt werden [vgl. G. Jones, "The Knoevenagel Condensation", in Organic Reactions, Vol. XV, 204 ff. (1967)].

Die als Ausgangsstoffe verwendeten Enaminocarbonsäureester der Formel (VI) und die Iminoether der allgemeinen Formel (VII) sind bekannt oder können nach literaturbekannten Methoden dargestellt werden [vgl. S.A. Glickman and A.C. Cope, J. Am. Chem. Soc. 67, 1017 (1945)].

Die als Ausgangsstoffe verwendeten β-Ketocarbonsäureester der allgemeinen Formel (IV) sind bekannt oder können nach literaturbekannten Methoden dargestellt werden [z.B. D. Borrmann, "Umsetzung von Diketen mit Alkoholen, Phenolen und Mercaptanen", in Houben-Weyl, Methoden der organischen Chemie, Vol. VII/4, 230 ff (1968); Y. Oikawa, K. Sugano und O. Yonemitsu, J. Org. Chem. 43, 2087 (1978)].

Die Verbindungen der allgemeinen Formel (III) sind teilweise bekannt oder im Fall, daß R⁵ für difluoriertes Pyridyl steht, neu, und können hergestellt werden, indem man Verbindungen der Formel (VIII)

R⁵-CN (VIII)

in welcher
- R⁵: die oben angegebene Bedeutung hat,
wie üblich über die Iminoether und abschließend mit Ammoniumchlorid in Methanol umsetzt [vgl. hierzu W.K. Fife, Heterocycles 22, 93-96 (1984); T. Sakamoto, S. Kaneda, S. Nishimura, H. Yamanaka, Chem. Pharm. Bull. 33, 565-571 (1986)] oder andere literaturbekannte Verfahren wie z.B. Garigipati, Tetrahedron Lett. 1990, S. 1969-1972, Boere et al., J. Organomet. Chem. 1987, 331, 161, Caton et al., J. Chem. Soc. 1967, 1204.

Alle Verfahrensschritte erfolgen bei Normaldruck und in einem Temperaturbereich von 0°C bis 130°C, bevorzugt von 20°C bis 100°C.

Die Erfindung betrifft insoweit auch ein Zwischenprodukt der folgenden Formel 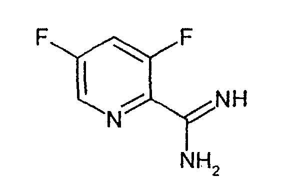 sowie deren Salze, aus dem sich bevorzugte Endprodukte herstellen lassen. Bezüglich der Salze dieser Verbindung sei auf die oben erwähnten Säureaddditionssalze und insbesondere auf das Hydrochlorid verwiesen. Die Herstellung dieser Verbindung erfolgt wie in den Beispielen beschrieben und diesbezüglich wird auch auf das unten dargestellte Reaktionsschema verwiesen.

Die Verbindungen der Formel (VIII) sind an sich bekannt oder können nach bekannten Verfahren analog Beispiel I und II hergestellt werden, indem man Pyridine der allgemeinen Formel (IX)

R⁵-H (IX)

in der der Wasserstoff ortho zum Stickstoff steht und in dem R⁵ die oben angegebene Bedeutung hat, zunächst bei 50 bis 150°C, vorzugsweise bei 100°C, in H₂O₂/Eisessig zu den entsprechenden N-Oxiden umsetzt und anschließend eine Umsetzung mit Trimethylsilylcyanid (TMSCN) nach literaturbekannten Verfahren in den oben aufgeführten inerten Lösungsmitteln, vorzugsweise Acetonitril, THF, Toluol bei Raumtemperatur oder bei Rückflußtemperatur durchführt, gegebenenfalls unter Zusatz von Basen wie Triethylamin oder DBU,
oder indem man in Verbindungen der Formel (X) 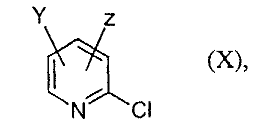 in der Y und Z die unter R⁵ angegebenen Substitutionsreste des Pyridylringes darstellen, mit Hilfe von Cyaniden, wie Kaliumcyanid oder Kupfercyanid das Chlor gegen Cyanid austauscht,
oder in dem Fall, daß R⁵ Difluorpyridyl bedeutet, Verbindungen der Formel (XI) 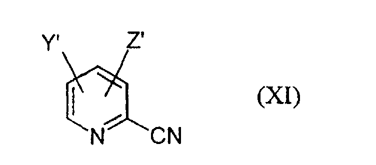 in der Y' und Z' gleich oder verschieden sind und Chlor oder Brom bedeuten, mit Alkali- bzw. Ammoniumfluoriden, vorzugsweise Kaliumfluorid, nach literaturbekannten Verfahren, in polaren Lösemitteln, wie beispielsweise Polyglycole und deren Ether, DMSO oder Sulfolan, gegebenenfalls unter Zusatz von Phasen-Transfer-Katalysatoren, im Sinne einer Halogen-Fluor-Austausch-Reaktion, umsetzt.

Insoweit betrifft die Erfindung auch eine Verbindung der folgenden Formel aus der sich das entsprechende Amidinzwischenprodukt in der in den Beispielen geschilderten Weise herstellen läßt:

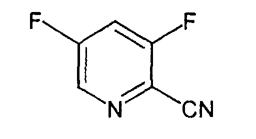

Das obige Verfahren wird bezüglich der 3,5-Difluorpyridylverbindungen beispielhaft durch das folgende Reaktionschema erläutert:

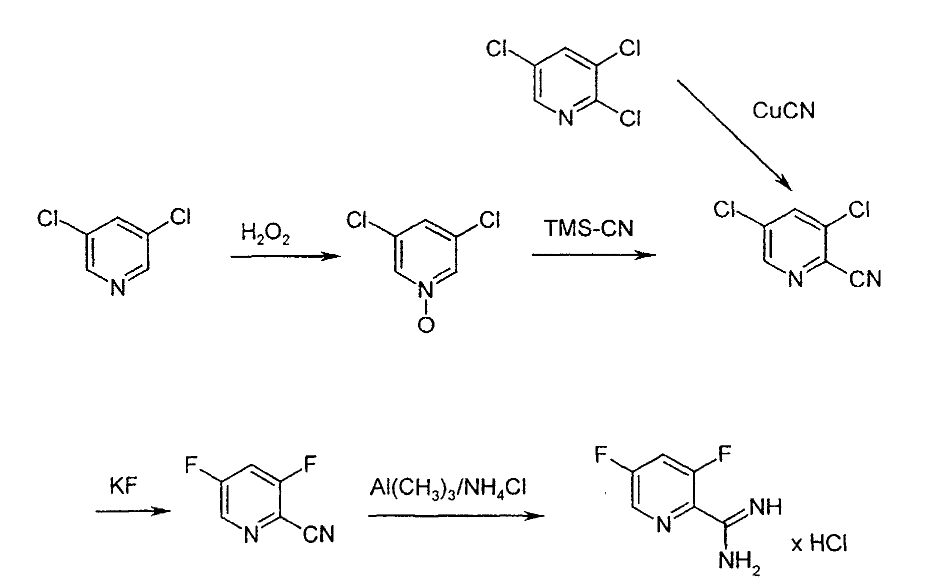

Die antivirale Wirkung der erfindungsgemäßen Verbindungen wurden in Anlehnung an die von Sells et al. ( M.A. Sells, M.-L. Chen, and G. Acs (1987) Proc. Natl. Acad. Sci. 84, 1005 - 1009) und Korba et al. (B.E. Korba and J.L. Gerin (1992) Antiviral Research 19, 55 - 70) beschriebenen Methoden untersucht.

Die antiviralen Tests wurden in 96-well-Mikrotiterplatten durchgeführt. Die erste vertikale Reihe der Platte erhielt nur Wachstumsmedium und HepG2.2.15-Zellen. Sie diente als Viruskontrolle.

Stammlösungen der Testverbindungen (50 mM) wurden zunächst in DMSO gelöst, weitere Verdünnungen wurden in Wachstumsmedium der HepG2.2.15 hergestellt. Die erfindungsgemäßen Verbindungen wurden in der Regel in einer Testkonzentration von 100 µM (1. Testkonzentration) jeweils in die zweite vertikale Testreihe der Mikrotiterplatte pipettiert und anschließend in 2-er Schritten 2¹⁰-fach in Wachstumsmedium plus 2 % foetales Kälberserum, verdünnt (Volumen 25 µl).

Jeder Napf der Mikrotiterplatte erhielt dann 225 µl einer HepG2.2.15 Zellsuspension (5 x 10⁴ Zellen/ml) in Wachstumsmedium plus 2 % fötales Kälberserum. Der Testansatz wurde 4 Tage, 37°C, 5 % CO₂, inkubiert.

Anschließend wurde der Überstand abgesaugt und verworfen, und die Näpfe erhielten 225 µl frisch zubereitetes Wachstumsmedium. Die erfindungsgemäßen Verbindungen wurden jeweils erneut als 10-fach konzentrierte Lösung in einem Volumen von 25 µl zugefügt. Die Ansätze wurden weitere 4 Tage inkubiert.

Vor der Ernte der Überstände zur Bestimmung des antiviralen Effektes wurden die HepG2.2.15-Zellen lichtmikroskopisch oder mittels biochemischer Nachweisverfahren (z.B. Alamar-Blue-Färbung oder Trypanblau-Färbung) auf zytotoxische Veränderungen untersucht.

Anschließend wurden die Überstände geerntet und mittels Vakuum auf mit Nylonmembran bespannten 96-Napf-Dot-Blot-Kammern (entsprechend den Angaben der Hersteller) gesogen.

### Zytotoxizitätsbestimmung

Substanzinduzierte zytotoxische oder zytostatische Veränderungen der HepG2.2.15-Zellen wurden z.B. lichtmikroskopisch als Änderungen der Zellmorphologie ermittelt. Derartige Substanz-induzierte Veränderungen der HepG2.2.15-Zellen im Vergleich zu unbehandelten Zellen wurden z.B. als Zellyse, Vakuolisierung oder veränderter Zellmorphologie sichtbar. 50 % Zytotoxizität (Tox.-50) bedeuten, daß 50 % der Zellen eine der entsprechenden Zellkontrolle vergleichbare Morphologie aufweisen.

Die Verträglichkeit einiger der erfindungsgemäßen Verbindungen wurde zusätzlich auf anderen Wirtszellen wie z.B. HeLa-Zellen, primäre periphere Blutzellen des Menschen oder transformierte Zellinien wie H-9-Zellen, getestet.

Es konnten keine zytotoxischen Veränderungen bei Konzentrationen der erfindungsgemäßen Verbindungen von >10µM festgestellt werden.

### Bestimmung der antiviralen Wirkung

Nach Transfer der Überstände auf die Nylon Membran der Blot Apparatur (s.o.) wurden die Überstände der HepG2.2.15 Zellen denaturiert (1.5 M NaCl/ 0.5 N NaOH), neutralisiert (3 M NaCl/ 0.5 M Tris HCI, pH 7.5) und gewaschen (2 x SSC). Anschließend wurde die DNA durch Inkubation der Filter bei 120°C, 2 - 4 Stunden, an die Membran gebacken.

### Hybridisierung der DNA

Der Nachweis der viralen DNA von den behandelten HepG2.2.15-Zellen auf den Nylonfiltern wurde in der Regel mit nichtradioaktiven, Digoxigenin-markierten Hepatitis B-spezifischen DNA-Sonden durchgeführt, die jeweils nach Angabe des Herstellers mit Digoxigenin markiert, gereinigt und zur Hybridisicrung eingesetzt wurden.

Die Prähybridisierung und Hybridisierung erfolgte in 5 x SSC, 1 x Blockierungsreagenz, 0.1 % N-Lauroylsarcosin, 0.02 % SDS und 100 µg Sperma DNA des Herings. Die Prähybridisierung erfolgte 30 Minuten bei 60°C, die spezifische Hybridisierung

### Nachweis der HBV DNA durch Digoxigenin Antikörper

Der immunologische Nachweis der Digoxigenin markierten DNA erfolgte nach Angaben des Herstellers:
Die Filter wurden gewaschen und in einem Blockierungsreagenz (nach Angabe des Herstellers) prähybridisiert. Anschließend wurde mit einem Anti-DIG-Antikörper, der mit alkalischer Phosphatase gekoppelt war, 30 Minuten hybridisiert. Nach einem Waschschritt wurde das Substrat der alkalischen Phosphatase, CSPD, zugefügt, 5 Minuten mit den Filtern inkubiert, anschließend in Plastikfolie eingepackt und weitere 15 Minuten bei 37°C inkubiert. Die Chemilumineszenz der Hepatitis B-spezifischen DNA-Signale wurde über eine Exposition der Filter auf einem Röntgenfilm sichtbar gemacht (Inkubation je nach Signalstärke: 10 Minuten bis 2 Stunden).

Die halbmaximale Hemmkonzentration (IC-50, inhibitorische Konzentration 50 %) wurde als die Konzentration bestimmt, bei der gegenüber einer unbehandelten Probe die Hepatitis B-spezifische Bande durch die erfindungsgemäße Verbindung um 50 % reduziert wurde.

Die Behandlung der Hepatits B-Virus produzierenden HepG2.2.15-Zellen mit den erfindungsgemäßen Verbindungen führte überraschenderweise zu einer Reduktion viraler DNA im Zellkulturüberstand, die von den Zellen in Form von Virionen in den Zellkulturüberstand ausgeschleust wird.

Die erfindungsgemäßen Verbindungen zeigen ein neue, nicht vorhersehbare und wertvolle Wirkung gegen Viren. Sie sind überraschenderweise antiviral gegen Hepatitis B (HBV) wirksam und sind somit zur Behandlung von virusinduzierten Erkrankungen, insbesondere von akut und chronisch persistenten Virusinfektionen des HBV geeignet. Ein chronische Viruserkrankung, hervorgerufen durch das HBV, kann zu unterschiedlich schweren Krankheitsbildern führen; bekanntermaßen führt die chrochronische Hepatitis B-Virusinfektion in vielen Fällen zur Leberzirrhose und/oder zum hepatozellulären Karzinom.

Als Indikationsgebiete für die erfindungsgemäß verwertbaren Verbindungen können beispielsweise genannt werden:

Die Behandlung von akuten und chronischen Virusinfektionen, die zu einer infektiösen Hepatitis führen können, beispielsweise die Infektionen mit Hepatitis B-Viren. Besonders bevorzugt ist die Behandlung von chronischen Hepatits-B Infektionen und die Behandlung von akuter Hepatitis-B Virusinfektion.

Zur vorliegenden Erfindung gehören pharmazeutische Zubereitungen, die neben nichttoxischen, inerten pharmazeutisch geeigneten Trägerstoffen eine oder mehrere Verbindungen der Formeln (I), (Ia) bzw. der Tabelle A enthalten oder die aus einem oder mehreren Wirkstoffen der Formeln (I) und (Ia) bestehen, sowie Verfahren zur Herstellung dieser Zubereitungen.

Die Wirkstoffe der Formeln (I) und (Ia) sollen in den oben aufgeführten pharmazeutischen Zubereitungen in einer Konzentration von etwa 0,1 bis 99,5 Gew.-%, vorzugsweise von etwa 0,5 bis 95 Gew.-% der Gesamtmischung, vorhanden sein.

Die oben aufgeführten pharmazeutischen Zubereitungen können außer den Verbindungen der Formeln (I) und (Ia) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels, sowie dem Zeitraum bzw. Intervall, innerhalb welchem die Verabreichung erfolgt.

### Ausgangsverbindungen

### Beispiel I

### 3-Fluorpyridin-N-oxid

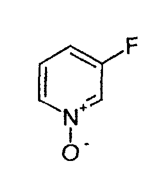

11,10 g (114,324 mmol) 3-Fluorpyridin werden in 74,00 ml Essigsäure gelöst. Man gibt 22,20 ml H₂O₂ hinzu und läßt 7 Stunden bei 100°C Badtemperatur rühren. Danach wird bis auf 30 ml eingeengt, 30 ml Wasser zugefügt und wieder auf 30 ml eingeengt. Die Lösung wird mit Dichlormethan verrührt, durch Zugabe von K₂CO₃ basisch gestellt, getrennt, die wäßrige Phase 2 mal mit Dichlormethan ausgeschüttelt, getrocknet und eingeengt.
Ausbeute: 11,5 g (88,9 %)
Schmp.: 66-68°C

### Beispiel II

### 2-Cyano-3-fluorpyridin

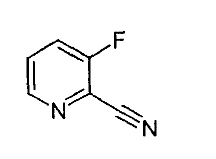

5,20 g (45,980 mmol) der Verbindung aus Beispiel I werden in 50 ml Acetonitril gelöst. Unter Argon werden 13,70 g (138,092 mmol) Trimethylsilylnitril zugegeben und langsam 12,80 ml Triethylamin zulaufen gelassen. Die Lösung wird 7 Stunden unter Rückfluß über Nacht bei Raumtemperatur gerührt. Nach dem Einengen mit einer Wasserstrahlpumpe wird in Dichlormethan aufgenommen, 2 x mit 50 ml 2 N Natriumcarbonat geschüttelt, mit Wasser gewaschen, getrocknet und eingeengt.
Ausbeute (roh): 5,3 g (Öl)
Säulenchromatographie: Methylenchlorid bis Methylenchlorid/Essigester 10:1
Öl wird fest!

### Beispiel III

### 2-Amidino-3-fluorpyridin-hydrochlorid

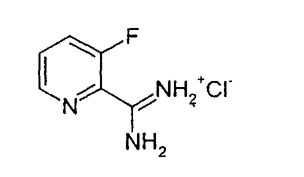

10,30 g (84,355 mmol) der Verbindung aus Beispiel II werden in 30 ml Methanol gelöst. Die Lösung wird mit einer Lösung von 0,40 g (17,391 mmol) Natrium in 65 ml Methanol versetzt und 72 Stunden bei 20°C gerührt. 5,44 g (101,682 mmol) Ammoniumchlorid (gemörsert) und 17,39 mmol (1,04 ml) Essigsäure werden zugegeben, 28 Stunden bei 40°C nachgerührt und abgekühlt. Es wird vom nichtlöslichen Salz abgesaugt (1,78 g), eingeengt, mit Aceton eingeengt, anschließend mit Aceton verrührt, abgesaugt und gewaschen.
Ausbeute: 10,6 g
Schmp.: ≈ 150°C Zers.

### Beispiel IV

### 2-Cyano-3,5-dichlor-pyridin

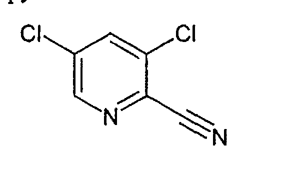

### Methode 1:

26 g (0,158 mol) 3,5-Dichlor-pyridin-1-oxid ( Johnson et al., J.Chem.Soc.B, **1967**, 1211) werden in 80 mL CH₂Cl₂ gelöst und nacheinander mit 21,8 ml (0,174 mol) Trimethylsilylcyanid und 14,6 ml (0,158 mol) Dimethylcarbamidsäurechlorid versetzt und 48 h bei Raumtemperatur gerührt. Es wird mit 100 mL einer 10%igen NaHCO₃-Lsg. versetzt und 10 min intensiv gerührt. Nach Separierung der Phasen wird 1x mit CH₂Cl₂ ausgeschüttelt und die vereinigten organischen Phasen getrocknet und eingeengt. Der Rückstand wird mit CH₂CI₂ an Kieselgel chromatographiert und aus wenig Methanol umkristallisiert.
Man erhält 11 g (40,2 %) 2-Cyano-3,5-dichlor-pyridin (FP: 102°C).

### Methode 2:

Analog Troschuetz, R. et al., J. Heterocycl. Chem. 1996, 33, 1815-1821 werden 150 ml Diethylenglykoldimethylether (Diglyme), 47,68 g (0,261 mol) 2,3,5-Trichlorpyridin, 2,0 g (0,005 mol) Tetraphenylphosphoniumbromid, 4,0 g (0,024 mol) feingepulvertes Kaliumiodid und 75,0 g (0,838 mol) Kupfer(I)cyanid unter Stickstoff zusammengegeben und 24 Stunden bei Rückfluß gerührt. Anschließend weitere 100ml Diglyme, 2,0g (0,005 mol) Tetraphenylphosphoniumbromid, 4,0g (0,024 mol) feingepulvertes KI und 75 g (0,838 mol) CuCN hinzugegeben, und man rührt weiter 89 Stunden bei RF. Nach Abkühlen auf Raumtempertur wird abgesaugt und das Filtrat destillativ weitgehend von Diglyme befreit. Der Rückstand wird in Toluol aufgenommen und mit einer wäßrigen Lösung von Mohr'schem Salz- und dann mit NaHCO₃-Lösung gewaschen (Peroxidtest). Dann wird mit Wasser Diglyme-frei gewaschen. Man filtriert über Celit, trocknet das Filtrat über MgSO₄ und engt die Lösung ein.
Man erhält 18,0 g (40,0 %) 2-Cyano-3,5-dichlorpyridin.

### Beispiel V

### 3,5-Difluor-pyridin-2-carbonitril

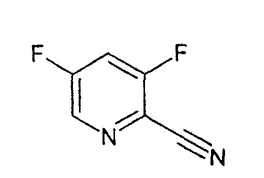

50 g (0,29 mol) 3,5-Dichlor-pyridin-2-carbonitril (Beispiel IV), 33,6 g (0,58 mol) Kaliumfluorid und 10 g Polyethylengycol 8000 werden mit 125 ml DMSO versetzt und 30 min auf 160°C erhitzt. Nach Abkühlung wird das Produkt zusammen mit dem DMSO im Hochvakuum abdestilliert, das Destillat auf Wasser gegeben, mit Toluol extrahiert und über Na₂SO₄ getrocknet. Das Produkt wird als Toluollösung weiter umgesetzt.
(R_{f}-Wert: 0,43 , Cyclohexan/Essigester = 7:3)

### Beispiel VI

### 3,5-Difluoro-2-pyridincarboximidamid-hydrochlorid

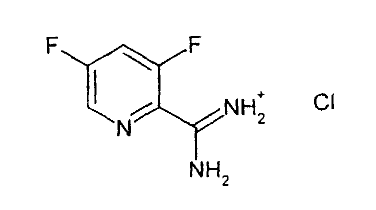

33,4 g (0,624 mol) Ammoniumchlorid werden in 1 l Toluol suspendiert und auf 0-5°C gekühlt. Dazu werden 328 ml Trimethylaluminium (2 M in Hexan, 0,624 mol) getropft und bei RT gerührt bis die Methanentwicklung beendet ist. Danach wird die toluolische Lösung von 3,5-Dichlor-pyridin-2-carbonitril (Lösung aus Beispiel V) zugetropft und anschließend über Nacht bei 80°C nachgerührt. Nach Kühlung auf 0 bis -5°C wird MeOH bis zur beendeten Gasentwicklung zugetropft, die Salze abgesaugt und 2x mit wenig MeOH gewaschen. Es wird einrotiert, der Rückstand in 500 ml CH₂Cl₂/MeOH (9:1) gelöst und nochmals von anorganischen Salzen abgesaugt. Nach Einrotieren verbleiben 23,6 g (39,1 %) 3,5-Difluoro-2-pyridincarboximidamid als Hydrochlorid (FP:183°C).

¹H-NMR(DMSO-D6): 8,3-8,45(m,1H), 8,8(d,J=2Hz,1H), 9,7(s,breit,4H) ppm.

### Beispiel VII

### 2-Acetyl-3-(2-chlor-4-fluorphenyl)-2-propensäuremethylester

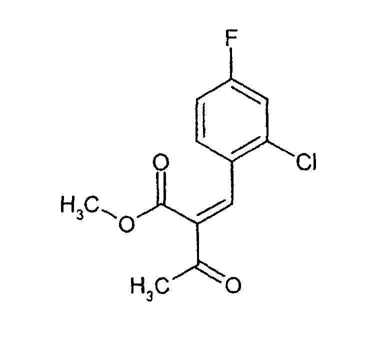

50 g (315 mmol) 2-Chlor-4-fluor-benzaldehyd und 36,6 g (315 mmol) Acetessigsäuremethylester werden in 150 ml Isopropanol gelöst und mit 1,7 ml Piperidinacetat versetzt. Nach Rühren über Nacht bei Raumtemperatur wird mit Methylenchlorid verdünnt und mit Wasser ausgeschüttelt, über Natriumsulfat getrocknet und eingeengt. Das Produkt wird roh als cis/trans-Gemisch weiter umgesetzt.

### Herstellungsbeispiele

### Beispiel 1

### 4-(2-Bromphenyl)-2-(3-fluorpyridin-2-yl)-6-methyl-1,4-dihydro-pyrimidin-5-carbonsäureethylester

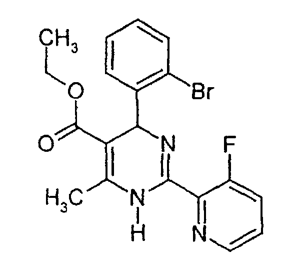

92,50 mg (500 µmol) 2-Brombenzaldehyd werden in 3,00 ml Ethanol nacheinander mit 65,0 mg Acetessigsäureethylester, 91,80 mg der Verbindung aus Beispiel III und 43,06 mg Natriumacetat vermischt und 6 Stunden gekocht. Es wird abgekühlt, eingeengt, in 2 ml l N HCl und 4 ml H₂O und Essigester gelöst, abgetrennt, die organische Phase mit 1 ml IN HCl und Wasser extrahiert und die vereinigten wäßrigen Phasen mit Ether gewaschen. Die wäßrige Phase wird mit verdünnter Ammoniaklösung basisch gestellt, mit Essigester extrahiert, es wird mit H₂O gewaschen, getrocknet und eingeengt. Es wird in wenig Ether gelöst und auskristallisiert. Es wird abgesaugt, mit Ether gewaschen, bei 60°C im Vakuum getrocknet.
DC: rein (Toluol/Essigester = 4:1)
Ausbeute: 92 mg (44 %)
Schmp.: 163-165°C

Analog der Vorschrift des Beispiels 1 werden die in Tabelle 1 aufgeführten Verbindungen hergestellt:

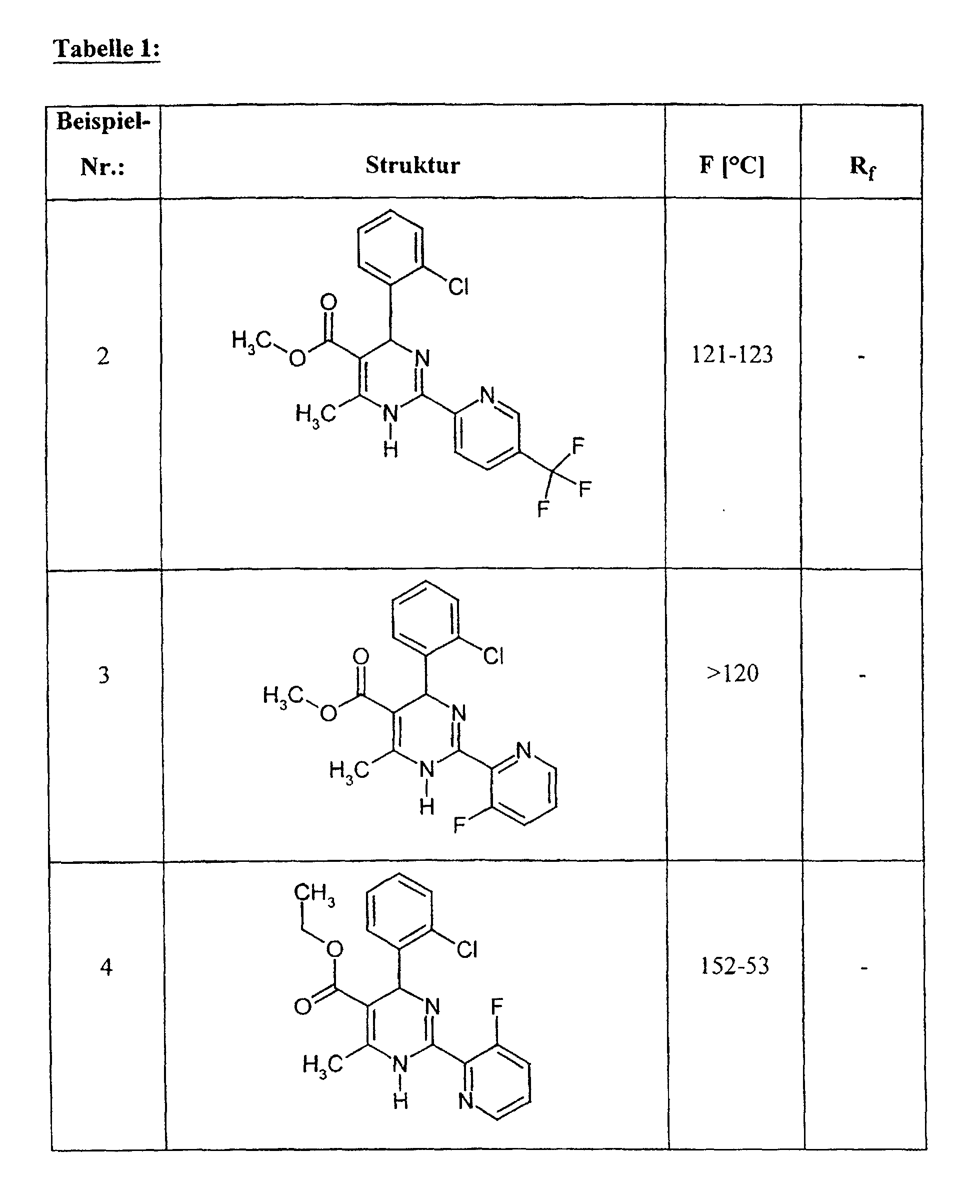

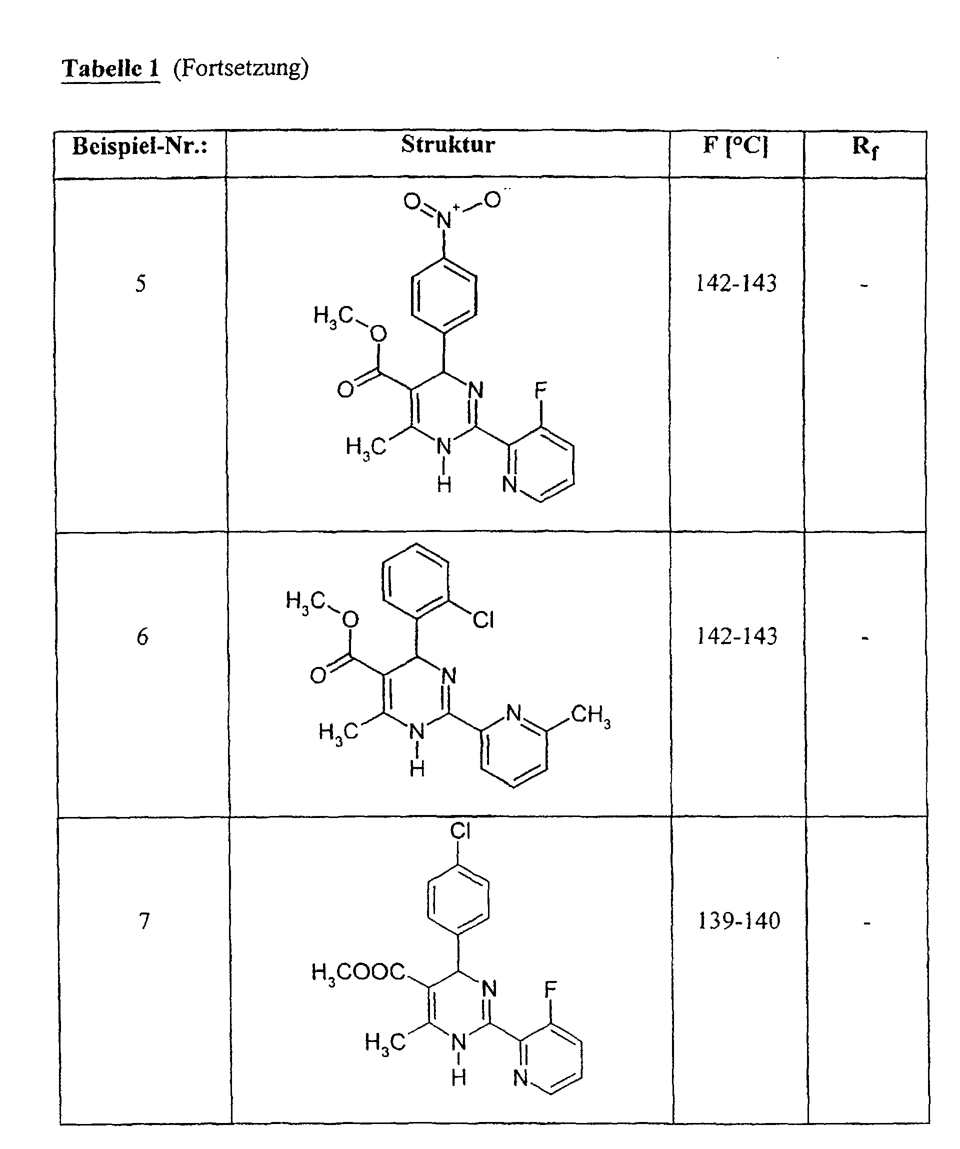

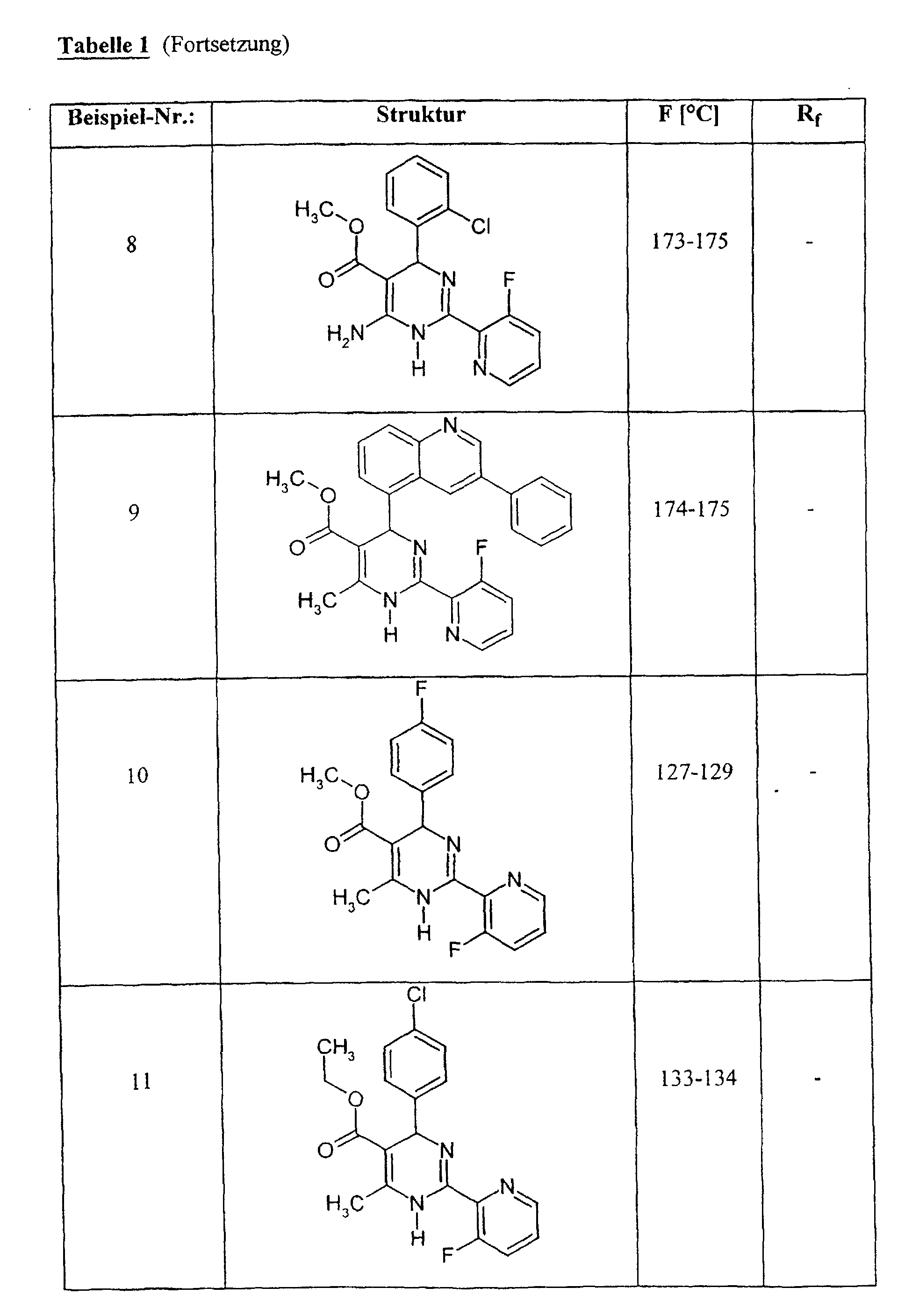

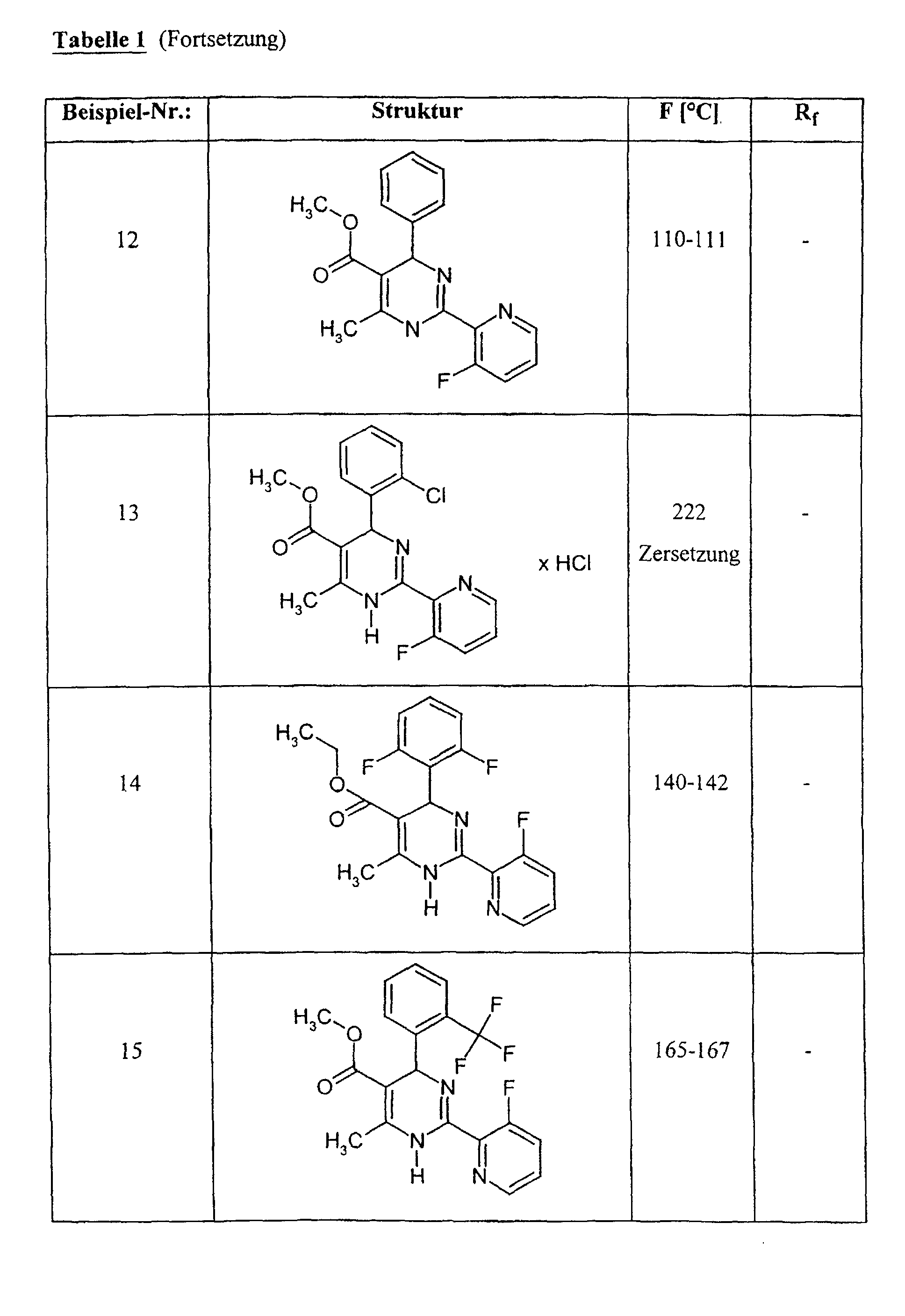

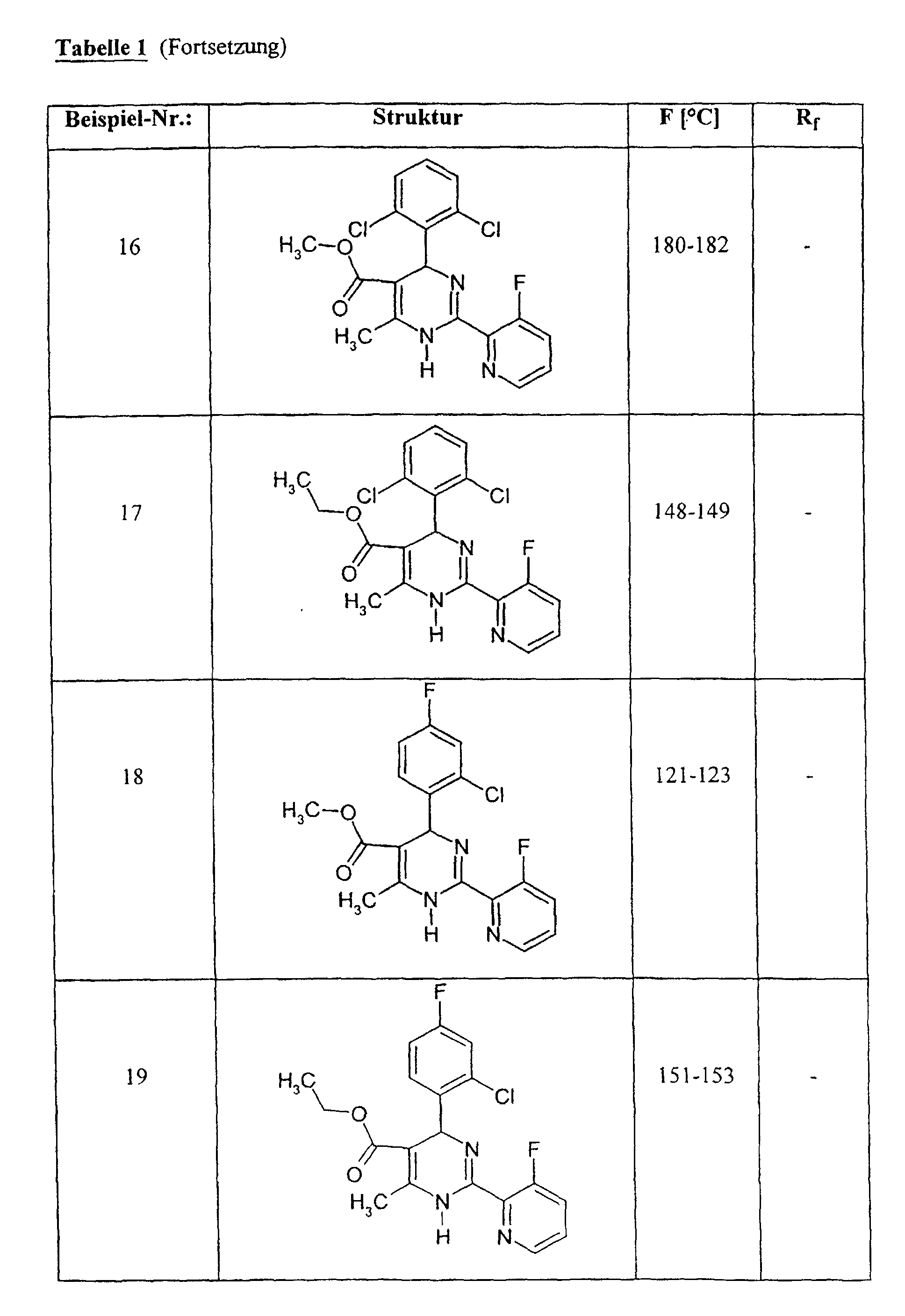

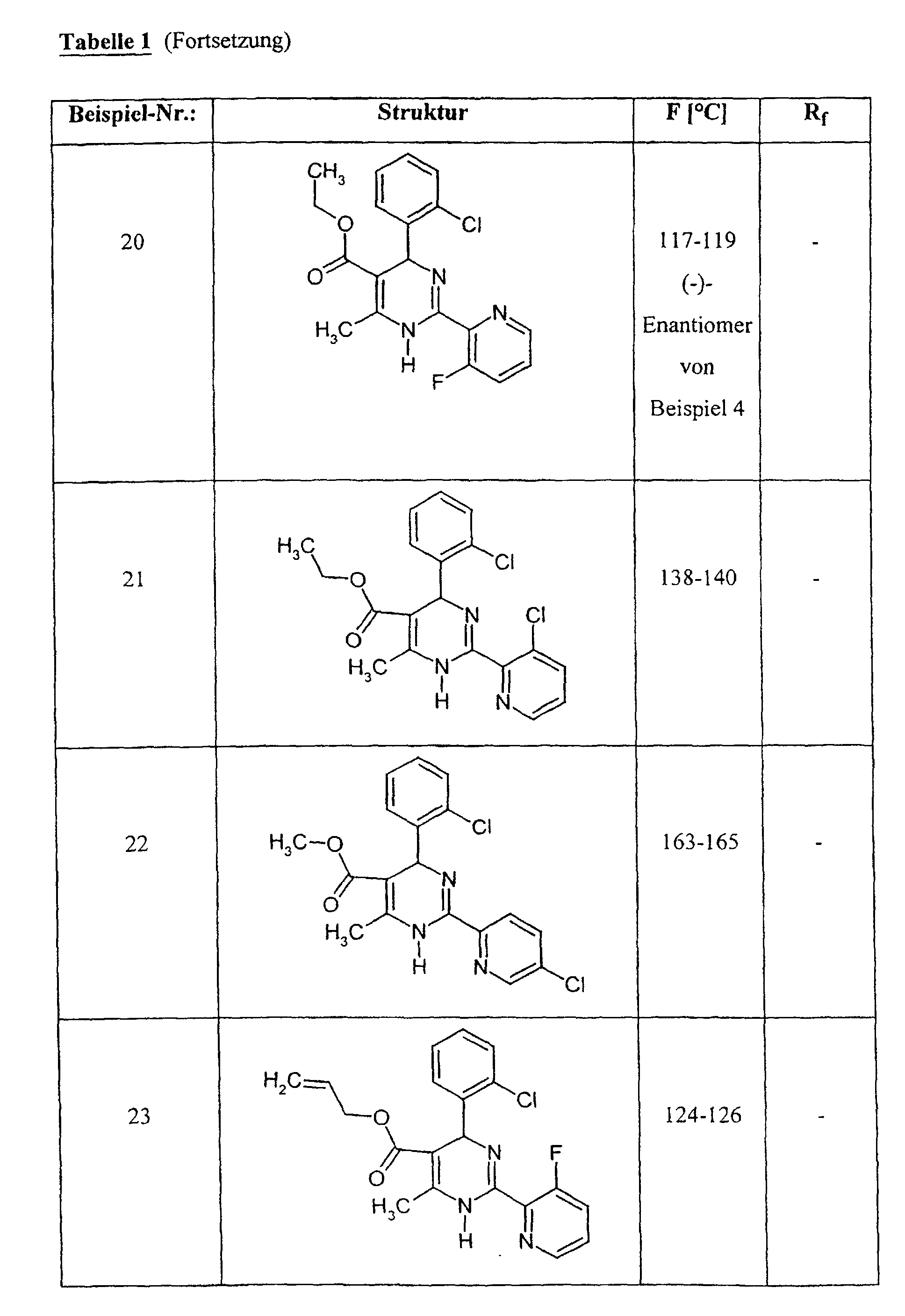

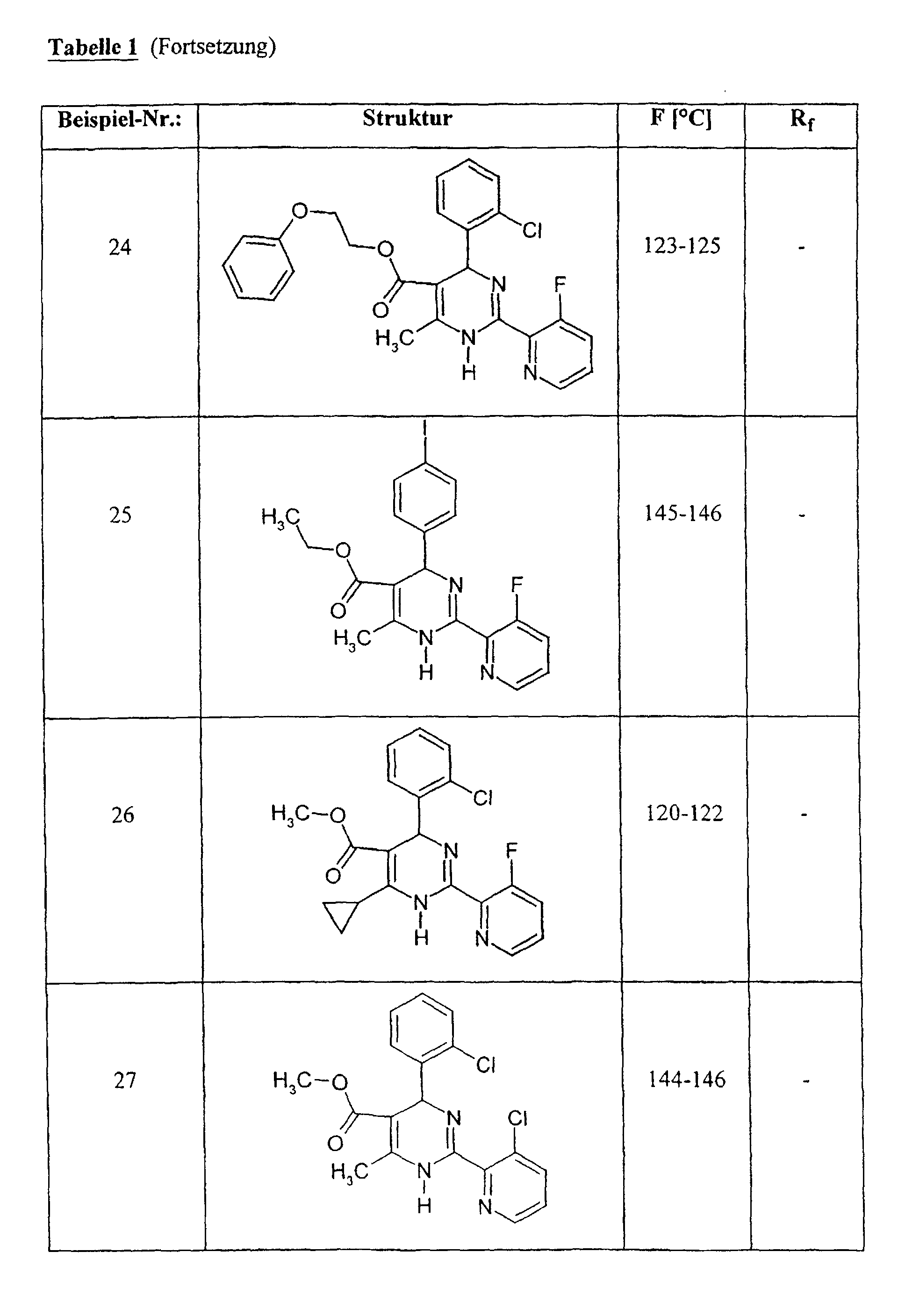

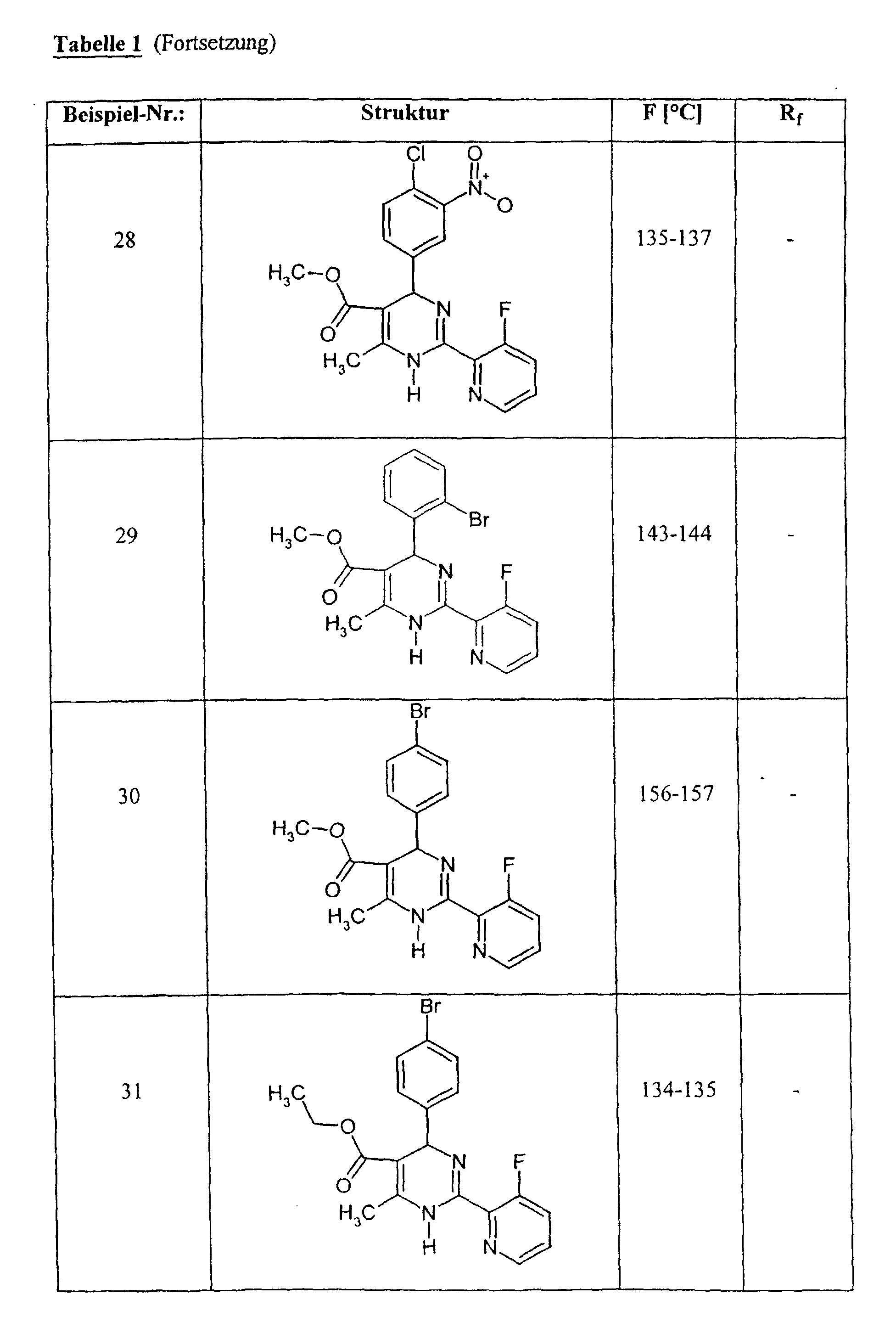

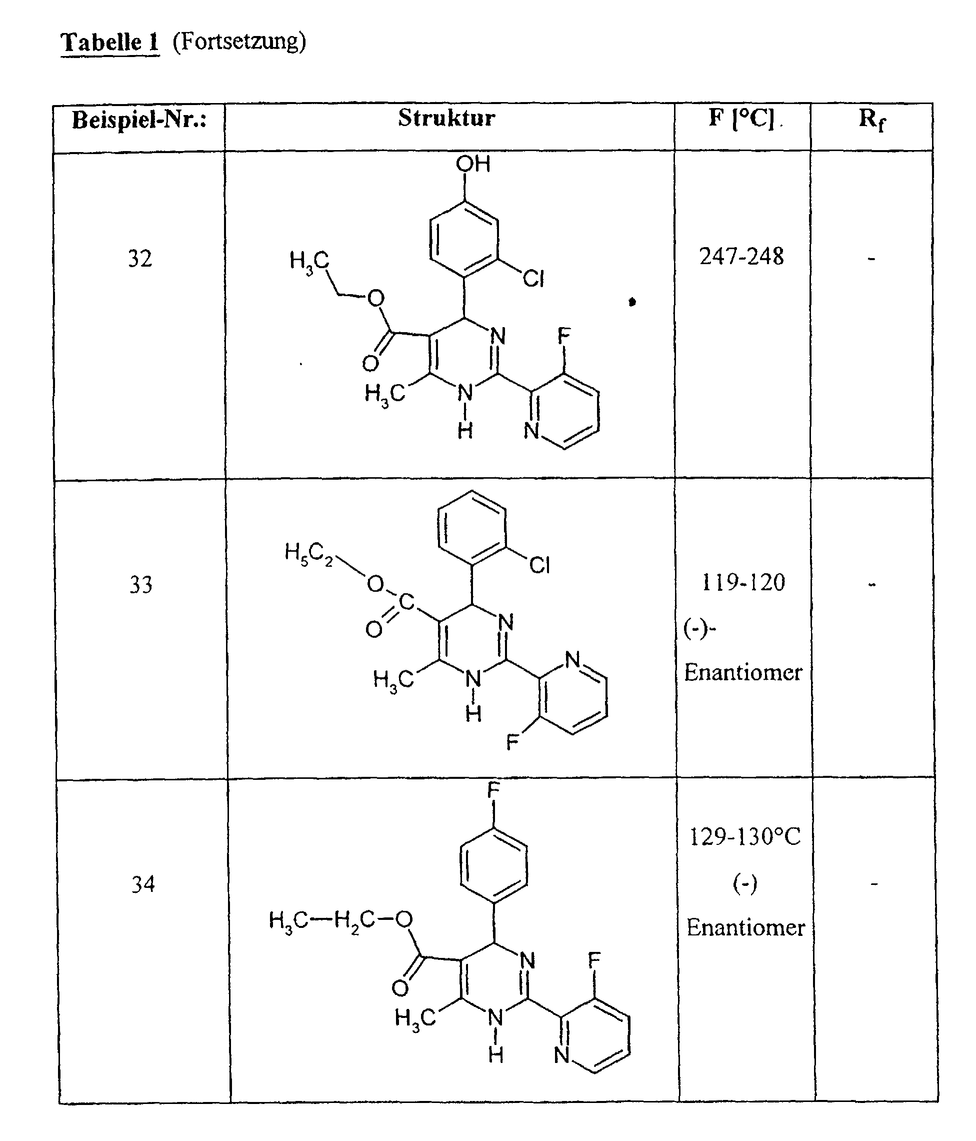

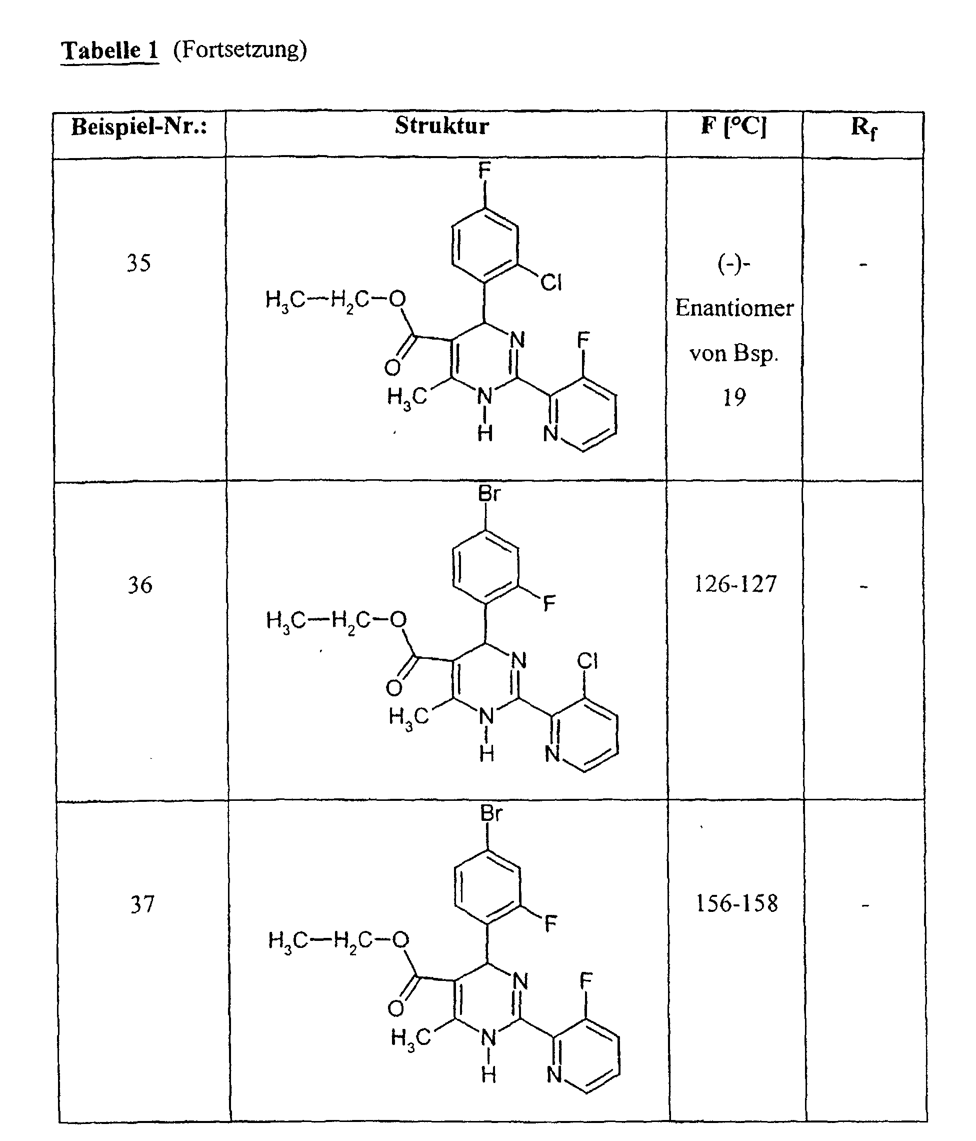

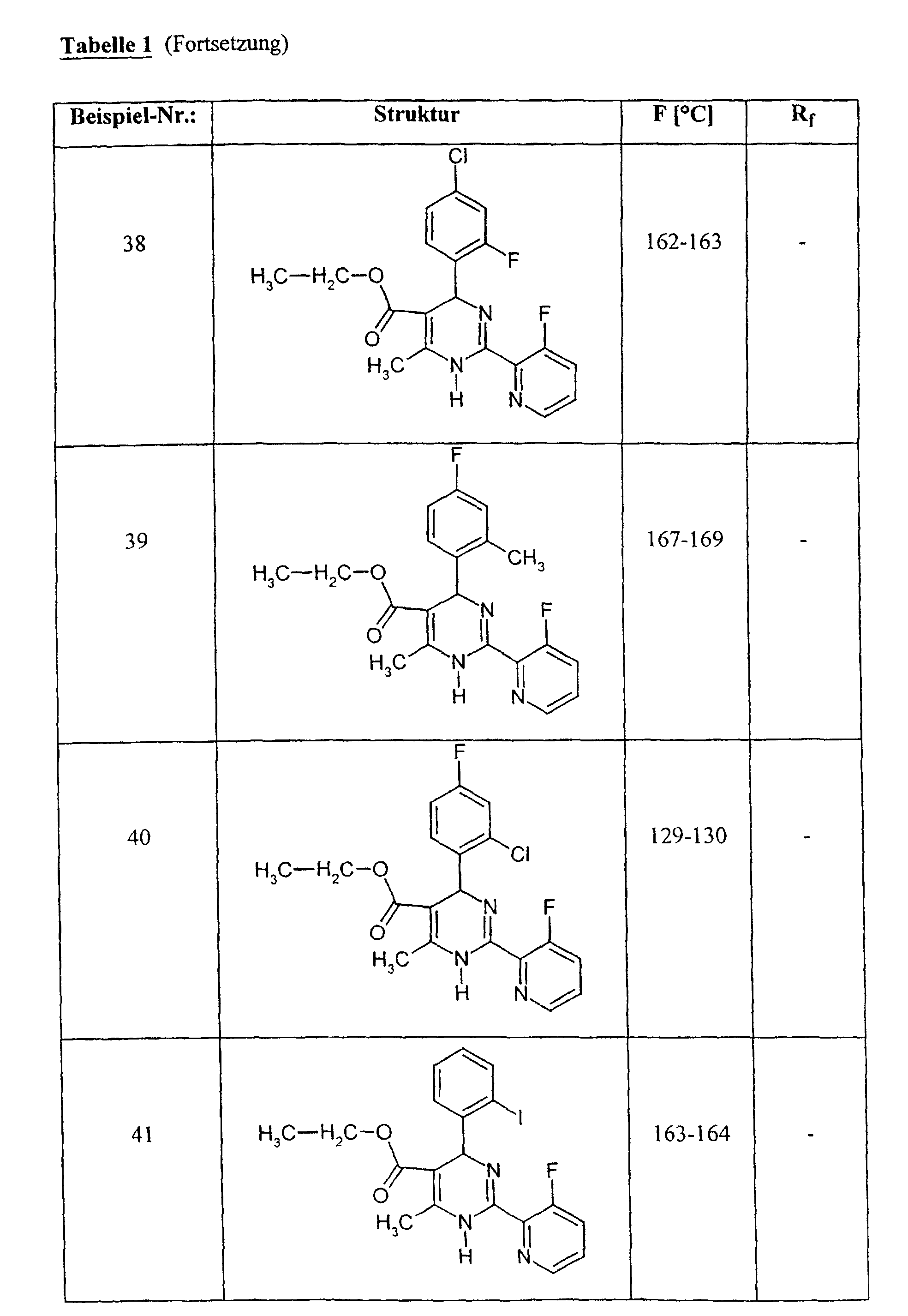

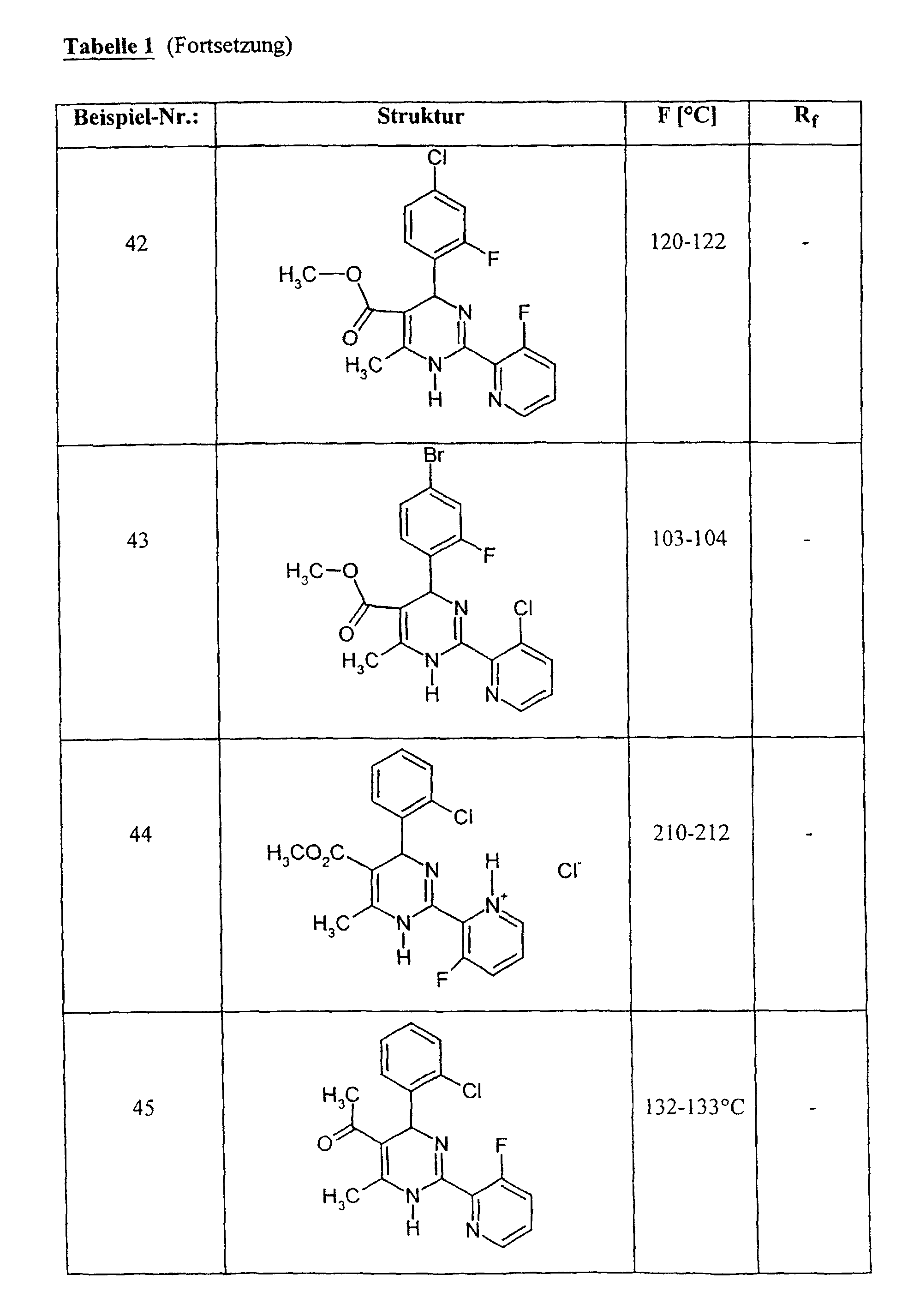

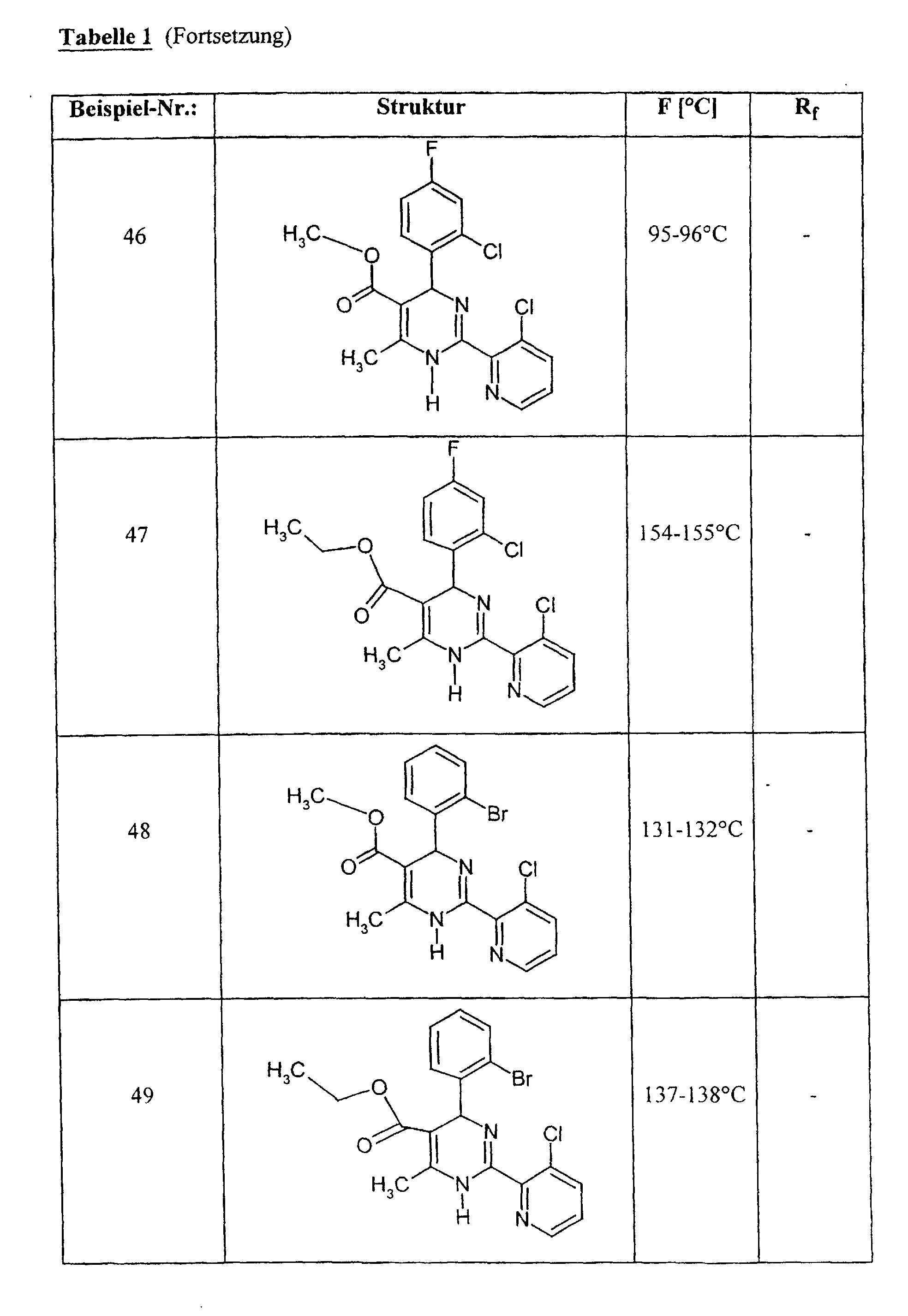

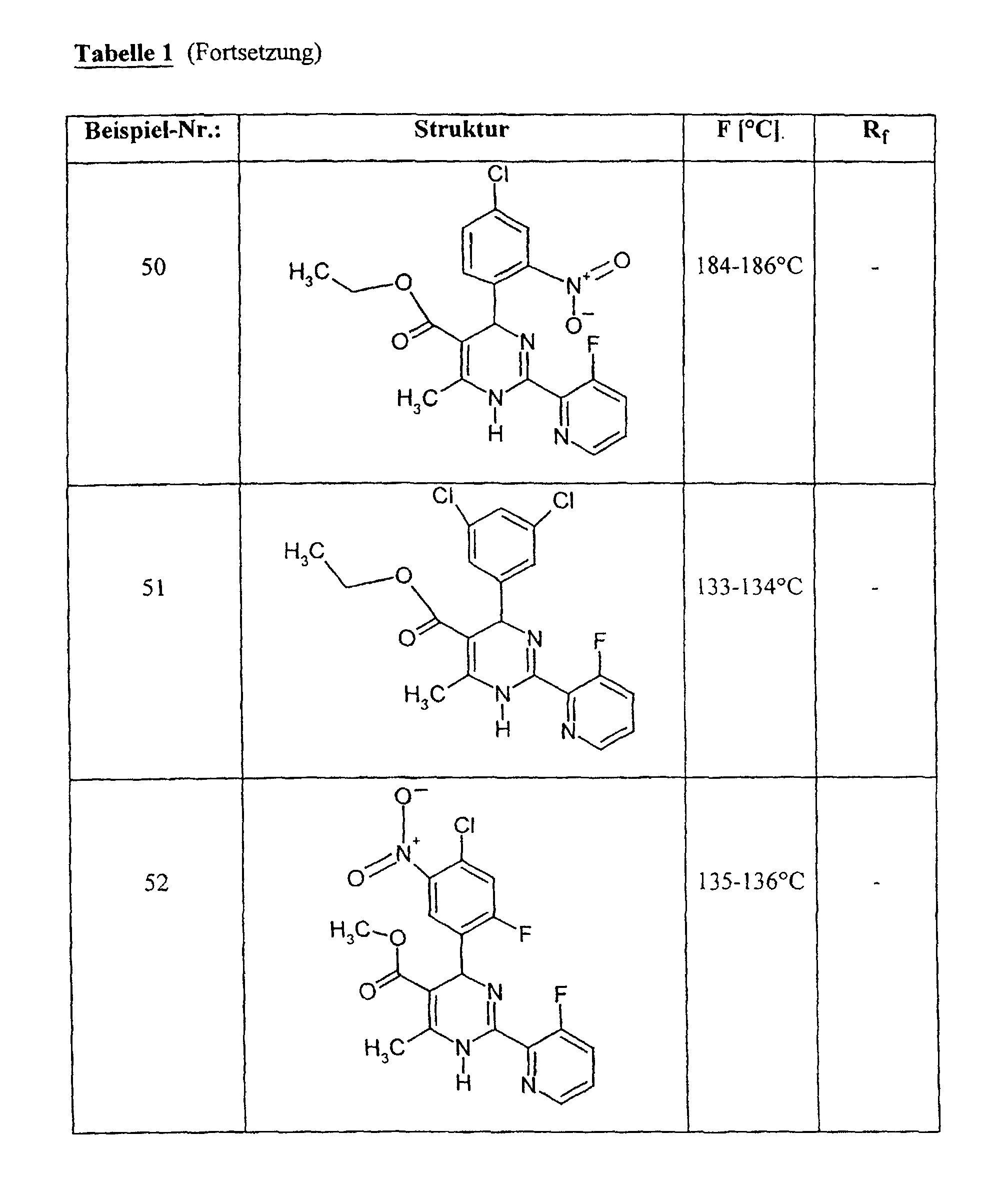

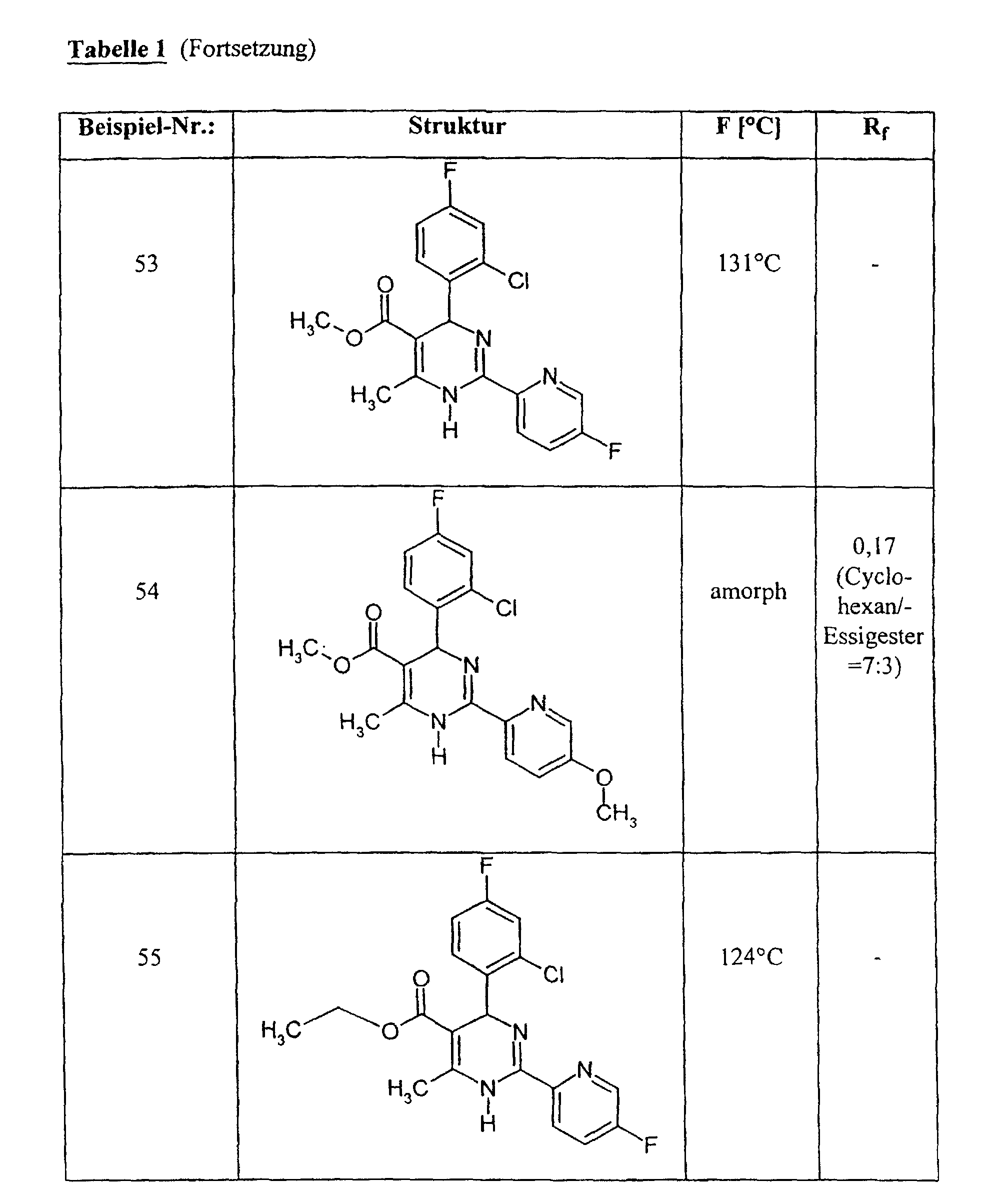

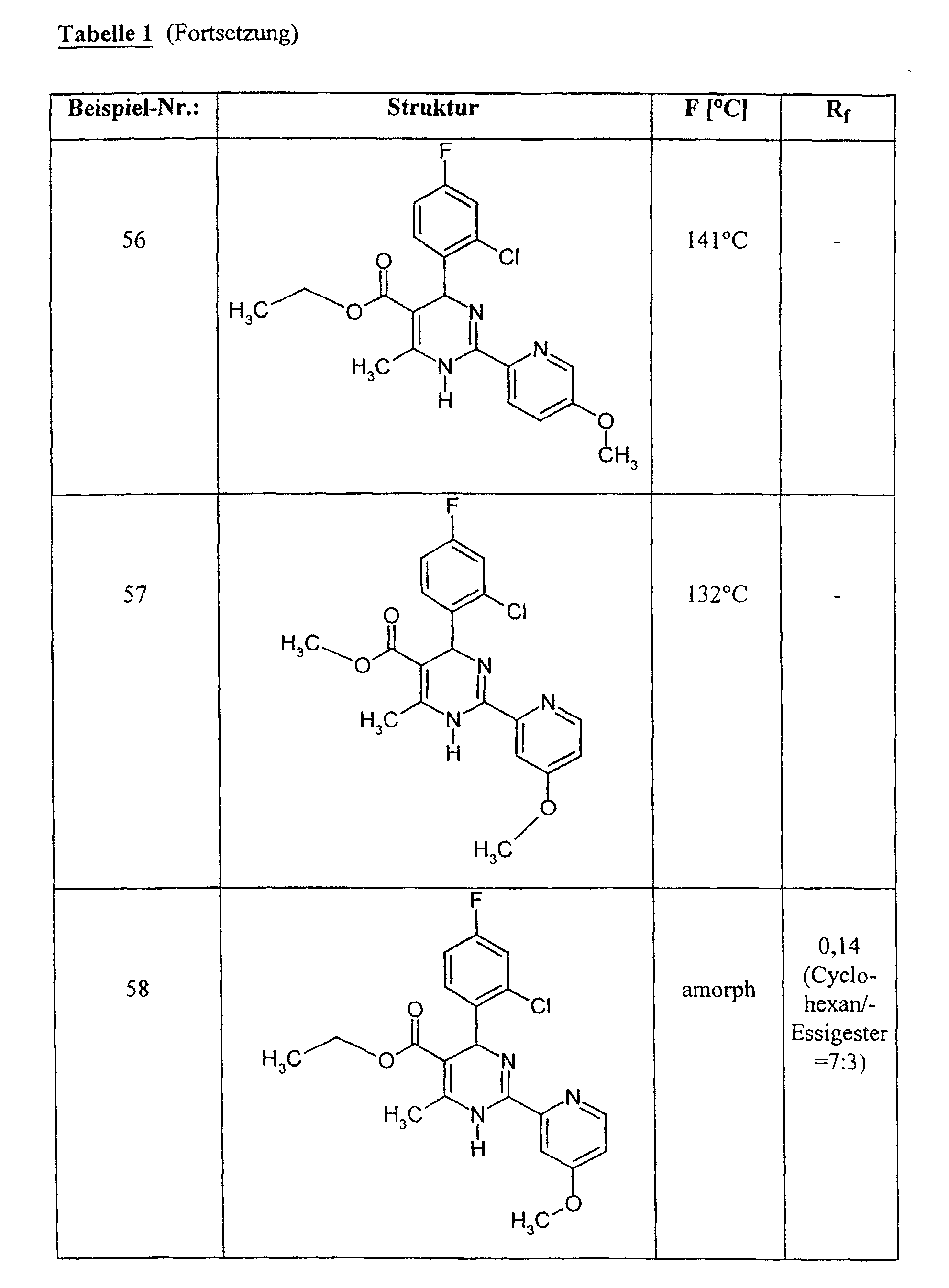

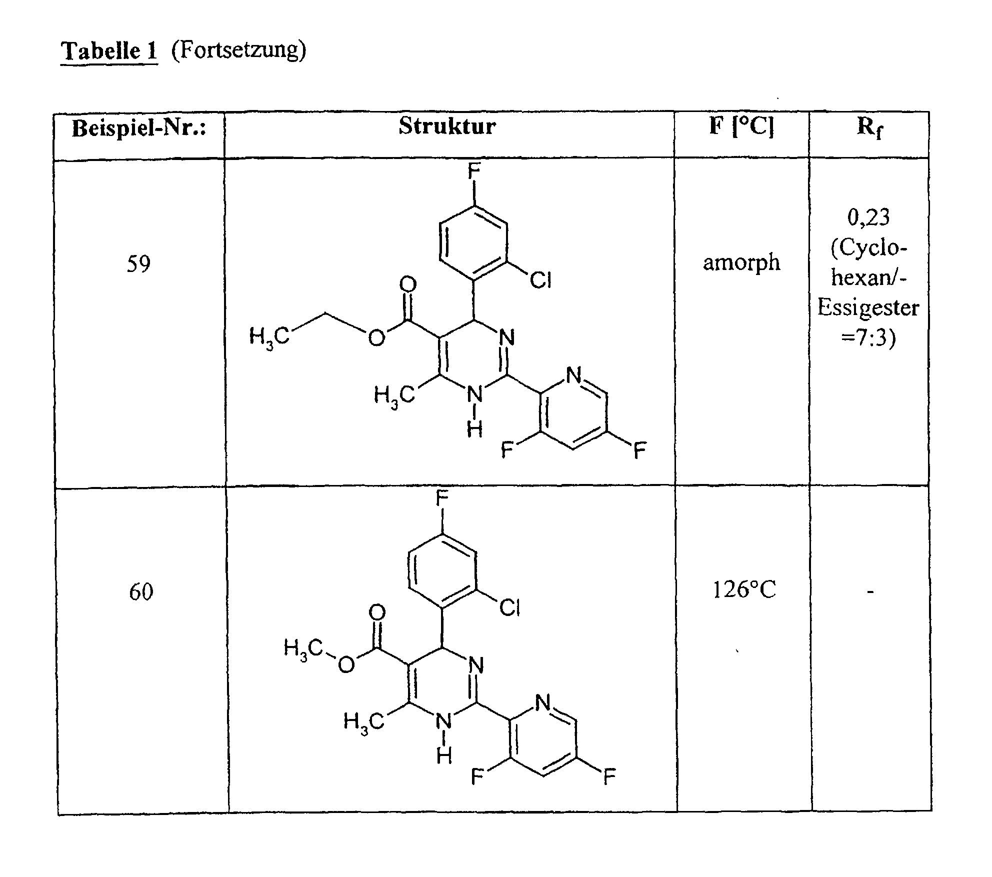

### Beispiel 61

### 4-(2-Chlor-4-fluorphenyl)-2-(3,5-difluor-2-pyridinyl)-6-methyl- 1,4-dihydropyrimidin-5-carbonsäure-methylester (s. Tabelle)

4,5 g (23,2 mmol) 3,5-Difluoro-2-pyridincarboximidamid-hydrochlorid **(Beispiel VI)** werden mit 7,7 g (30 mmol) 2-Acetyl-3-(2-chlor-4-fluorphenyl)-2-propensäuremethylester **(Beispiel VII)** und 2,3 g (27,9 mmol) Natriumacetat in 120 ml Isopropanol gelöst bzw.suspendiert und 4 h unter Rückfluß gekocht.

Nach Abkühlung auf Raumtemperatur wird von anorganischen Salzen abgesaugt und eingeengt. Der Rückstand wird in 30 ml IN HCl und 35 mL Essigester aufgenommen und die Phasen getrennt. Die Essigesterphase wird einmal mit 30 ml l N HCl nachextrahiert. Die vereinigten wäßrigen Phasen werden dreimal mit je 10 ml Diethylether ausgeschüttelt. Die wäßrige Phase wird mit NaOH alkalisch gestellt und mit Essigester ausgeschüttelt. Die organischen Phasen werden über Na₂SO₄ getrocknet und eingengt.
Man erhält 7,4 g (80 %) Produkt. (FP: 126°C)

¹H-NMR (DMSO-D₆): 2,4(s,3H), 3,5(s,3H), 6,0(s,1H), 7,2(m,1H), 7,4(m,2H), 8,0(m,1H), 8,55(d,J=2Hz,1H), 9,75(s,NH) ppm.

Nach Trennung der Enantiomeren an chiralen Säulen (Chiralpak AS von Baker, Laufmittel n-Heptan/Ethanol=8:2) konnte das (-)-Enantiomer erhalten werden.
FP: 117°C (aus Ethanol)
Spez.Dreh.: -62.8° (MeOH)

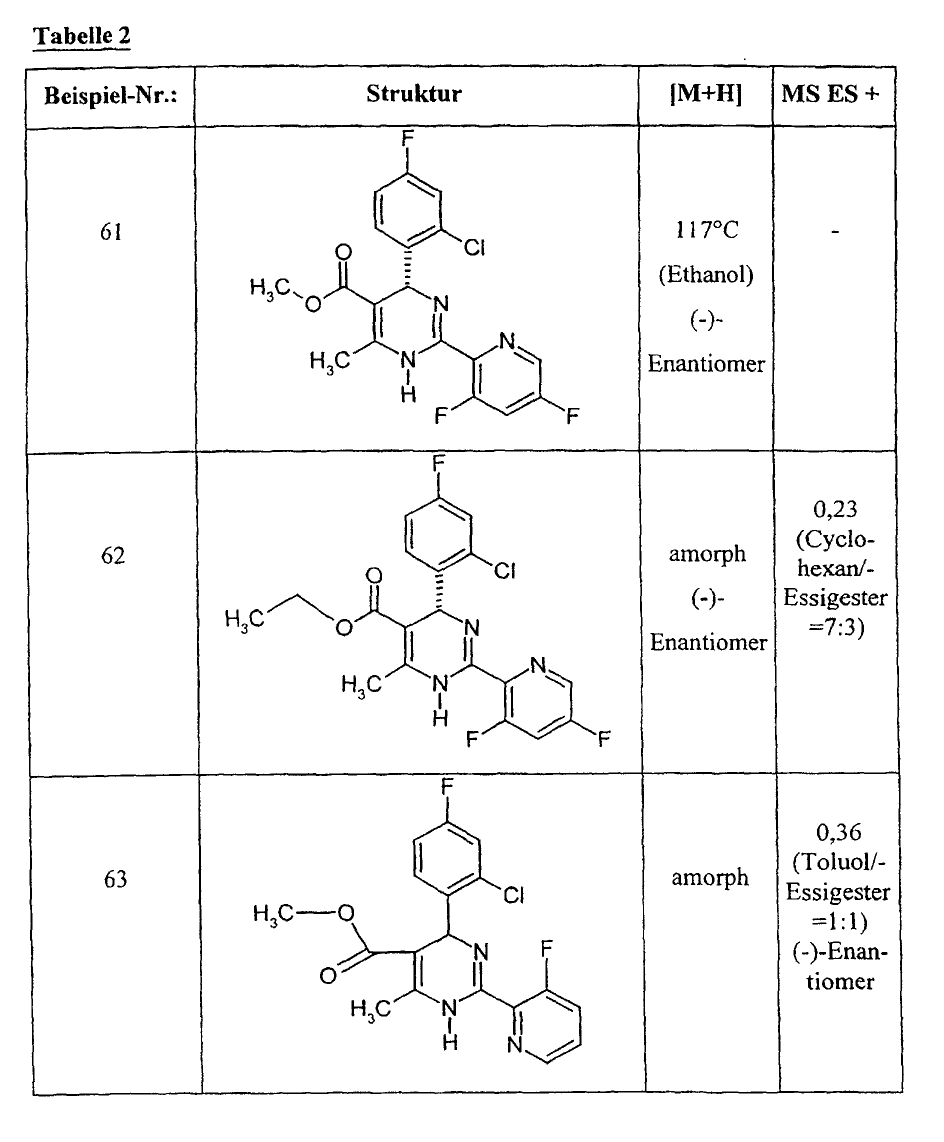

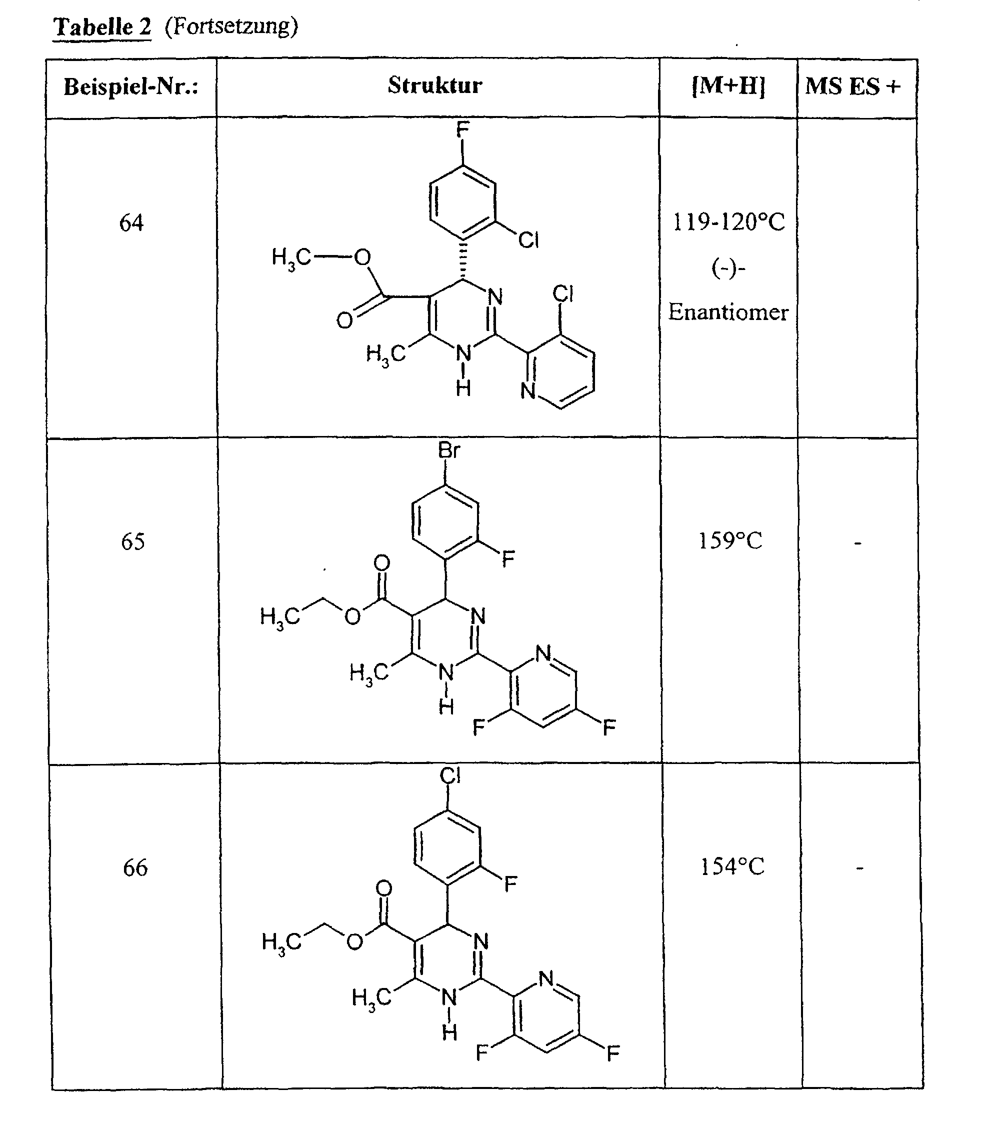

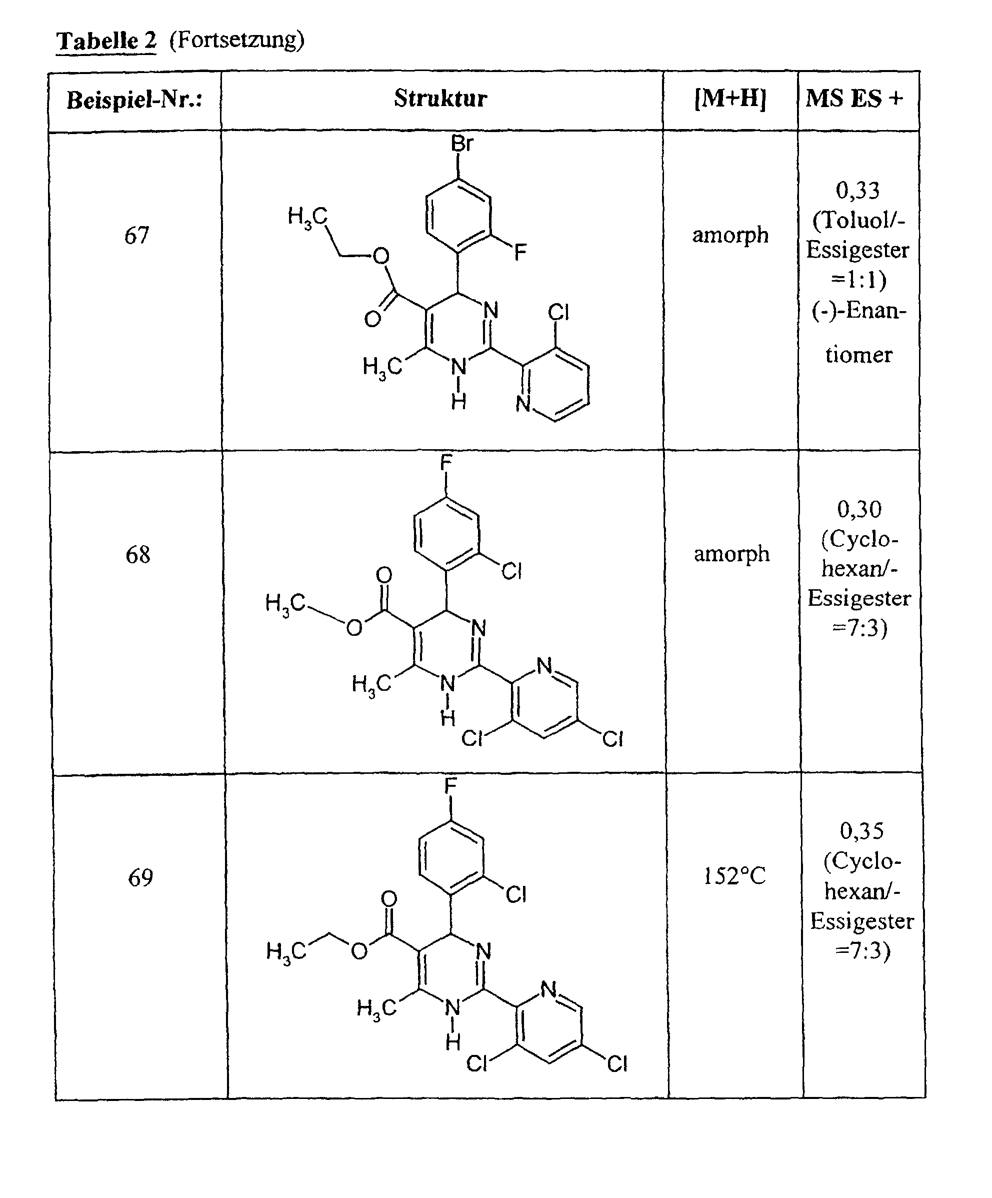

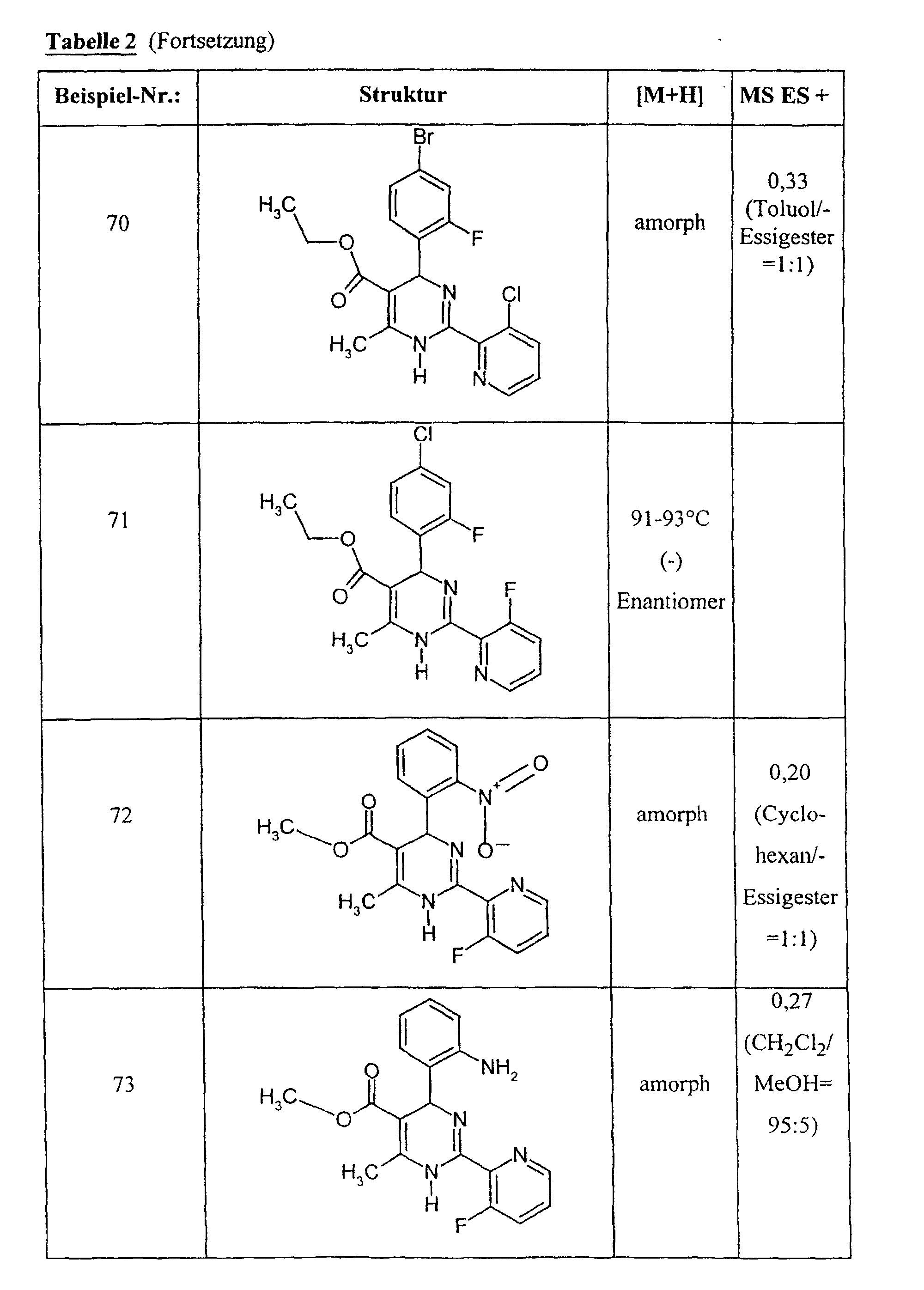

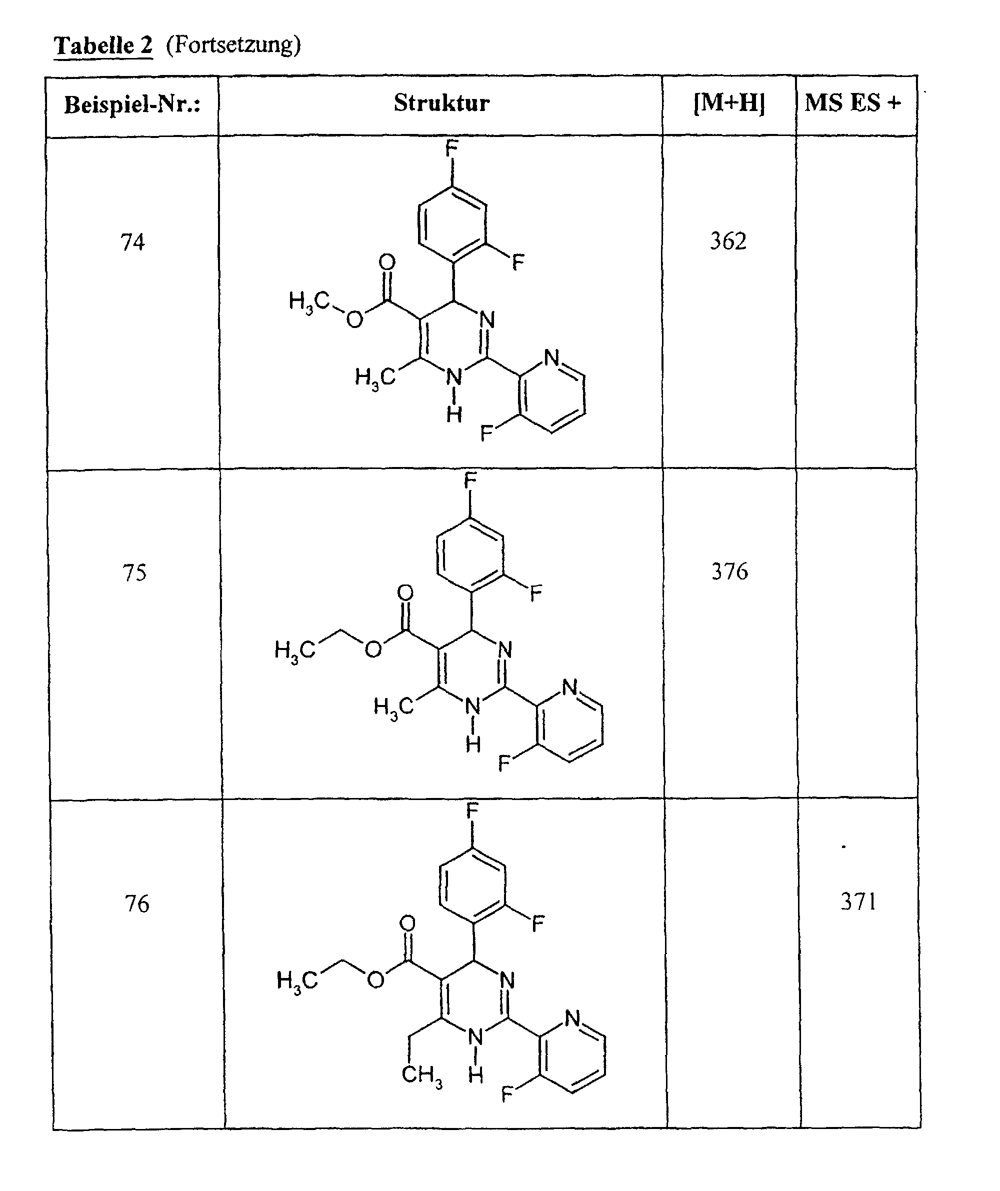

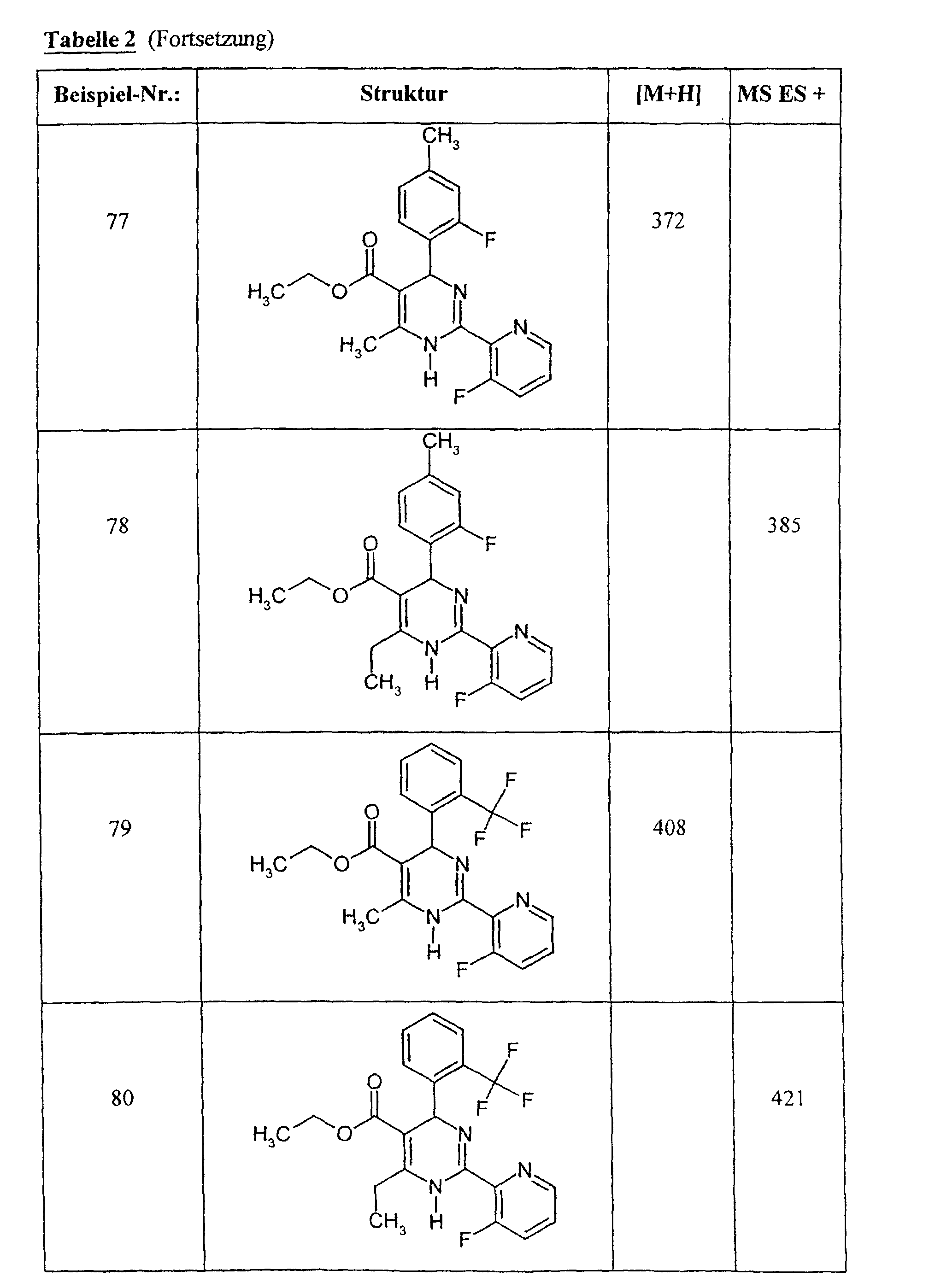

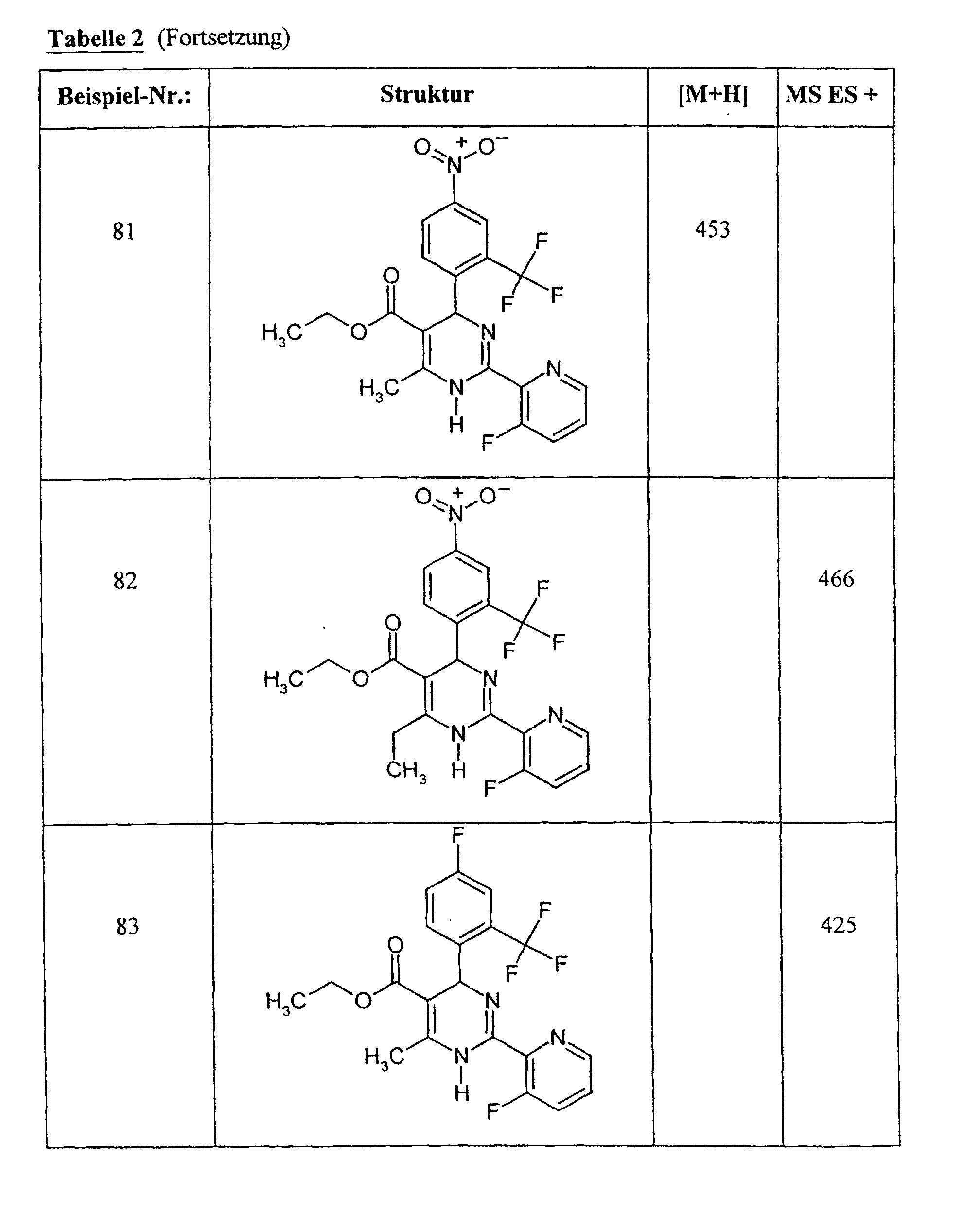

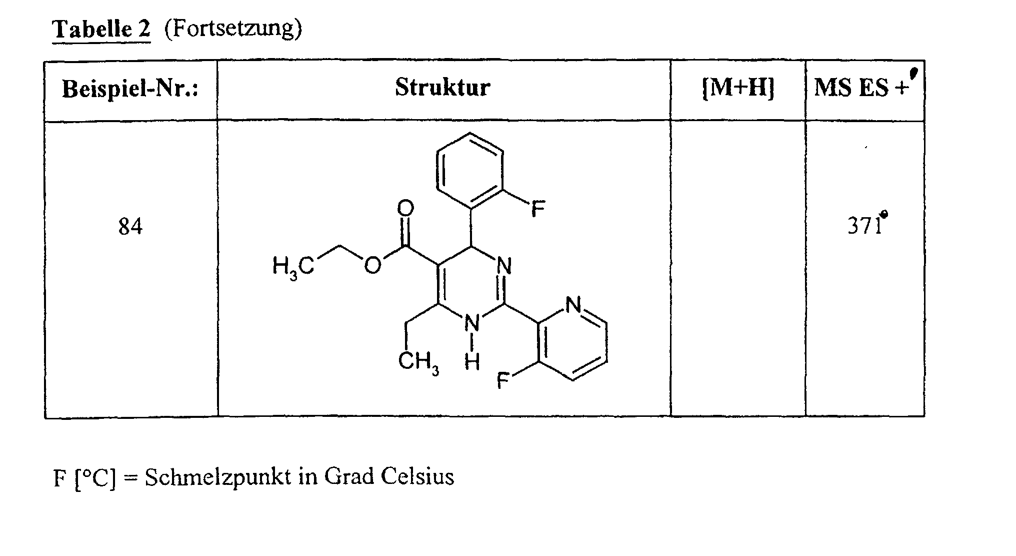

## Patentansprüche

1. Verbindungen der allgemeinen Formel (I) 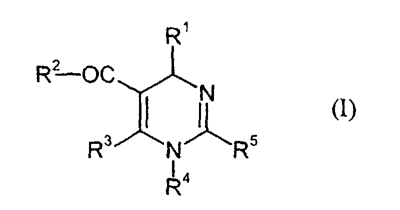 bzw. deren isomere Form (Ia) 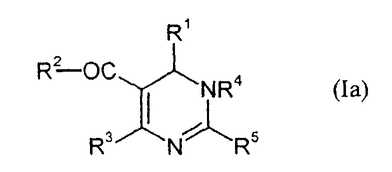 in welcher
R¹ für Phenyl, Furyl, Thienyl, Triazolyl, Pyridyl, Cycloalkyl mit 3 bis 6 Kohlenstoffatomen oder für Reste der Formeln 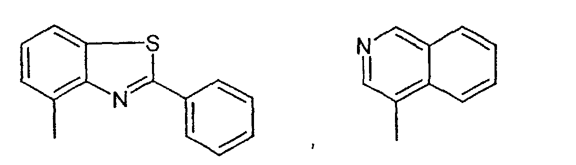 oder 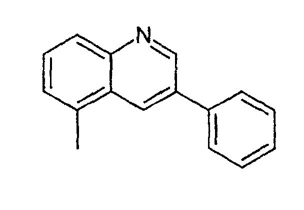 steht,
wobei die oben aufgeführten Ringsysteme gegebenenfalls einfach oder mehrfach, gleich oder verschieden durch Substituenten, ausgewählt aus der Gruppe Halogen, Trifluormethyl, Nitro, Cyano, Trifluormethoxy, Carboxyl, Hydroxyl, (C₁-C₆)-Alkoxy, (C₁-C₆)-Alkoxycarbonyl und (C₁-C₆)-Alkyl substituiert sind, das seinerseits durch Aryl mit 6 bis 10 Kohlenstoffatomen oder Halogen substituiert sein kann,
und/oder die aufgeführten Ringsysteme gegebenenfalls durch Gruppen der Formeln -S-R⁶, NR⁷R⁸, CO-NR⁹R¹⁰, SO₂-CF₃ und -A-CH₂-R¹¹ substituiert sind,
worin
R⁶ Phenyl bedeutet, das gegebenenfalls durch Halogen substituiert ist,
R⁷, R⁸, R⁹ und R¹⁰ gleich oder verschieden sind und Wasserstoff, Phenyl, Hydroxy-substituiertes Phenyl, Hydroxy, (C₁-C₆)-Acyl oder (C₁-C₆)-Alkyl bedeuten, das seinerseits durch Hydroxy, (C₁-C₆)-Alkoxycarbonyl, Phenyl oder Hydroxy substituiertes Phenyl substituiert sein kann,
A einen Rest O, S, SO oder SO₂ bedeutet,
R¹¹ Phenyl bedeutet, das gegebenenfalls ein- bis mehrfach, gleich oder verschieden durch Substituenten, ausgewählt aus der Gruppe Halogen, Nitro, Trifluormethyl, (C₁-C₆)-Alkyl und (C₁-C₆)-Alkoxy substituiert ist,
R² für einen Rest der Formel -XR¹² oder -NR¹³R¹⁴ steht,
worin
X eine Bindung oder Sauerstoff bedeutet,
R¹² Wasserstoff, geradkettiges oder verzweigtes (C₁-C₆)-Alkoxycarbonyl oder einen geradkettigen, verzweigten oder cyclischen, gesättigten oder ungesättigten (C₁-C₈)-Kohlenwasserstoffrest bedeutet, der gegebenenfalls eine oder zwei gleiche oder verschiedene Heterokettenglieder aus der Gruppe O, CO, NH, -NH-(C₁-C₄)-Alkyl, -N-((C₁-C₄)-Alkyl)₂, S und SO₂ enthält und der gegebenenfalls substituiert ist durch Halogen, Nitro, Cyano, Hydroxy, Aryl mit 6 bis 10 Kohlenstoffatomen oder Aralkyl mit 6 bis 10 Kohlenstoffatomen, Heteroaryl oder einer Gruppe der Formel -NR¹⁵R¹⁶,
worin
R¹⁵ und R¹⁶ gleich oder verschieden sind und Wasserstoff, Benzyl oder (C₁-C₆)-Alkyl bedeuten,
R¹³ und R¹⁴ gleich oder verschieden sind und Wasserstoff, (C₁-C₆)-Alkyl oder Cycloalkyl mit 3 bis 6 Kohlenstoffatomen bedeuten,
R³ für Wasserstoff, Amino oder
für einen Rest der Formel 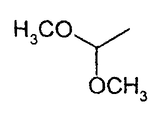 steht oder
für Formyl, Cyano, Trifluormethyl oder Pyridyl steht, oder
für einen geradkettigen, verzweigten oder cyclischen, gesättigten oder ungesättigten Kohlenwasserstoffrest mit bis zu 8 Kohlenstoffatomen steht, der gegebenenfalls ein- oder mehrfach, gleich oder verschieden durch Aryloxy mit 6 bis 10 Kohlenstoffatomen, Azido, Cyano, Hydroxy, Carboxyl, (C₁-C₆)-Alkoxycarbonyl, einen 5- bis 7-gliedrigen heterocyclischen Ring, (C₁-C₆)-Alkylthio oder (C₁-C₆)-Alkoxy substituiert ist, das seinerseits durch Azido oder Amino substituiert sein kann,
und/oder durch Triazolyl substituiert ist, das seinerseits bis zu 3-fach durch (C₁-C₆)-Alkoxycarbonyl substituiert sein kann,
und/oder durch Gruppen der Formeln -OSO₂-CH₃ oder (CO)ₐ-NR¹⁷R¹⁸ substituiert sein kann,
worin
a eine Zahl 0 oder 1 bedeutet,
R¹⁷ und R¹⁸ gleich oder verschieden sind und Wasserstoff oder Aryl, Aralkyl mit 6 bis 10 Kohlenstoffatomen bedeuten,
oder (C₁-C₆)-Alkyl bedeuten, das gegebenenfalls durch (C₁-C₆)-Alkoxycarbonyl, Hydroxyl, Phenyl oder Benzyl substituiert ist, wobei Phenyl oder Benzyl gegebenenfalls ein- oder mehrfach, gleich oder verschieden durch Hydroxy, Carboxyl, (C₁-C₆)-Alkyl oder (C₁-C₆)-Alkoxy substituiert sind,
oder (C₁-C₆)-Alkyl gegebenenfalls durch Gruppen der Formeln NH-CO-CH₃ oder NH-CO-CF₃ substituiert ist,
oder
R¹⁷ und R¹⁸ gemeinsam mit dem Stickstoffatom einen Morpholin-, Piperidinyl- oder Pyrrolidinylring bilden,
oder
R³ für Phenyl steht, das gegebenenfalls durch Methoxy substituiert ist,
oder
R² und R³ gemeinsam einen Rest der Formel 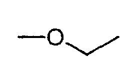 bilden
R⁴ für Wasserstoff, (C₁-C₄)-Alkyl, (C₂-C₄)-Alkenyl, Benzoyl oder für Acyl mit 2 bis 6 Kohlenstoffatomen steht,
R⁵ für Pyridyl steht, das bis zu 3-fach gleich oder verschieden durch Halogen, Hydroxy, Cyano, Trifluormethyl, (C₁-C₆)-Alkoxy, (C₁-C₆)-Alkyl, (C₁-C₆)-Alkylthio, Carbalkoxy, (C₁-C₆)-Acyloxy, Amino, Nitro, Mono- oder (C₁-C₆)-Dialkylamino substituiert ist,
und deren Salze.

2. Verbindungen der allgemeinen Formeln (I) bzw. (Ia) gemäß Anspruch 1, in welcher
R¹ für Phenyl, Furyl, Thienyl, Pyridyl, Cyclopentyl oder Cyclohexyl oder für Reste der Formeln 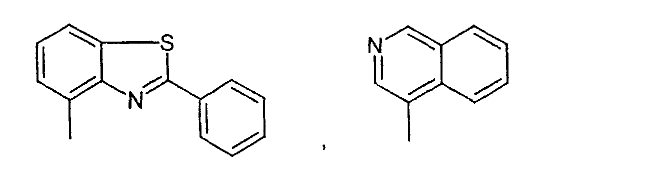 oder 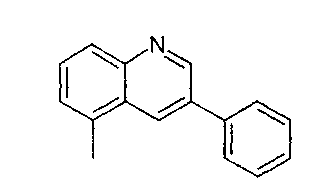 steht,
wobei die oben aufgeführten Ringsysteme gegebenenfalls ein- oder 2-fach gleich oder verschieden durch Substituenten, ausgewählt aus der Gruppe Halogen, Trifluormethyl, Nitro. SO₂-CF₃, Methyl, Cyano, Amino, Trifluormethoxy. Hydroxy, Carboxyl, Methoxycarbonyl und Resten der Formeln -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -S-pCl-C₆H₄ substituiert sind,
R² für einen Rest der Formel -XR¹² oder -NR¹³R¹⁴ steht,
worin
X eine Bindung oder ein Sauerstoffatom bedeutet,
R¹² Wasserstoff, (C₁-C₄)-Alkenyl, (C₁-C₄)-Alkoxycarbonyl oder (C₁-C₄)-Alkyl bedeutet, die gegebenenfalls durch Pyridyl, Cyano, Phenoxy, Benzyl oder durch einen Rest der Formel -NR¹⁵R¹⁶ substituiert sind,
worin
R¹⁵ und R¹⁶ gleich oder verschieden sind und Wasserstoff, Benzyl oder (C₁-C₄)-Alkyl bedeuten,
R¹³ und R¹⁴ gleich oder verschieden sind und Wasserstoff, (C₁-C₄)-Alkyl oder Cyclopropyl bedeuten,
R³ für Wasserstoff, Amino oder einen Rest der Formel 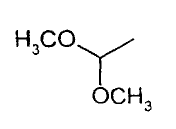 steht,
oder
für Formyl, Cyano, Trifluormethyl, Cyclopropyl oder Pyridyl steht. oder
für (C₁-C₄)-Alkyl steht, das gegebenenfalls durch Halogen, (C₁-C₄)-Alkoxycarbonyl, Hydroxy oder durch Triazolyl substituiert ist, das seinerseits bis zu 3-fach durch (C₁-C₄)-Alkoxycarbonyl substituiert sein kann,
und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
C₁-C₄-Alkyl bedeuten, das gegebenenfalls durch (C₁-C₄)-Alkoxycarbonyl, Hydroxyl, Phenyl oder Benzyl substituiert ist, wobei Phenyl oder Benzyl gegebenenfalls ein- oder mehrfach gleich oder verschieden durch Hydroxy, Carboxy, (C₁-C₄)-Alkyl oder (C₁-C₄)-Alkoxy substituiert sind,
und/oder (C₁-C₄)-Alkyl gegebenenfalls durch Reste der Formeln -NH-CO-CH₃ oder -NH-CO-CF₃ substituiert ist,
oder
R¹⁷ und R¹⁸ gemeinsam mit dem Stickstoffatom einen Morpholin-, Piperidinyl- oder Pyrrolidinylring bilden,
oder
R³ für Phenyl steht, das gegebenenfalls durch Methoxy substiuiert ist,
oder
R² und R³ gemeinsam einen Rest der Formel 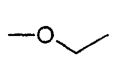 bilden,
R⁴ für Wasserstoff, Methyl, Benzoyl oder Acetyl steht,
R⁵ für Pyridyl steht, das bis zu 2-fach gleich oder verschieden durch Fluor, Chlor, Brom, (C₁-C₄)-Alkoxy oder (C₁-C₄)-Alkyl substituiert ist,
und deren Salze.

3. Verbindungen der allgemeinen Formeln (I) und (Ia) gemäß Anspruch 1
in welcher
R¹ für Phenyl, Furyl, Thienyl, Pyridyl, Cyclopentyl, Cyclohexyl oder für Reste der Formeln 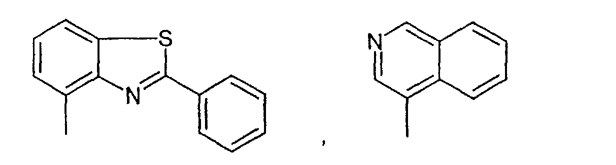 oder 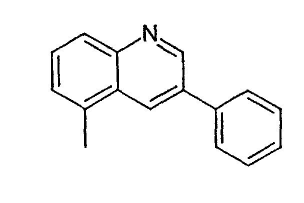 steht,
wobei die oben aufgeführten Ringsysteme gegebenenfalls bis zu 2-fach, gleich oder verschieden durch Substituenten, ausgewählt aus der Gruppe Fluor, Chlor, Brom, Jod, Hydroxy, Trifluormethyl, Amino, Nitro, SO₂-CF₃, Methyl, Cyano, Trifluormethoxy, Carboxyl. Methoxycarbonyl und Resten der Formeln -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ oder -S-pCl-C₆H₄ substituiert sind,
R² für einen Rest der Formel -XR¹² oder -NR¹³R¹⁴ steht,
worin
X eine Bindung oder ein Sauerstoffatom bedeutet,
R¹² Wasserstoff, (C₁-C₃)-Alkenyl, (C₁-C₄)-Alkoxycarbonyl oder (C₁-C₄)-Alkyl bedeutet, die gegebenenfalls durch Pyridyl, Cyano, Phenoxy, Benzyl oder durch einen Rest der Formel -NR¹⁵R¹⁶ substituiert sind,
worin
R¹⁵ und R¹⁶ gleich oder verschieden sind und Wasserstoff oder Methyl bedeuten,
R¹³ und R¹⁴ gleich oder verschieden sind und Wasserstoff, (C₁-C₃)-Alkyl oder Cyclopropyl bedeuten,
R³ für Wasserstoff, Amino oder für einen Rest der Formel 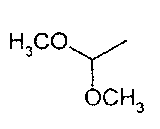 steht,
oder
für Formyl, Cyano. Trifluormethyl, Cyclopropyl oder Pyridyl steht, oder
für (C₁-C₄)-Alkyl steht, das gegebenenfalls durch Fluor, Chlor, (C₁-C₃)-Alkoxycarbonyl, Hydroxy oder durch Triazolyl substituiert ist, das seinerseits bis zu 3-fach durch (C₁-C₃)-Alkoxycarbonyl substituiert sein kann,
und/oder Alkyl gegebenenfalls durch Gruppen der Formeln -OSO₂-CH₃ oder (CO)ₐ-NR¹⁷R¹⁸ substituiert ist,
worin
a eine Zahl 0 oder 1 bedeutet,
R¹⁷ und R¹⁸ gleich oder verschieden sind und Wasserstoff, Phenyl oder Benzyl bedeuten, oder
(C₁-C₃)-Alkyl bedeuten, das gegebenenfalls durch (C₁-C₃)-Alkoxycarbonyl, Hydroxyl, Phenyl oder Benzyl substituiert ist, wobei Phenyl oder Benzyl gegebenenfalls ein- oder zweifach, gleich oder verschieden durch Hydroxy, Carboxy, (C₁-C₃)-Alkyl oder (C₁-C₃)-Alkoxy substituiert sind,
und/oder (C₁-C₄)-Alkyl gegebenenfalls durch Reste der Formeln -NH-CO-CH₃ oder -NH-CO-CF₃ substituiert ist,
oder
R¹⁷ und R¹⁸ gemeinsam mit dem Stickstoffatom einen Morpholin-, Piperidinyl- oder Pyrrolidinylring bilden,
oder
R³ für Phenyl steht, das gegebenenfalls durch Methoxy substiuiert ist,
oder
R² und R³ gemeinsam einen Rest der Formel 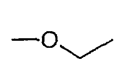 bilden,
R⁴ für Wasserstoff, Methyl, Benzoyl oder Acetyl steht,
R⁵ für Pyridyl steht, das bis zu 2-fach gleich oder verschieden durch Fluor, Chlor, (C₁-C₃)-Alkoxy oder (C₁-C₃)-Alkyl substituiert ist,
und deren Salze.

4. Verbindungen der allgemeinen Formeln (I) bzw. (Ia) gemäß Anspruch 1,
in welcher
R¹ für Phenyl steht, das gegebenenfalls bis zu 2-fach gleich oder verschieden durch Fluor, Chlor, Brom, Jod, Methyl oder Nitro substituiert ist,
R² für -XR¹² steht, worin X für Sauerstoff und R¹² für geradkettiges oder verzweigtes Alkyl mit bis zu 4 Kohlenstoffatomen steht,
R³ für Methyl, Ethyl oder Cyclopropyl steht,
oder
R² und R³ gemeinsam einen Rest der Formel 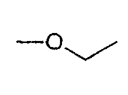 bilden,
R⁴ für Wasserstoff oder Acetyl steht,
und
R⁵ für Pyridyl steht, das bis zu zweifach, gleich oder verschieden durch durch Fluor oder Chlor substituiert ist,
und deren Salze.

5. Verbindungen der allgemeinen Formel (I) bzw. (Ia) gemäß irgend einem der Ansprüche 1 bis 4, in welcher R⁵ für 2-Pyridyl steht, daß durch 1 oder 2 Fluoratome substituiert ist.

6. Verbindungen nach Anspruch 1 der folgenden Strukturen

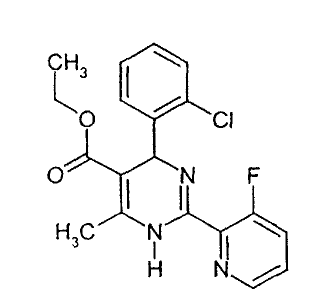

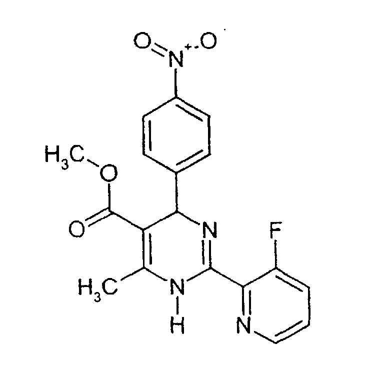

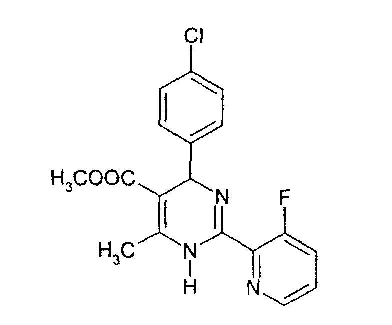

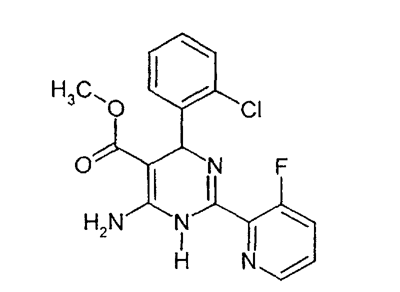

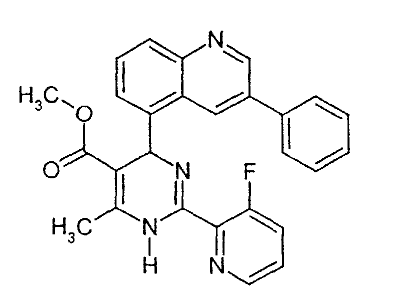

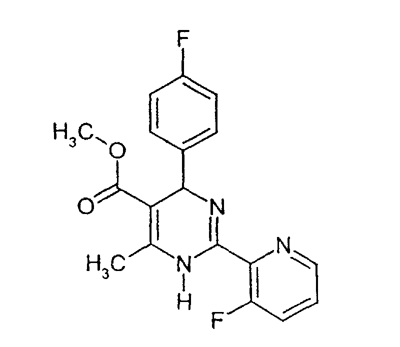

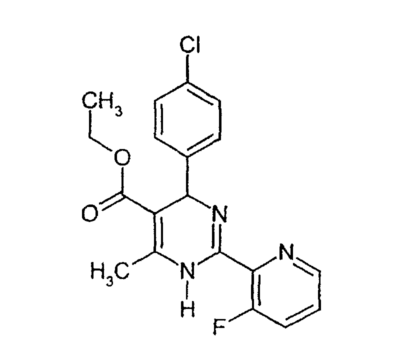

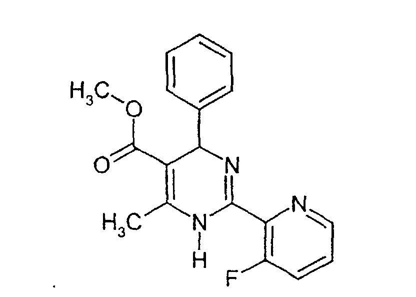

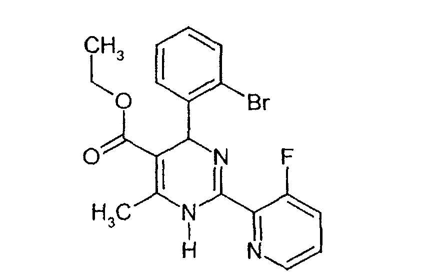

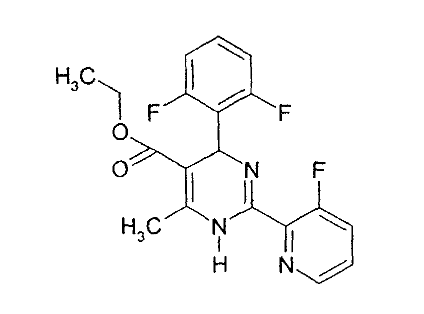

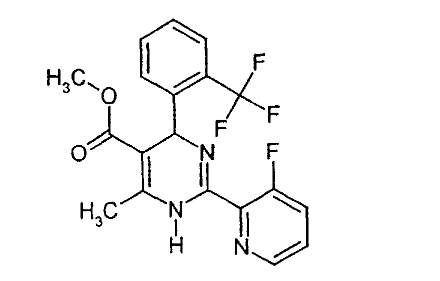

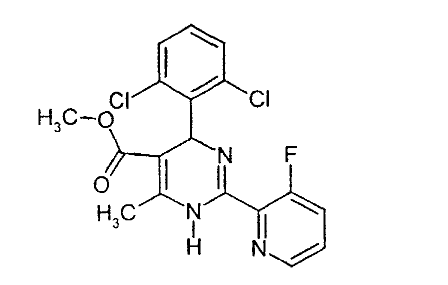

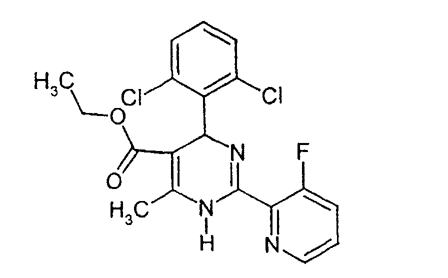

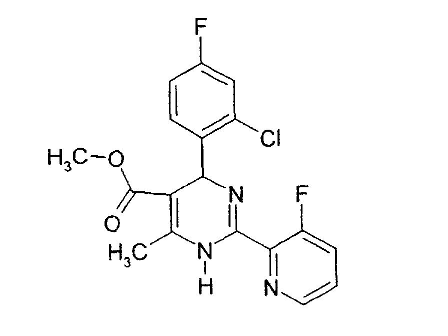

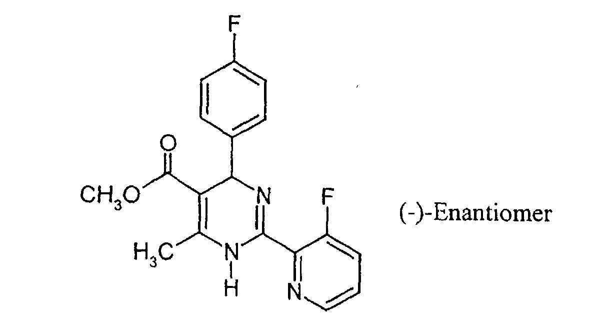

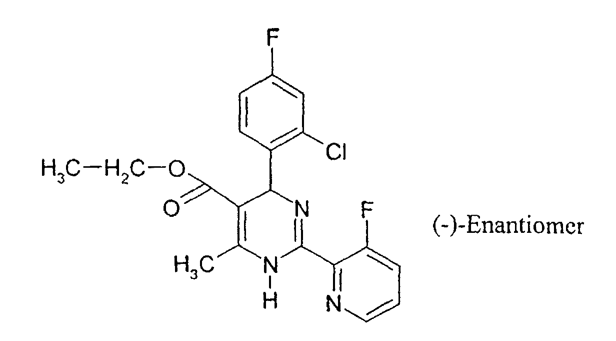

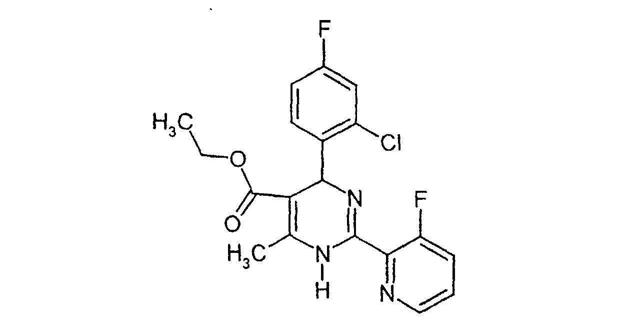

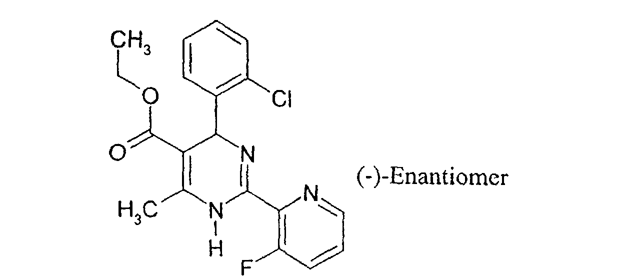

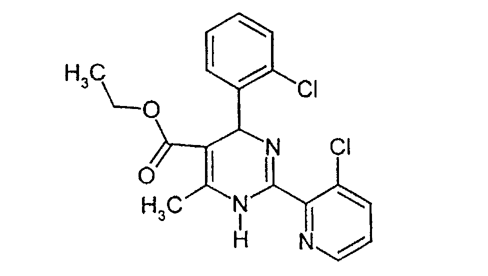

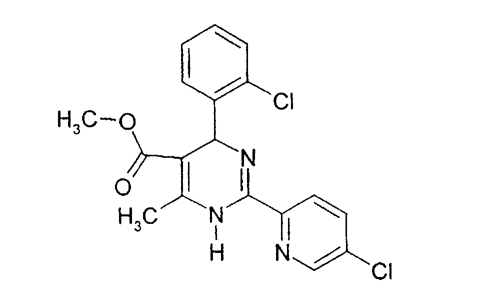

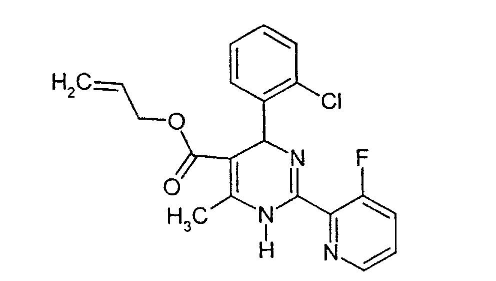

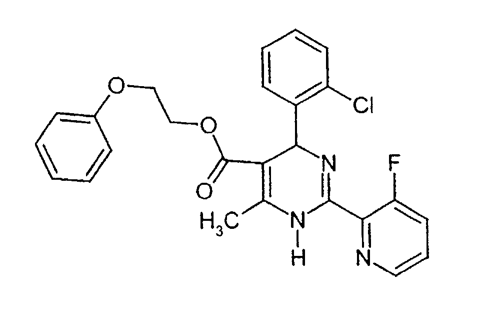

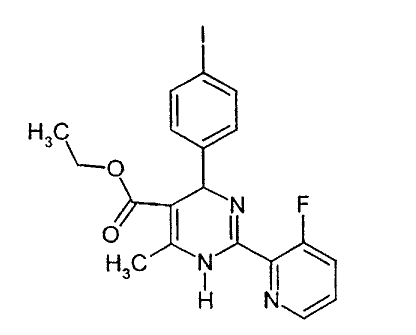

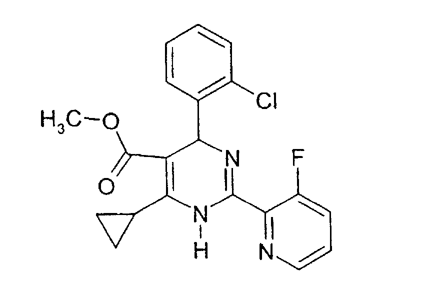

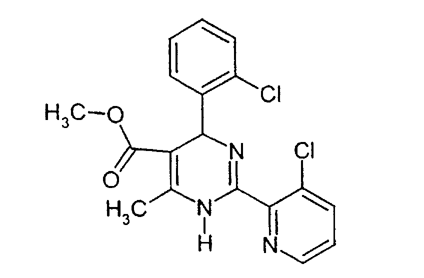

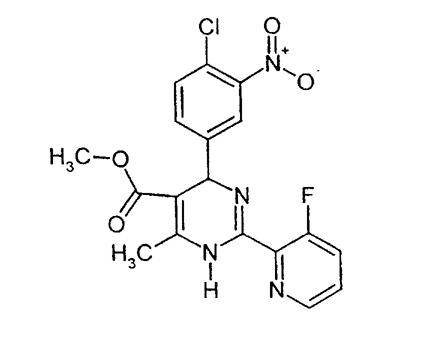

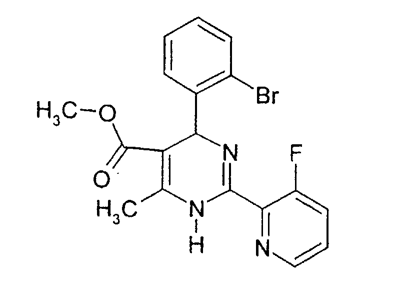

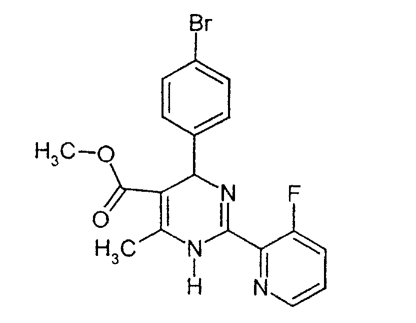

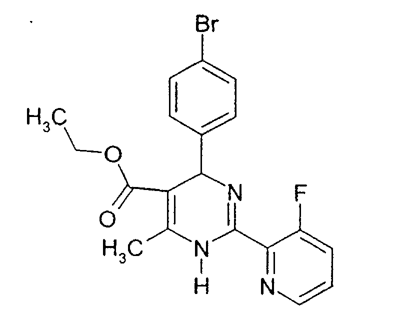

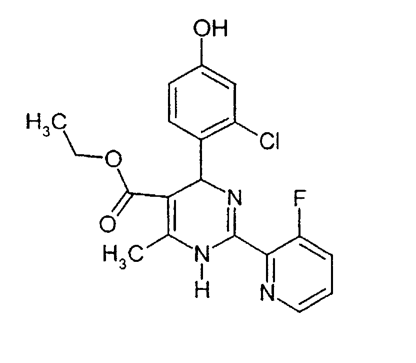

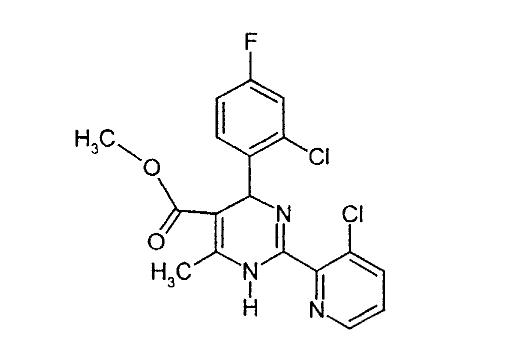

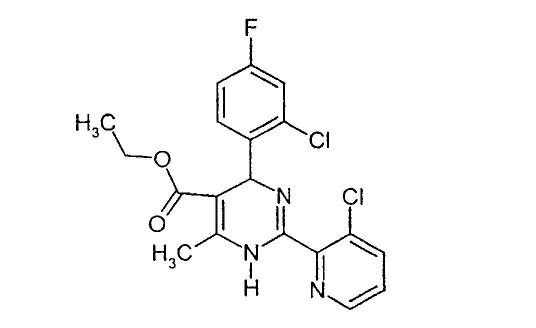

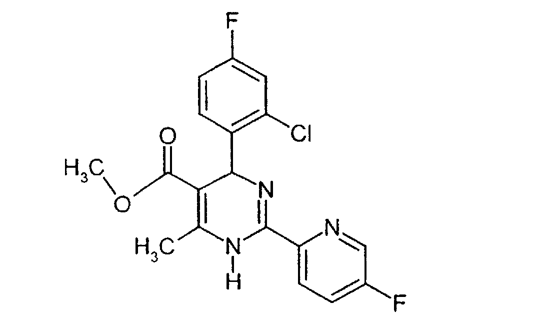

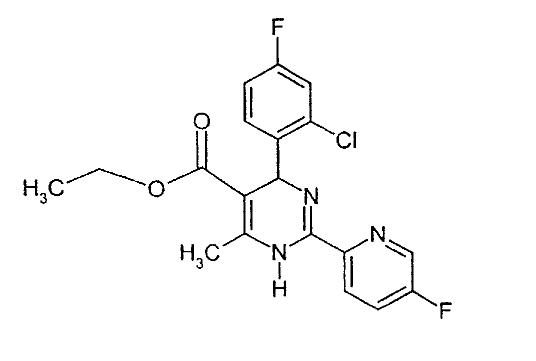

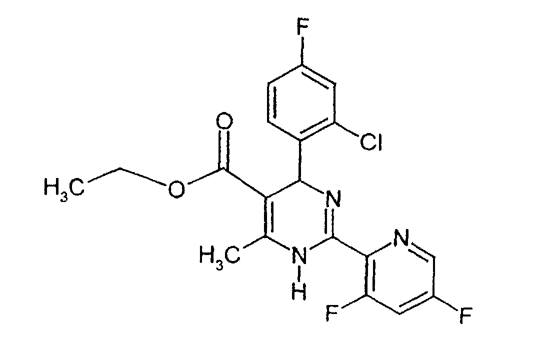

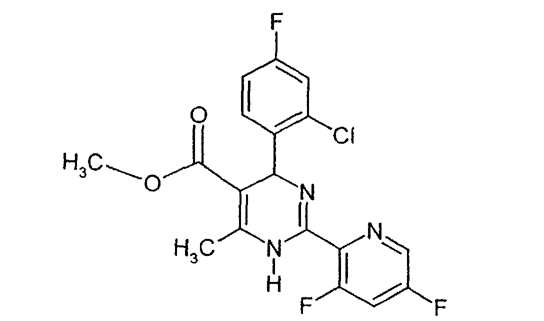

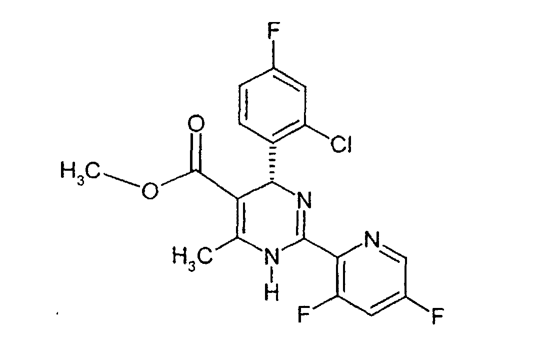

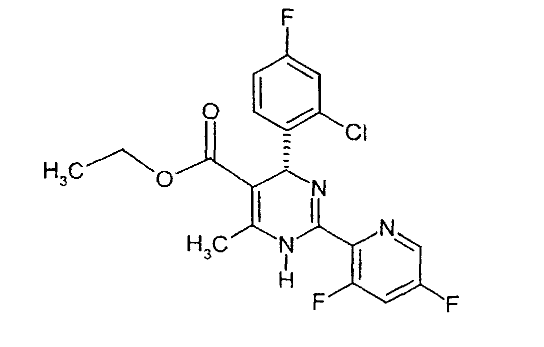

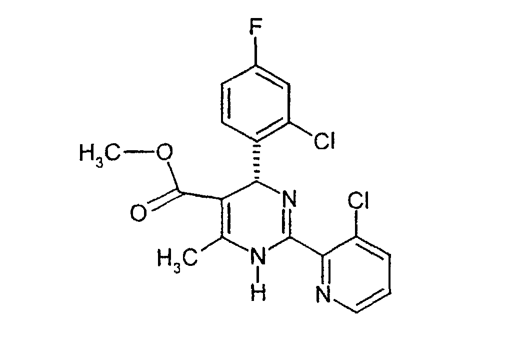

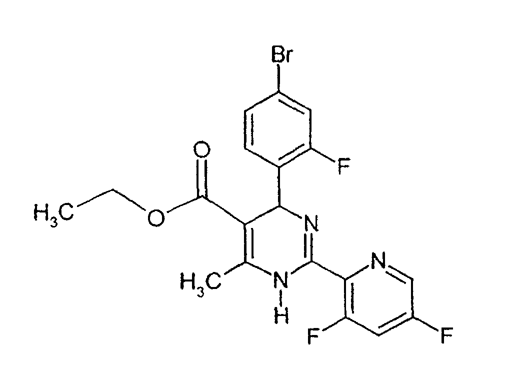

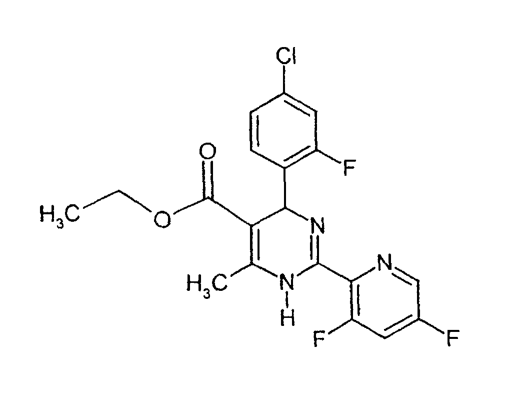

7. Verbindungen nach Anspruch 1 der folgenden Strukturen: 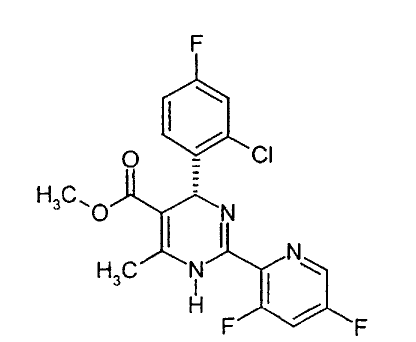 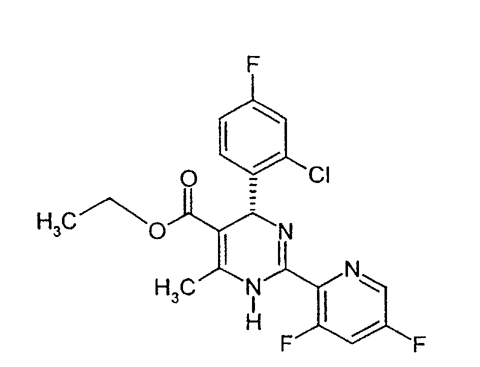 oder Salze davon.

8. Verfahren zur Herstellung der Verbindungen gemäß den Ansprüchen 1 bis 7, **dadurch gekennzeichnet, daß**
[A] Aldehyde der allgemeinen Formel (II)
R¹-CHO (II)
in welcher
R¹ die oben angegebene Bedeutung hat,
mit Amidinen oder deren Hydrochloriden der Formel (III) 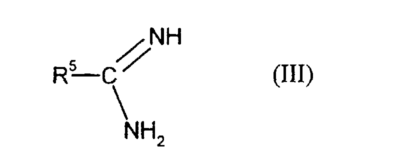 in welcher
R⁵ die oben angegebene Bedeutung hat,
und Verbindungen der allgemeinen Formel (IV)
R³-CO-CH₂-CO-R² (IV)
in welcher
R² und R³ die oben angegebene Bedeutung haben,
gegebenenfalls in Gegenwart inerter organischer Lösemittel mit oder ohne Basen- bzw. Säurezusatz umsetzt,
oder
[B] Verbindungen der allgemeinen Formel (V) 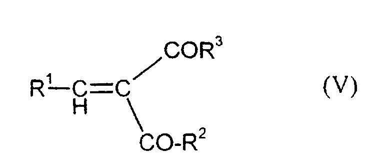 in welcher
R¹, R² und R³ die oben angegebene Bedeutung haben,
mit Amidinen der allgemeinen Formel (III) 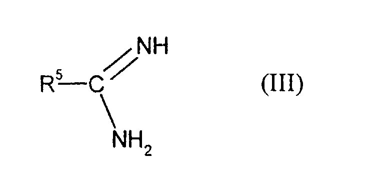 in welcher
R⁵ die oben angegebene Bedeutung hat,
gegebenenfalls in Gegenwart inerter organischer Lösemittel bei Temperaturen zwischen 20°C und 150°C mit oder ohne Basen- oder Säurezusatz umsetzt,
oder
[C] Aldehyde der allgemeinen Formel (II)
R¹-CHO (II)
in welcher
R¹ die oben angegebene Bedeutung hat,
mit Verbindungen der allgemeinen Formel (VI) 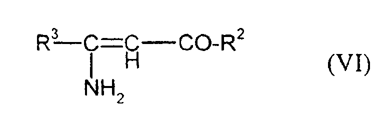 in welcher
R² und R³ die oben angegebene Bedeutung haben,
und Amidinen der allgemeinen Formel (III) wie oben beschrieben umsetzt,
oder
[D] Aldehyde der allgemeinen Formel (II) mit Verbindungen der allgemeinen Formel (IV) und Iminoether der allgemeinen Formel (VII) 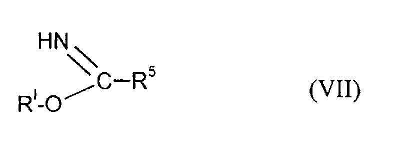 in welcher
R⁵ die oben angegebene Bedeutung hat,
und
R¹ für (C₁-C₄)-Alkyl steht,
in Gegenwart von Ammoniumsalzen umsetzt werden.

9. Verbindung der Formel: 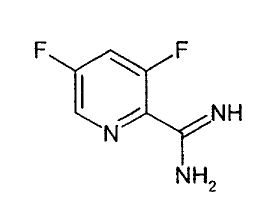 sowie Salze davon.

10. Verbindung der Formel:

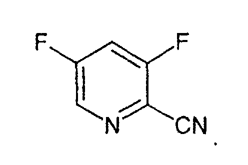

11. Arzneimittel, enthaltend mindestens eine Verbindung der allgemeinen Formel (I) oder (Ia) gemäß einem der Ansprüche 1 bis 7 und gegebenenfalls enthaltend weitere pharmazeutische Wirkstoffe.

12. Verfahren zur Herstellung von Arzneimitteln, **dadurch gekennzeichnet, daß** mindestens eine Verbindung der allgemeinen Formel (I) oder (Ia) gemäß einem der Ansprüche 1 bis 7, gegebenenfalls unter Verwendung von üblichen Hilfs- und Trägerstoffen in eine geeignete Applikationsform überführt wird.

13. Verbindungen der allgemeinen Formel (I) oder (Ia) gemäß einem der Ansprüche 1 bis 7 zur Verwendung als Arzneimittel.

14. Verwendung von Verbindungen der allgemeinen Formel (I) oder (Ia) gemäß einem der Ansprüche 1 bis 7 zur Herstellung eines Arzneimittels.

15. Verwendung von Verbindungen der allgemeinen Formel (I) oder (Ia) gemäß einem der Ansprüche 1 bis 7 zur Herstellung eines Arzneimittels zur Behandlung von akuten oder chronischen Viruserkrankungen.

16. Verwendung von Verbindungen der allgemeinen Formel (I) oder (Ia) gemäß einem der Ansprüche 1 bis 7 zur Herstellung eines Arzneimittels zur Behandlung von akuten oder chronischen Hepatitis-B-Infektionen.

## Claims

1. Compounds of the general formula (I) 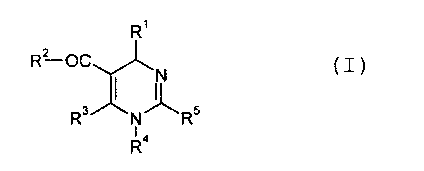 or their isomeric form (Ia) 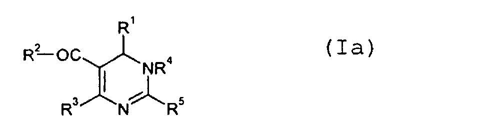 in which
R¹ represents phenyl, furyl, thienyl, triazolyl, pyridyl, cycloalkyl having 3 to 6 carbon atoms or represents radicals of the formulae 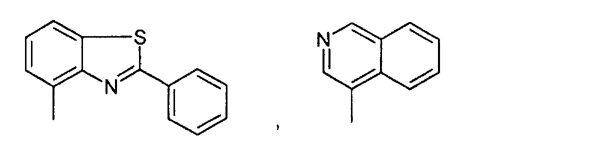 or 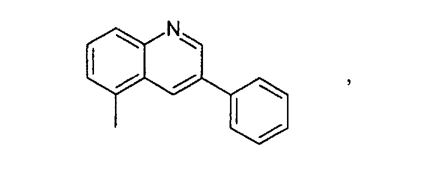 where the abovementioned ring systems are optionally mono- or polysubstituted by identical or different substituents selected from the group consisting of halogen, trifluoromethyl, nitro, cyano, trifluoromethoxy, carboxyl, hydroxyl, (C₁-C₆)-alkoxy, (C₁-C₆)-alkoxycarbonyl and (C₁-C₆)-alkyl, which for its part may be substituted by aryl having 6 to 10 carbon atoms or halogen,
and/or the ring systems mentioned are optionally substituted by groups of the formulae -S-R⁶, NR⁷R⁸, CO-NR⁹R¹⁰, SO₂-CF₃ and -A-CH₂-R¹¹,
in which
R⁶ represents phenyl which is optionally substituted by halogen,
R⁷, R⁸, R⁹ and R¹⁰ are identical or different, and each represents hydrogen, phenyl, hydroxyl-substituted phenyl, hydroxyl, (C₁-C₆)-acyl or (C₁-C₆)-alkyl, which for its part may be substituted by hydroxyl, (C₁-C₆)-alkoxycarbonyl, phenyl or hydroxyl-substituted phenyl,
A represents a radical O, S, SO or SO₂,
R¹¹ represents phenyl which is optionally mono- to polysubstituted by identical or different substituents selected from the group consisting of halogen, nitro, trifluoromethyl, (C₁-C₆)-alkyl and (C₁-C₆)-alkoxy,
R² represents a radical of the formula -XR¹² or -NR¹³R¹⁴
in which
X represents a bond or oxygen,
R¹² represents hydrogen, straight-chain or branched (C₁-C₆)-alkoxycarbonyl or a straight-chain, branched or cyclic saturated or unsaturated (C₁-C₈)-hydrocarbon radical which optionally contains one or two identical or different hetero chain members from the group consisting of O, CO, NH, -NH-(C₁-C₄)-alkyl, -N-((C₁-C₄)-alkyl)₂, S and SO₂ and which is optionally substituted by halogen, nitro, cyano, hydroxyl, aryl having 6 to 10 carbon atoms or aralkyl having 6 to 10 carbon atoms, heteroaryl or a group of the formula -NR¹⁵R¹⁶,
in which
R¹⁵ and R¹⁶ are identical or different, and each represents hydrogen, benzyl or (C₁-C₆)-alkyl,
R¹³ and R¹⁴ are identical or different, and each represents hydrogen, (C₁-C₆)-alkyl or cycloalkyl having 3 to 6 carbon atoms,
R³ represents hydrogen, amino or represents a radical of the formula 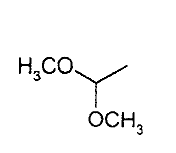 or represents formyl, cyano, trifluoromethyl or pyridyl, or
represents a straight-chain, branched or cyclic saturated or unsaturated hydrocarbon radical having up to 8 carbon atoms which is optionally mono- or polysubstituted by identical or different substituents from the group consisting of aryloxy having 6 to 10 carbon atoms, azido, cyano, hydroxyl, carboxyl, (C₁-C₆)-alkoxycarbonyl, a 5- to 7-membered heterocyclic ring, (C₁-C₆)-alkylthio and (C₁-C₆)-alkoxy, which for its part may be substituted by azido or amino, and/or is substituted by triazolyl, which for its part may be substituted up to 3 times by (C₁-C₆)-alkoxycarbonyl,
and/or may be substituted by groups of the formulae -OSO₂-CH₃ or (CO)ₐ-NR¹⁷R¹⁸,
in which
a represents a number 0 or 1,
R¹⁷ and R¹⁸ are identical or different, and each represents hydrogen or aryl, aralkyl having 6 to 10 carbon atoms,
or represents (C₁-C₆)-alkyl which is optionally substituted by (C₁-C₆)-alkoxycarbonyl, hydroxyl, phenyl or benzyl, where phenyl or benzyl are optionally mono- or polysubstituted by identical or different substituents from the group consisting of hydroxyl, carboxyl, (C₁-C₆)-alkyl and (C₁-C₆)-alkoxy,
or (C₁-C₆)-alkyl is optionally substituted by groups of the formulae NH-CO-CH₃ or NH-CO-CF₃,
or
R¹⁷ and R¹⁸ together with the nitrogen atom form a morpholine, piperidinyl or pyrrolidinyl ring,
or
R³ represents phenyl which is optionally substituted by methoxy,
or
R² and R³ together form a radical of the formula 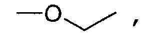
R⁴ represents hydrogen, (C₁-C₄)-alkyl, (C₂-C₄)-alkenyl, benzoyl or represents acyl having 2 to 6 carbon atoms,
R⁵ represents pyridyl which is substituted up to 3 times by identical or different substituents from the group consisting of halogen, hydroxyl, cyano, trifluoromethyl, (C₁-C₆)-alkoxy, (C₁-C₆)-alkyl, (C₁-C₆)-alkylthio, carbalkoxy, (C₁-C₆)-acyloxy, amino, nitro, mone- and (C₁-C₆)-dialkylamino,
and salts thereof.

2. Compounds of the general formulae (I) and (Ia) according to Claim 1, in which
R¹ represents phenyl, furyl, thienyl, pyridyl, cyclopentyl or cyclohexyl or represents radicals of the formulae 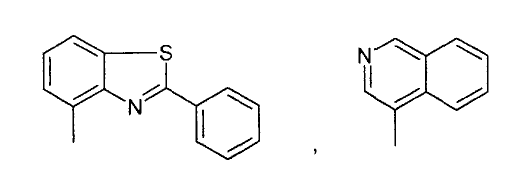 or 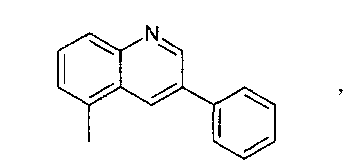 where the abovementioned ring systems are optionally mono- or disubstituted by identical or different substituents selected from the group consisting of halogen, trifluoromethyl, nitro, SO₂-CF₃, methyl, cyano, amino, trifluoromethoxy, hydroxyl, carboxyl, methoxycarbonyl and radicals of the formulae -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ or -S-pCl-C₆H₄,
R² represents a radical of the formula -XR¹² or -NR¹³R¹⁴,
in which
X represents a bond or an oxygen atom,
R¹² represents hydrogen, (C₁-C₄)-alkenyl, (C₁-C₄)-alkoxycarbonyl or (C₁-C₄)-alkyl which are optionally substituted by pyridyl, cyano, phenoxy, benzyl or by a radical of the formula -NR¹⁵R¹⁶,
in which
R¹⁵ and R¹⁶ are identical or different, and each represents hydrogen, benzyl or (C₁-C₄)-alkyl,
R¹³ and R¹⁴ are identical or different, and each represents hydrogen, (C₁-C₄)-alkyl or cyclopropyl,
R³ represesents hydrogen, amino or a radical of the formula 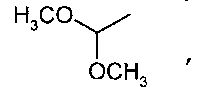 or
represents formyl, cyano, trifluoromethyl, cyclopropyl or pyridyl, or
represents (C₁-C₄)-alkyl which is optionally substituted by halogen, (C₁-C₄)-alkoxycarbonyl, hydroxyl or by triazolyl, which for its part may be substituted up to 3 times by (C₁-C₄)-alkoxycarbonyl,
and/or alkyl is optionally substituted by groups of the formulae -OSO₂-CH₃ or (CO)ₐ-NR¹⁷R¹⁸,
in which
a represents a number 0 or 1,
R¹⁷ and R¹⁸ are identical or different, and each represents hydrogen, phenyl or benzyl, or
represents C₁-C₄-alkyl which is optionally substituted by (C₁-C₄)-alkoxycarbonyl, hydroxyl, phenyl or benzyl, where phenyl or benzyl are optionally mono- or polysubstituted by identical or different substitutents from the group consisting of hydroxyl, carboxyl, (C₁-C₄)-alkyl and (C₁-C₄)-alkoxy,
and/or (C₁-C₄)-alkyl is optionally substituted by radicals of the formulae -NH-CO-CH₃ or -NH-CO-CF₃,
or
R¹⁷ and R¹⁸ together with the nitrogen atom form a morpholine, piperidinyl or pyrrolidinyl ring,
or
R³ represents phenyl which is optionally substituted by methoxy,
or
R² and R³ together form a radical of the formula 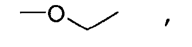
R⁴ represents hydrogen, methyl, benzoyl or acetyl,
R⁵ represents pyridyl which is substituted up to 2 times by identical or different substituents from the group consisting of fluorine, chlorine, bromine, (C₁-C₄)-alkoxy and (C₁-C₄)-alkyl,
and salts thereof.

3. Compounds of the general formulae (I) and (Ia) according to Claim 1
in which
R¹ represents phenyl, furyl, thienyl, pyridyl, cyclopentyl, cyclohexyl or represents radicals of the formulae 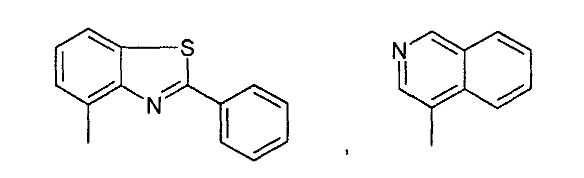 or 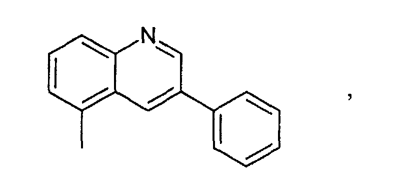 where the abovementioned ring systems are optionally substituted up to 2 times by identical or different substituents selected from the group consisting of fluorine, chlorine, bromine, iodine, hydroxyl, trifluoromethyl, amino, nitro, SO₂-CF₃, methyl, cyano, trifluoromethoxy, carboxyl, methoxycarbonyl and radicals of the formulae -CO-NH-CH₂-C(CH₃)₃, -CO-NH(CH₂)₂OH, -CO-NH-CH₂-C₆H₅, -CO-NH-C₆H₅, -CO-NH- (pOH) -C₆H₄, -O-CH₂-C₆H₅ or -S-pCl-C₆H₄,
R² represents a radical of the formula -XR¹² or -NR¹³R¹⁴,
in which
X represents a bond or an oxygen atom,
R¹² represents hydrogen, (C₁-C₃)-alkenyl, (C₁-C₄)-alkoxycarbonyl or (C₁-C₄)-alkyl which are optionally substituted by pyridyl, cyano, phenoxy, benzyl or by a radical of the formula -NR¹⁵R¹⁶,
in which
R¹⁵ and R¹⁶ are identical or different, and each represents hydrogen or methyl,
R¹³ and R¹⁴ are identical or different, and each represents hydrogen, (C₁-C₃)-alkyl or cyclopropyl,
R³ represents hydrogen, amino or represents a radical of the formula 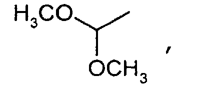 or
represents formyl, cyano, trifluoromethyl, cyclopropyl or pyridyl, or
represents (C₁-C₄)-alkyl which is optionally substituted by fluorine, chlorine, (C₁-C₃)-alkoxycarbonyl, hydroxyl or by triazolyl, which for its part may be substituted up to 3 times by (C₁-C₃)-alkoxycarbonyl,
and/or alkyl is optionally substituted by groups of the formulae -OSO₂-CH₃ or (CO)ₐ-NR¹⁷R¹⁸,
in which
a represents a number 0 or 1,
R¹⁷ and R¹⁸ are identical or different, and each represents hydrogen, phenyl or benzyl, or
represents (C₁-C₃)-alkyl which is optionally substituted by (C₁-C₃)-alkoxycarbonyl, hydroxyl, phenyl or benzyl, where phenyl or benzyl are optionally mono- or disubstituted by identical or different substituents from the group consisting of hydroxyl, carboxyl, (C₁-C₃)-alkyl and (C₁-C₃)-alkoxy,
and/or (C₁-C₄)-alkyl is optionally substituted by radicals of the formulae -NH-CO-CH₃ or -NH-CO-CF₃,
or
R¹⁷ and R¹⁸ together with the nitrogen atom form a morpholine, piperidinyl or pyrrolidinyl ring,
or
R³ represents phenyl which is optionally substituted by methoxy,
or
R² and R³ together form a radical of the formula 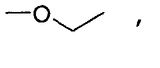
R⁴ represents hydrogen, methyl, benzoyl or acetyl,
R⁵ represents pyridyl which is substituted up to 2 times by identical or different substituents from the group consisting of fluorine, chlorine, (C₁-C₃)-alkoxy and (C₁-C₃)-alkyl,
and salts thereof.

4. Compounds of the general formulae (I) and (Ia) according to Claim 1, in which
R¹ represents phenyl which is optionally substituted up to 2 times by identical or different substituents from the group consisting of fluorine, chlorine, bromine, iodine, methyl and nitro,
R² represents -XR¹² in which X represents oxygen and R¹² represents straight-chain or branched alkyl having up to 4 carbon atoms,
R³ represents methyl, ethyl or cyclopropyl,
or
R² and R³ together form a radical of the formula 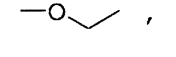
R⁴ represents hydrogen, or acetyl,
and
R⁵ represents pyridyl which is substituted up to two times by identical or different substituents from the group consisting of fluorine and chlorine,
and salts thereof.

5. Compounds of the general formulae (I) and (Ia) according to any of Claims 1 to 4 in which R⁵ represents 2-pyridyl which is substituted by 1 or 2 fluorine atoms.

6. Compounds according to Claim 1 of the structures below

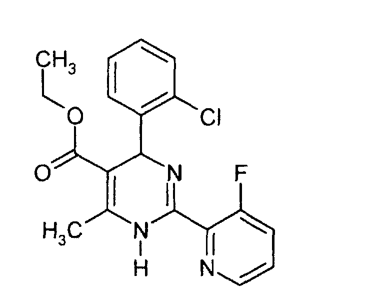

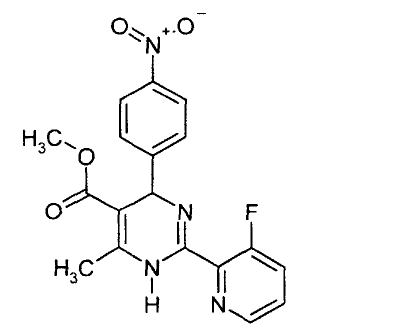

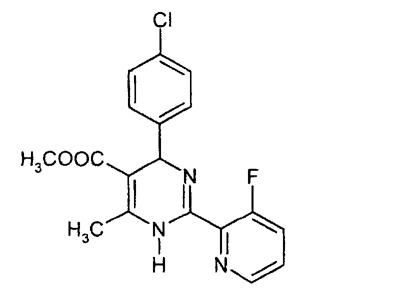

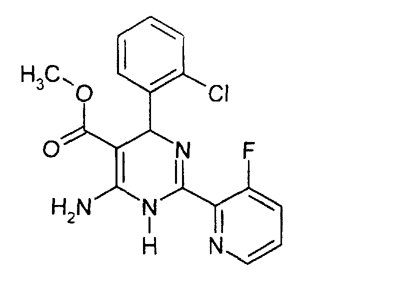

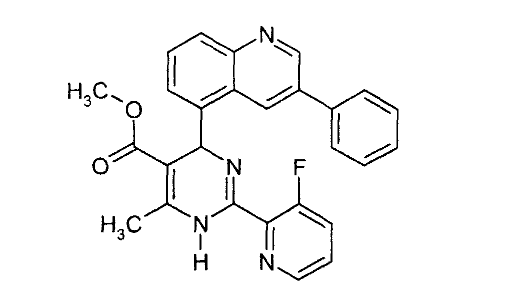

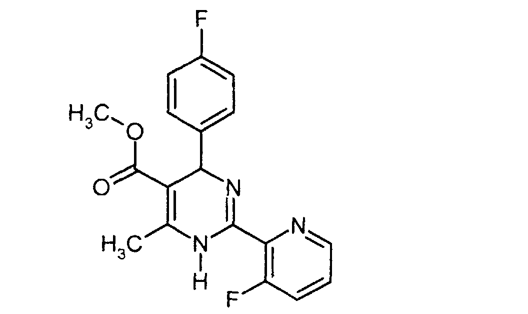

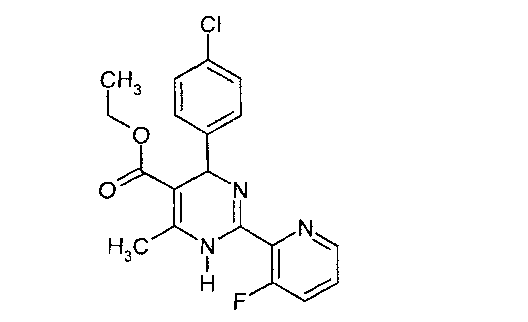

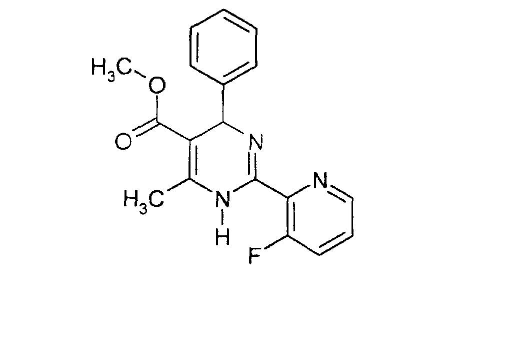

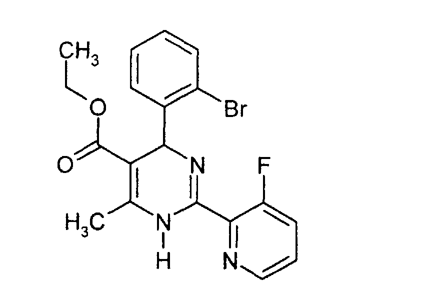

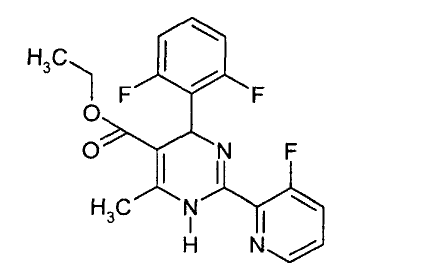

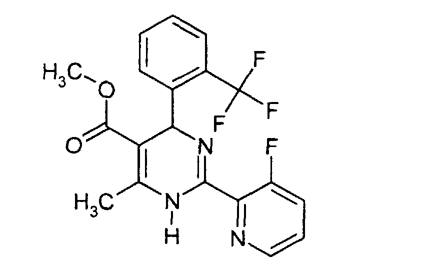

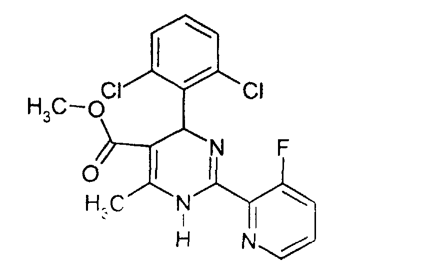

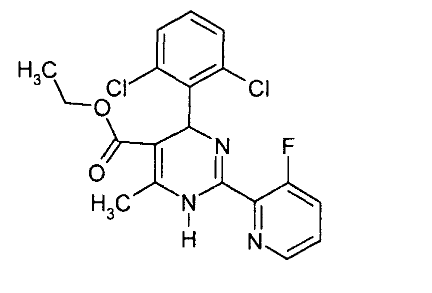

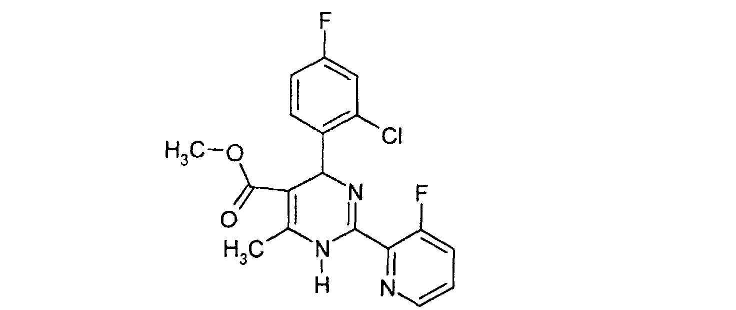

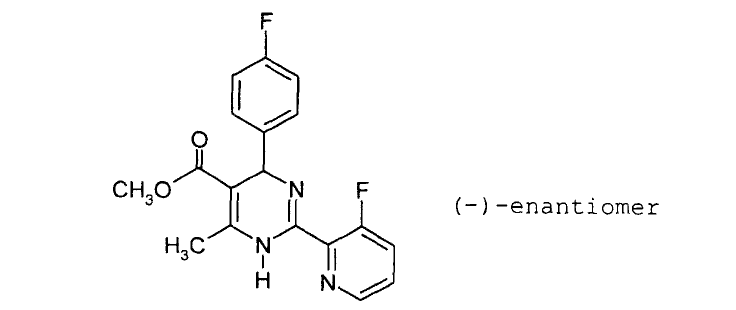

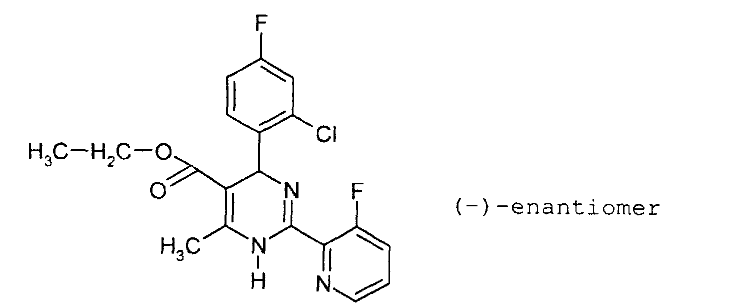

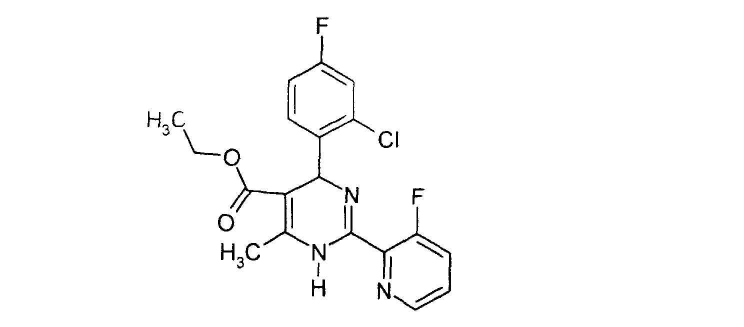

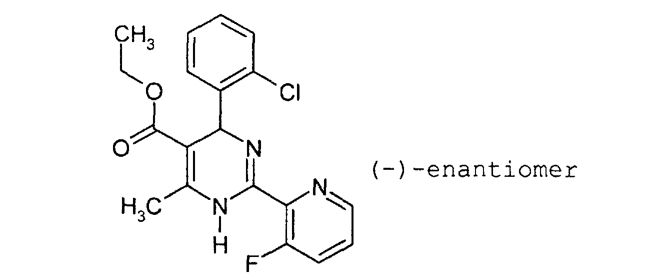

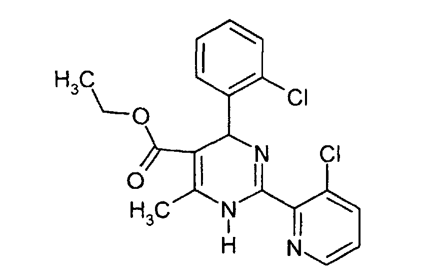

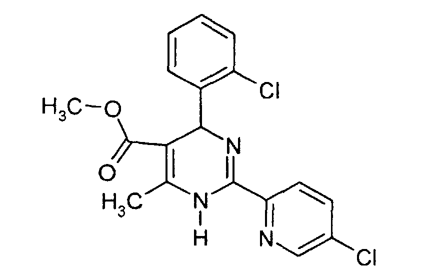

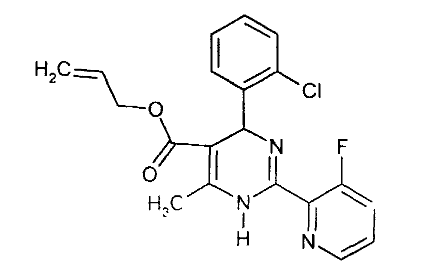

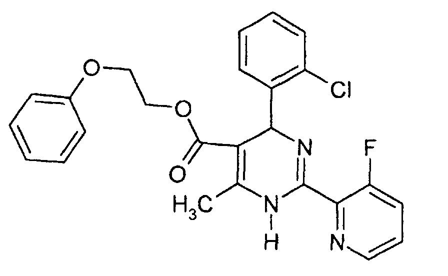

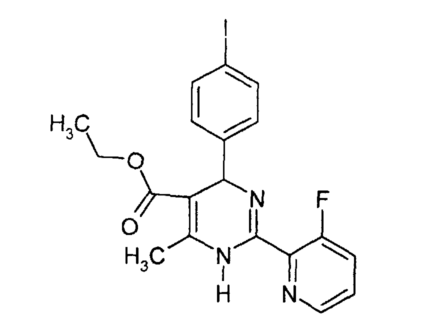

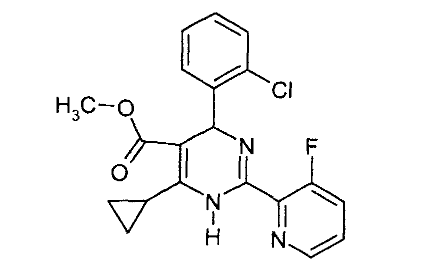

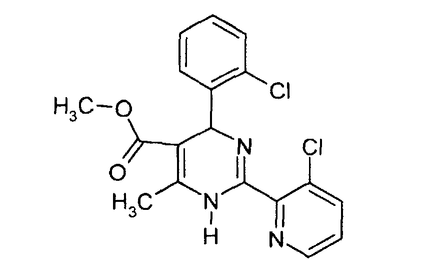

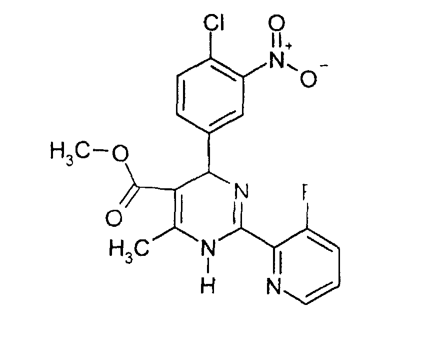

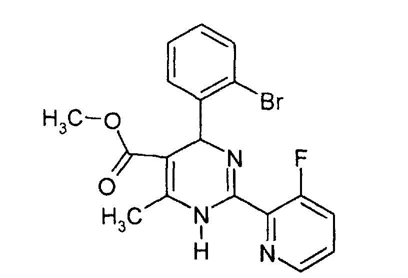

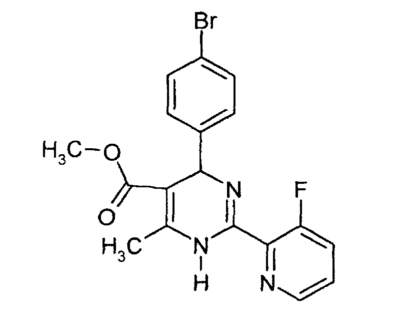

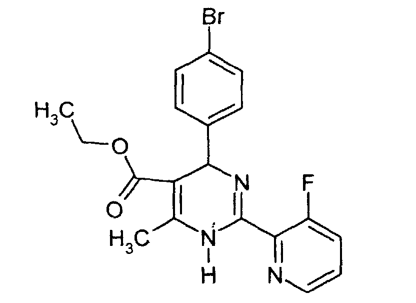

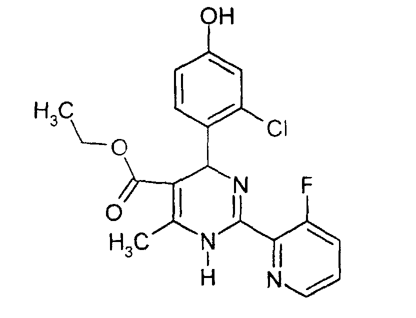

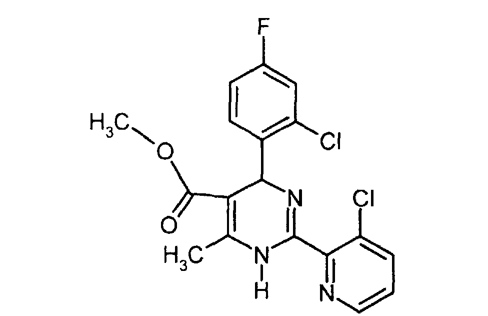

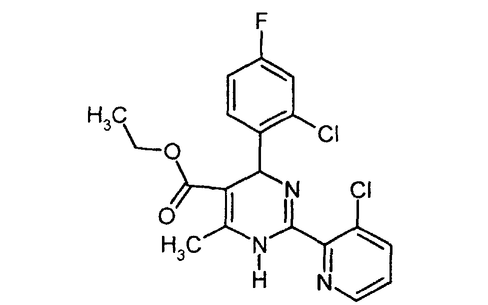

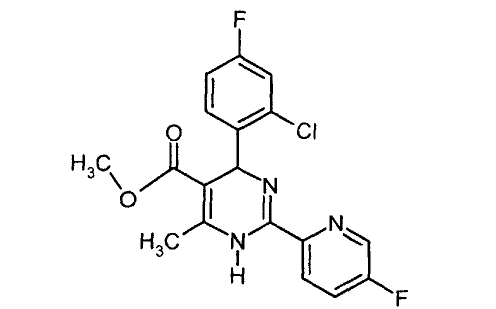

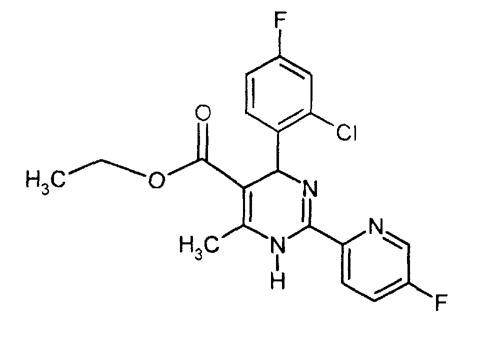

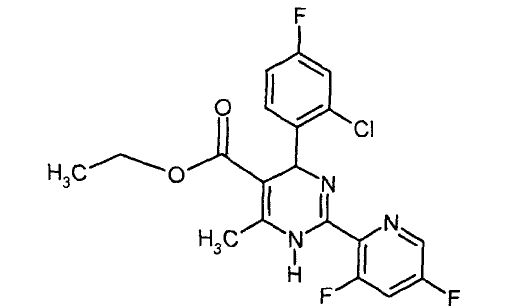

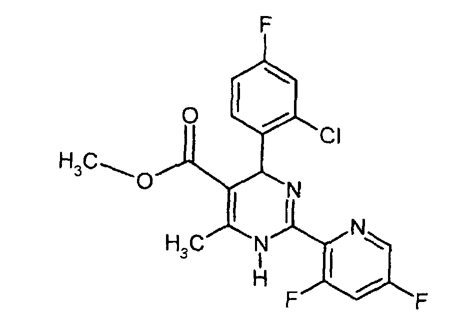

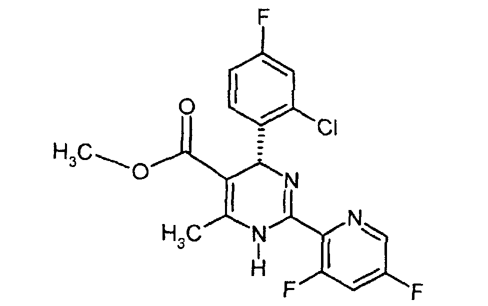

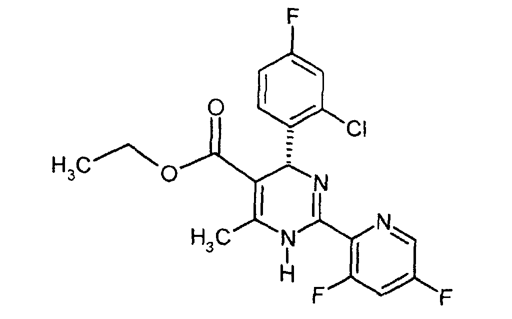

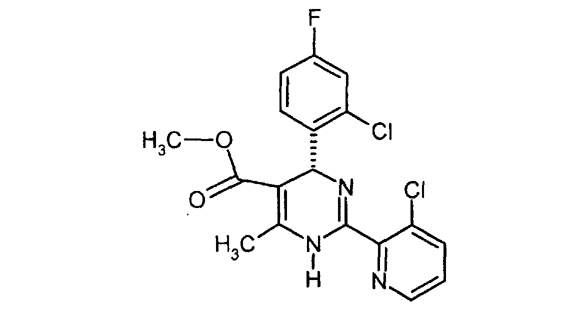

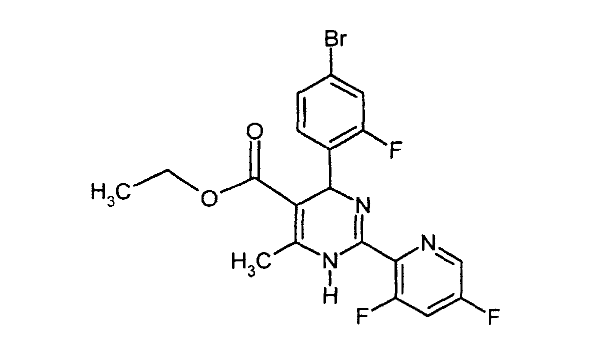

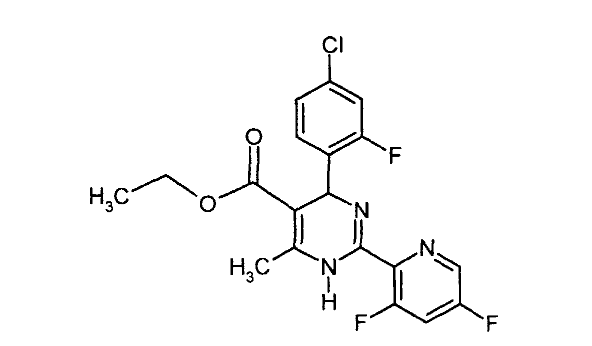

7. Compounds according to Claim 1 of the structures below: 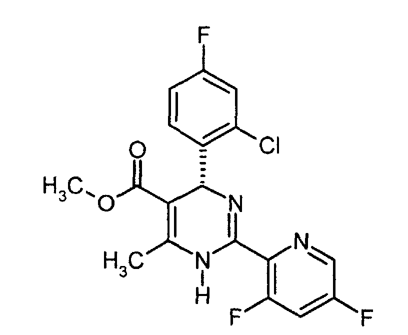 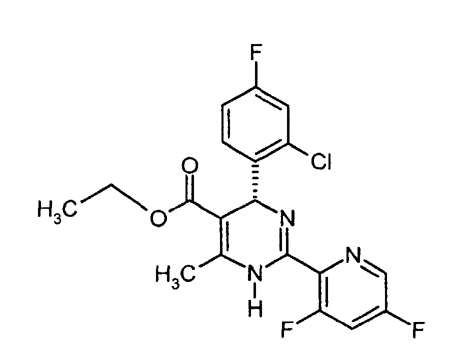 or salts thereof.

8. Process for preparing the compounds according to Claims 1 to 7, **characterized in that**,
[A] aldehydes of the general formula (II)
R¹-CHO (II)
in which
R¹ is as defined above,
are reacted with amidines or their hydrochlorides of the formula (III) 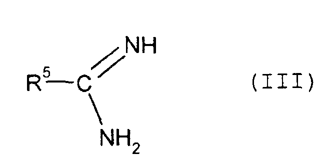 in which
R⁵ is as defined above,
and compounds of the general formula (IV)
R³-CO-CH₂-CO-R² (IV)
in which
R² and R³ are each as defined above,
if appropriate in the presence of inert organic solvents, with or without addition of base or acid,
or
[B] compounds of the general formula (V) 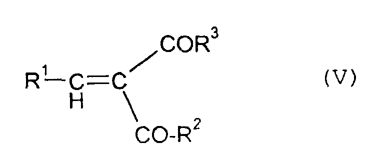 in which
R¹, R² and R³ are each as defined above,
are reacted with amidines of the general formula (III) 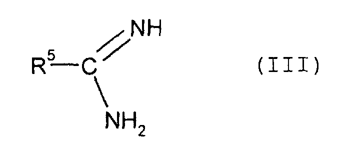 in which
R⁵ is as defined above,
if appropriate in the presence of inert organic solvents at temperatures between 20°C and 150°C, with or without addition of base or acid,
or
[C] aldehydes of the general formula (II)
R¹-CHO (II)
in which
R¹ is as defined above,
are reacted with compounds of the general formula (VI) 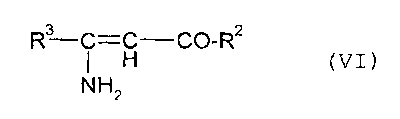 in which
R² and R³ are each as defined above,
and amidines of the general formula (III) as described above,
or
[D] aldehydes of the general formula (II) are reacted with compounds of the general formula (IV) and imino ethers of the general formula (VII) 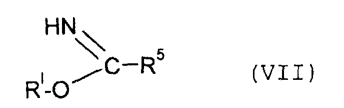 in which
R⁵ is as defined above,
and
R^{I} represents (C₁-C₄)-alkyl,
in the presence of ammonium salts.

9. Compound of the formula: 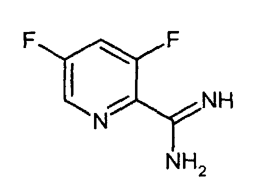 and salts thereof.

10. Compound of the formula:

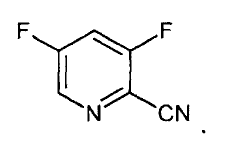

11. Medicaments, containing at least one compound of the general formula (I) or (Ia) according to one of Claims 1 to 7 and, if appropriate, containing further pharmaceutically active compounds.

12. Process for producing medicaments, **characterized in that** at least one compound of the general formula (I) or (Ia) according to one of Claims 1 to 7 is converted into a suitable administration form, if appropriate using customary auxiliaries and excipients.

13. Compounds of the general formula (I) or (Ia) according to one of Claims 1 to 7 for use as medicaments.

14. Use of compounds of the general formula (I) or (Ia) according to one of Claims 1 to 7 for producing a medicament.

15. Use of compounds of the general formula (I) or (Ia) according to one of Claims 1 to 7 for producing a medicament for treating acute or chronic viral diseases.

16. Use of compounds of the general formula (I) or (Ia) according to one of Claims 1 to 7 for producing a medicament for treating acute or chronic hepatitis B infections.

## Revendications

1. Composés de formule générale (I) 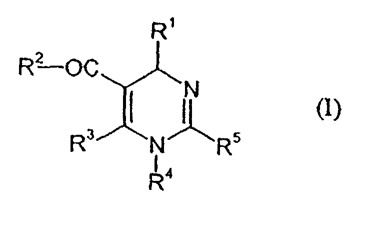 ou de sa forme isomère (Ia) 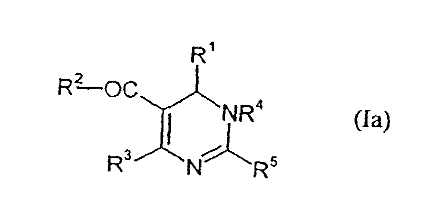 formules dans lesquelles
R¹ est un reste phényle, furyle, thiényle, triazolyle, pyridyle, cycloalkyle ayant 3 à 6 atomes de carbone ou des restes de formules 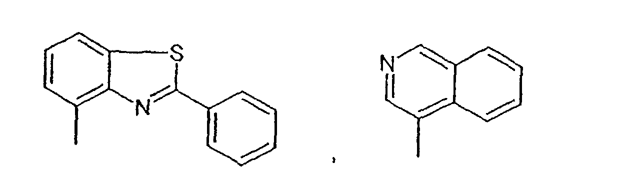 ou 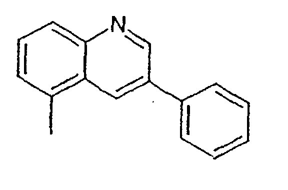 les systèmes cycliques énumérés ci-dessus portant éventuellement un ou plusieurs substituants, identiques ou différents, choisis dans le groupe halogène, trifluorométhyle, nitro, cyano, trifluorométhoxy, carboxyle, hydroxyle, alkoxy en C₁ à C₆, (alkoxy en C₁ à C₆)-carbonyle et alkyle en C₁ à C₆ qui peut lui-même être substitué par un radical aryle ayant 6 à 10 atomes de carbone ou un halogène,
et/ou les systèmes cycliques énumérés étant éventuellement substitués par des groupes de formules -S-R⁶, NR⁷R⁸, CO-NR⁹R¹⁰, SO₂-CF₃ et -A-CH₂-R¹¹,
où
R⁶ représente un groupe phényle qui est éventuellement substitué par un halogène,
R⁷, R⁸, R⁹ et R¹⁰ sont identiques ou différents et représentent l'hydrogène, un groupe phényle, phényle à substituant hydroxy, hydroxy, acyle en C₁ à C₆ ou alkyle en C₁ à C₆ qui peut lui-même être substitué par un radical hydroxy, (alkoxy en C₁ à C₆)-carbonyle, phényle ou phényle à substituant hydroxy,
A est un reste O, S, SO ou SO₂,
R¹¹ est un groupe phényle qui est éventuellement substitué une ou plusieurs fois identiques ou différentes par des substituants choisis dans le groupe halogène, nitro, trifluorométhyle, alkyle en C₁ à C₆ et alkoxy en C₁ à C₆,
R² est un reste de formule -XR¹² ou -NR¹³R¹⁴,
dans laquelle
X représente une liaison ou l'oxygène,
R¹² représente l'hydrogène, un groupe (alkoxy en C₁ à C₆)-carbonyle linéaire ou ramifié ou un reste d'hydrocarbure en C₁ à C₈ saturé ou non saturé linéaire, ramifié ou cyclique, qui contient un ou deux chaînons hétéroatomiques identiques ou différents du groupe O, CO, NH, -NH-(alkyle en C₁ à C₄), -N-(alkyle en C₁ à C₄)₂, S et SO₂ et qui est éventuellement substitué par un radical halogéno, nitro, cyano, hydroxy, aryle ayant 6 à 10 atomes de carbone ou aralkyle ayant 6 à 10 atomes de carbone, hétéroaryle ou un groupe de formule -NR¹⁵R¹⁶,
dans laquelle
R¹⁵ et R¹⁶ sont identiques ou différents et représentent l'hydrogène, un groupe benzyle ou un groupe alkyle en C₁ à C₆,
R¹³ et R¹⁴ sont identiques ou différents et représentent l'hydrogène, un groupe alkyle en C₁ à C₆ ou un groupe cycloalkyle ayant 3 à 6 atomes de carbone,
R³ représente l'hydrogène, un groupe amino ou un reste de formule 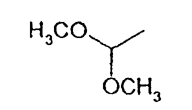 ou représente
un groupe formyle, cyano, trifluorométhyle ou pyridyle, ou représente
un reste d'hydrocarbure saturé ou non saturé linéaire, ramifié ou cyclique ayant jusqu'à 8 atomes de carbone, qui est éventuellement substitué une ou plusieurs fois identiques ou différentes par un radical aryloxy ayant 6 à 10 atomes de carbone, azido, cyano, hydroxy, carboxyle, (alkoxy en C₁ à C₆)-carbonyle, un noyau hétérocyclique pentagonal à heptagonal, un radical alkylthio en C₁ à C₆ ou alkoxy en C₁ à C₆ qui peut lui-même être substitué par un radical azido ou amino,
et/ou est substitué par un reste triazolyle qui peut lui-même être substitué jusqu'à 3 fois par un radical (alkoxy en C₁ à C₆)-carbonyle,
et/ou peut être substitué par des groupes de formules -OSO₂-CH₃ ou (CO)ₐ-NR¹⁷R¹⁸,
où
a représente le nombre 0 ou 1,
R¹⁷ et R¹⁸ sont identiques ou différents et représentent l'hydrogène ou un groupe aryle, aralkyle ayant 6 à 10 atomes de carbone ou alkyle ayant 1 à 6 atomes de carbone, qui est éventuellement substitué par un radical (alkoxy en C₁ à C₆)-carbonyle, hydroxyle, phényle ou benzyle, les radicaux phényle ou benzyle étant éventuellement substitués une ou plusieurs fois identiques ou différentes par un radical hydroxy, carboxyle, alkyle en C₁ à C₆ ou alkoxy en C₁ à C₆, ou bien alkyle en C₁ à C₆ éventuellement substitué par des groupes de formules NH-CO-CH₃ ou NH-CO-CF₃,
ou bien
R¹⁷ et R¹⁸ forment conjointement avec l'atome d'azote un noyau morpholine, pipéridinyle ou pyrrolidinyle,
ou bien
R³ est un groupe phényle qui est éventuellement substitué par un radical méthoxy,
ou bien
R² et R³ forment ensemble un reste de formule 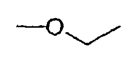
R⁴ représente l'hydrogène, un groupe alkyle en C₁ à C₄, alcényle en C₂ à C₄, benzoyle ou un groupe acyle ayant 2 à 6 atomes de carbone,
R⁵ est un groupe pyridyle qui est substitué jusqu'à 3 fois identiques ou différentes par un radical halogéno, hydroxy, cyano, trifluorométhyle, alkoxy en C₁ à C₆, alkyle en C₁ à C₆, alkylthio en C₁ à C₆, carbalkoxy, acyloxy en C₁ à C₆, amino, nitro, mono- ou di-(alkyle en C₁ à C₆)-amino,
et leurs sels.

2. Composés de formules générales (I) et (Ia) suivant la revendication 1, formules dans lesquelles
R¹ représente un reste phényle, furyle, thiényle, pyridyle, cyclopentyle ou cyclohexyle ou des restes de formules 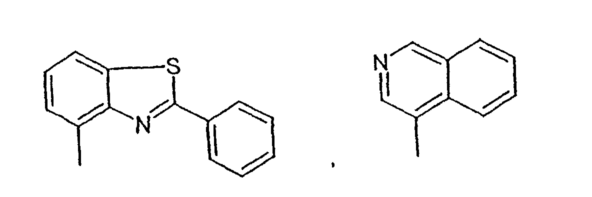 ou 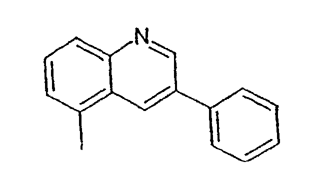 les systèmes cycliques énumérés ci-dessus étant substitués, le cas échéant, une ou deux fois identiques ou différentes par des substituants choisis dans le groupe halogène, trifluorométhyle, nitro, SO₂-CF₃, méthyle, cyano, amino, trifluorométhoxy, hydroxy, carboxyle, méthoxycarbonyle et des restes de formules -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ ou - S-pCl-C₆H₄,
R² représente un reste de formule -XR¹² ou -NR¹³R¹⁴,
dans laquelle
X est une liaison ou un atome d'oxygène,
R¹² représente l'hydrogène, un groupe alcényle en C₁ à C₄, (alkoxy en C₁ à C₄)-carbonyle ou alkyle en C₁ à C₄, substitués, le cas échéant, par un reste pyridyle, cyano, phénoxy, benzyle ou par un reste de formule -NR¹⁵R¹⁶,
où
R¹⁵ et R¹⁶ sont identiques ou différents et représentent l'hydrogène, un groupe benzyle ou alkyle en C₁ à C₄,
R¹³ et R¹⁴ sont identiques ou différents et représentent l'hydrogène, un groupe alkyle en C₁ à C₄ ou cyclopropyle,
R³ représente l'hydrogène, un groupe amino ou un reste de formule 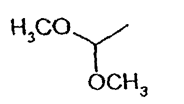 ou bien
un groupe formyle, cyano, trifluorométhyle, cyclopropyle ou pyridyle,
ou bien
un groupe alkyle en C₁ à C₄ qui est éventuellement substitué par un radical halogéno, (alkoxy en C₁ à C₄)-carbonyle, hydroxy ou par un radical triazolyle qui peut lui-même être substitué jusqu'à 3 fois par un radical (alkoxy en C₁ à C₄)-carbonyle,
et/ou un groupe alkyle éventuellement substitué par des groupes de formules -OSO₂-CH₃ ou (CO)ₐ-NR¹⁷R¹⁸,
où
a désigne le nombre 0 ou 1,
R¹⁷ et R¹⁸ sont identiques ou différents et représentent l'hydrogène, un groupe phényle ou benzyle ou représentent
un groupe alkyle en C₁ à C₄ qui est éventuellement substitué par un radical (alkoxy en C₁ à C₄)-carbonyle, hydroxyle, phényle ou benzyle, les radicaux phényle ou benzyle étant éventuellement substitués une ou plusieurs fois identiques ou différentes par un radical hydroxy, carboxy, alkyle en C₁ à C₄ ou alkoxy en C₁ à C₄, et/ou le groupe alkyle en C₁ à C₄ est éventuellement substitué par des restes de formules -NH-CO-CH₃ ou -NH-CO-CF₃,
ou bien
R¹⁷ et R¹⁸ forment conjointement avec l'atome d'azote un noyau morpholine, pipéridinyle ou pyrrolidinyle,
ou bien
R³ désigne un groupe phényle qui est éventuellement substitué par un radical méthoxy,
ou bien
R² et R³ forment ensemble un reste de formule 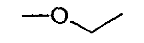
R⁴ représente l'hydrogène, un groupe méthyle, benzoyle ou acétyle,
R⁵ est un groupe pyridyle qui est substitué jusqu'à 2 fois identiques ou différentes par du fluor, du chlore, du brome, un radical alkoxy en C₁ à C₄ ou alkyle en C₁ à C₄,
et leurs sels.

3. Composés de formules générales (I) et (Ia) suivant la revendication 1, dans lesquelles
R¹ représente un reste phényle, furyle, thiényle, pyridyle, cyclopentyle, cyclohexyle ou des restes de formules 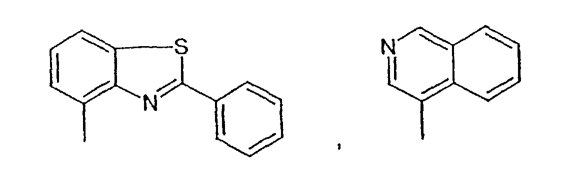 ou 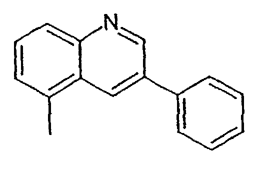 les systèmes cycliques énumérés ci-dessus étant substitués, le cas échéant, jusqu'à 2 fois identiques ou différentes par des substituants choisis dans le groupe fluor, chlore, brome, iode, hydroxy, trifluorométhyle, amino, nitro, SO₂-CF₃-, méthyle, cyano, trifluorométhoxy, carboxyle, méthoxycarbonyle et des restes de formules -CO-NH-CH₂-C(CH₃)₃, -CO-NH(CH₂)₂OH, -CO-NH-CH₂-C₆H₅, -CO-NH-C₆H₅, -CO-NH-(pOH)-C₆H₄, -O-CH₂-C₆H₅ ou -S-pCl-C₆H₄,
R² représente un reste de formule -XR¹² ou -NR¹³R¹⁴,
où
X représente une liaison ou un atome d'oxygène,
R¹² représente l'hydrogène, un groupe alcényle en C₁ à C₃, (alkoxy en C₁ à C₄)-carbonyle ou alkyle en C₁ à C₄, éventuellement substitués par un reste pyridyle, cyano, phénoxy, benzyle ou par un reste de formule -NR¹⁵R¹⁶,
où
R¹⁵ et R¹⁶ sont identiques ou différents et représentent l'hydrogène ou un groupe méthyle,
R¹³ et R¹⁴ sont identiques ou différents et représentent l'hydrogène, un groupe alkyle en C₁ à C₃ ou cyclopropyle,
R³ représente l'hydrogène, un groupe amino ou un reste de formule 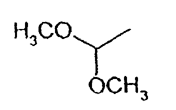 ou bien
un groupe formyle, cyano, trifluorométhyle, cyclopropyle ou pyridyle,
ou bien
un groupe alkyle en C₁ à C₄ qui est éventuellement substitué par du fluor, du chlore, un radical (alkoxy en C₁ à C₃)-carbonyle, hydroxy ou par un radical triazolyle qui peut lui-même être substitué jusqu'à 3 fois par un radical (alkoxy en C₁ à C₃)-carbonyle,
et/ou un groupe alkyle éventuellement substitué par des groupes de formules -OSO₂-CH₃ ou (CO)ₐ-NR¹⁷R¹⁸,
où
a représente le nombre 0 ou 1,
R¹⁷ et R¹⁸ sont identiques ou différents et représentent l'hydrogène, un groupe phényle ou benzyle, ou bien
un groupe alkyle en C₁ à C₃ qui est éventuellement substitué par un radical (alkoxy en C₁ à C₃)-carbonyle, hydroxyle, phényle ou benzyle,
les restes phényle ou benzyle étant éventuellement substitués une ou deux fois identiques ou différentes par un radical hydroxy, carboxy, alkyle en C₁ à C₃ ou alkoxy en C₁ à C₃,
et/ou le groupe alkyle en C₁ à C₄ est éventuellement substitué par des restes de formules -NH-CO-CH₃ ou -NH-CO-CF₃,
ou bien
R¹⁷ et R¹⁸ forment conjointement avec l'atome d'azote un noyau morpholine, pipéridinyle ou pyrrolidinyle,
ou bien
R³ est un groupe phényle qui est éventuellement substitué par un radical méthoxy,
ou bien
R² et R³ forment ensemble un reste de formule 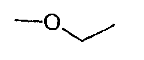
R⁴ représente l'hydrogène, un groupe méthyle, benzoyle ou acétyle,
R⁵ est un groupe pyridyle qui est substitué jusqu'à 2 fois identiques ou différentes par du fluor, du chlore, un radical alkoxy en C₁ à C₃ ou alkyle en C₁ à C₃,
et leurs sels.

4. Composés de formules générales (I) et (Ia) suivant la revendication 1, dans lesquelles
R¹ est un groupe phényle qui est éventuellement substitué jusqu'à 2 fois identiques ou différentes par du fluor, du chlore, du brome, de l'iode, un radical méthyle ou nitro,
R² est un groupe -XR¹², dans lequel X représente l'oxygène et R¹² est un groupe alkyle linéaire ou ramifié ayant jusqu'à 4 atomes de carbone,
R³ représente un groupe méthyle, éthyle ou cyclopropyle,
ou bien
R² et R³ forment ensemble un reste de formule 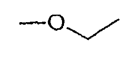
R⁴ représente l'hydrogène ou un groupe acétyle,
et
R⁵ est un groupe pyridyle qui est substitué jusqu'à deux fois identiques ou différentes par du fluor ou du chlore,
et leurs sels.

5. Composés de formules générales (I) et (Ia) suivant l'une quelconque des revendications 1 à 4, formules dans lesquelles R⁵ représente un groupe 2-pyridyle qui est substitué par 1 ou 2 atomes de fluor.

6. Composés suivant la revendication 1, de structures suivantes

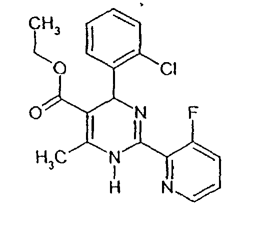

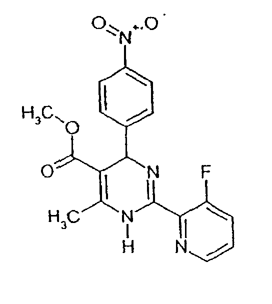

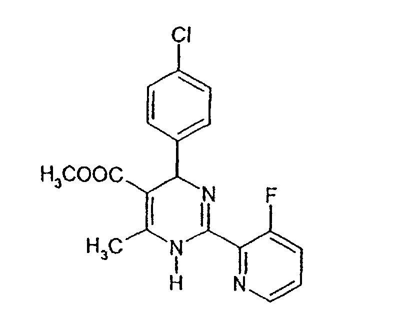

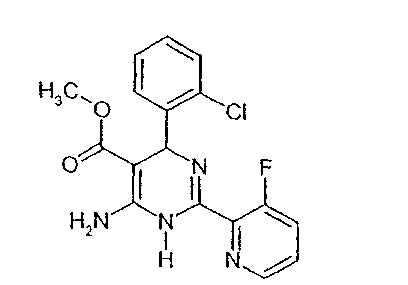

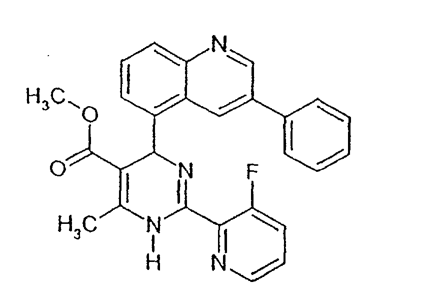

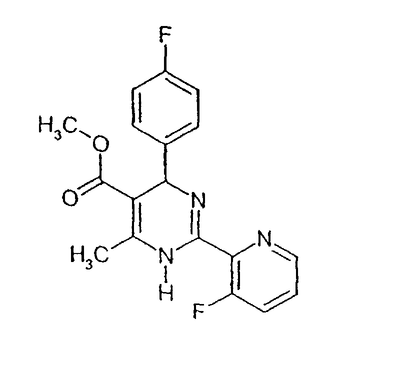

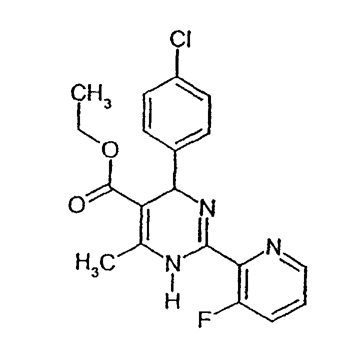

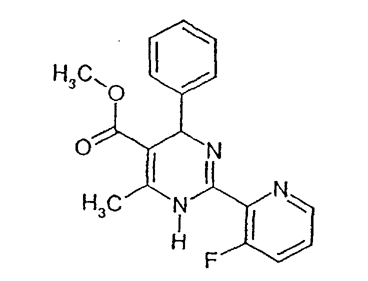

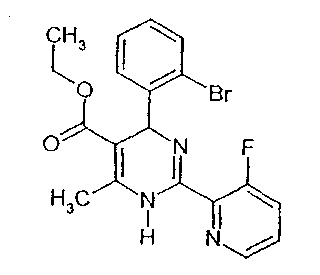

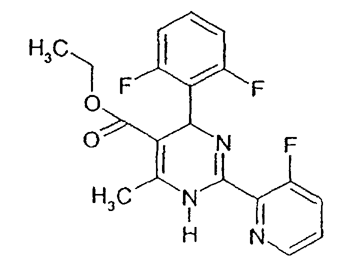

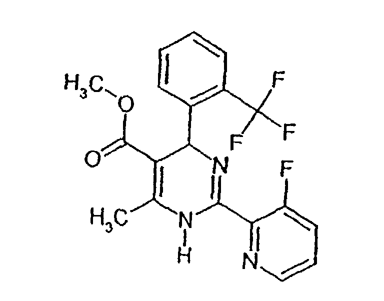

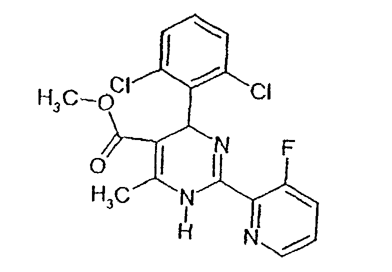

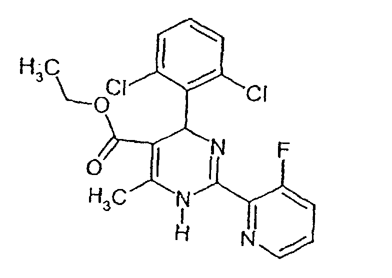

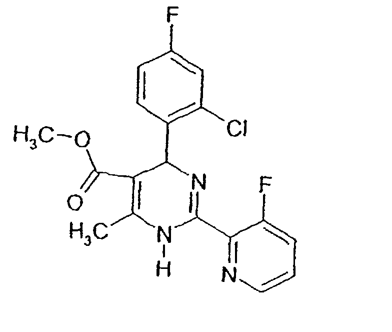

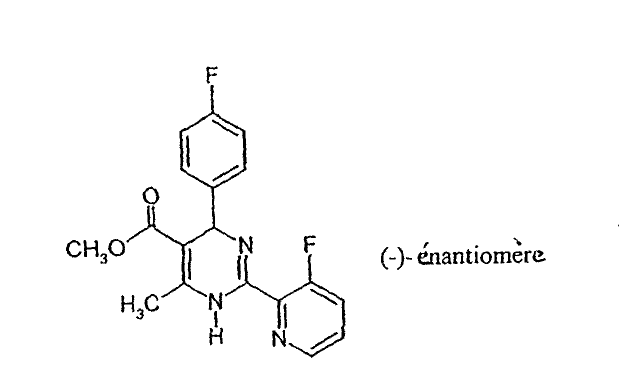

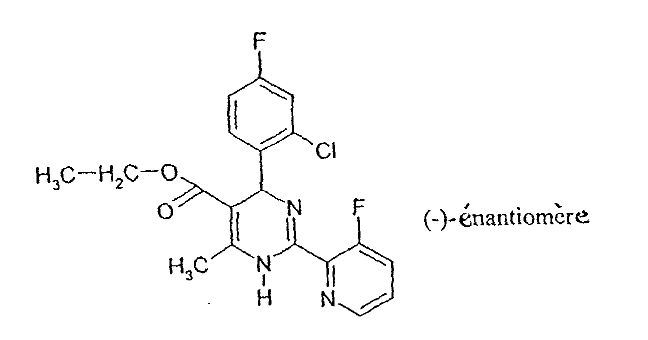

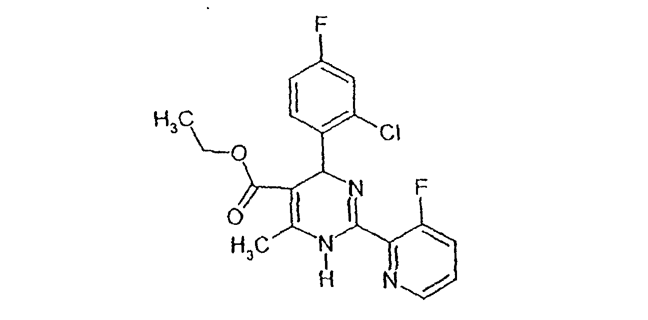

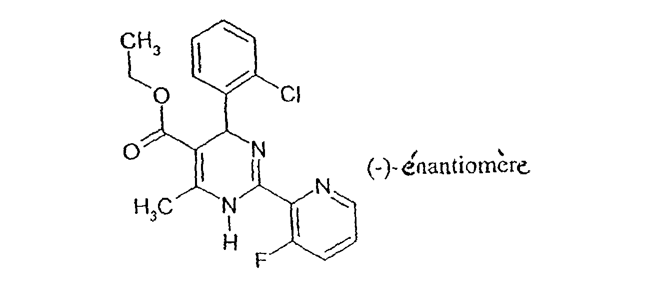

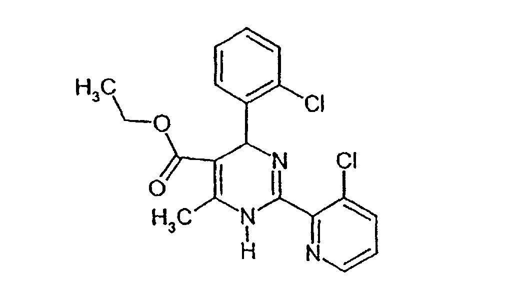

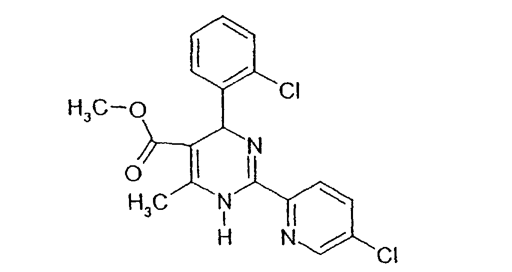

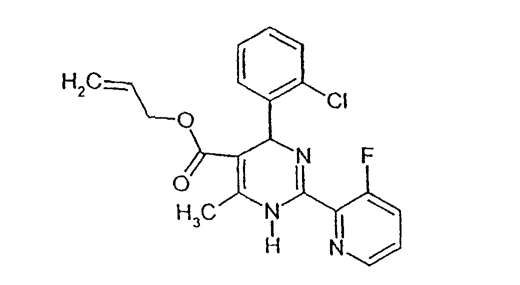

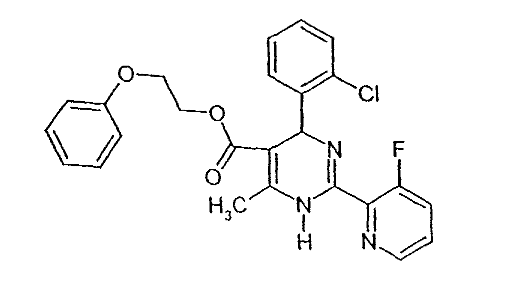

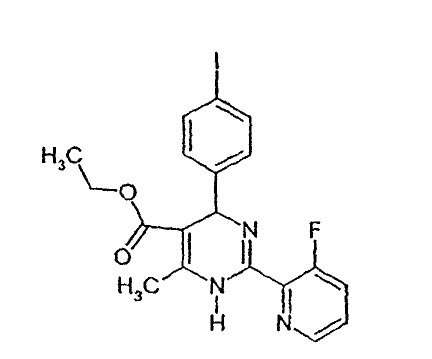

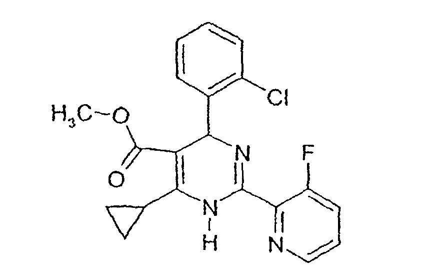

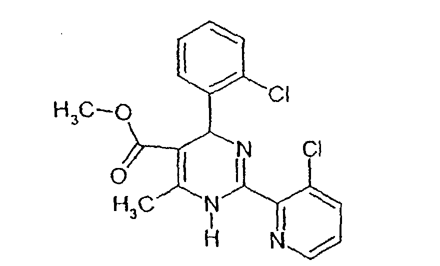

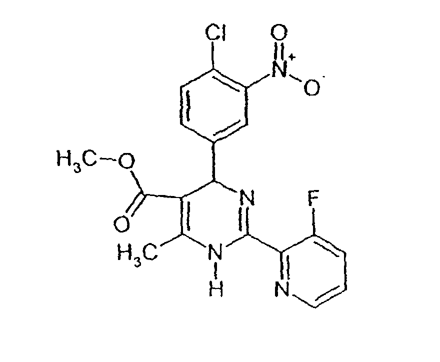

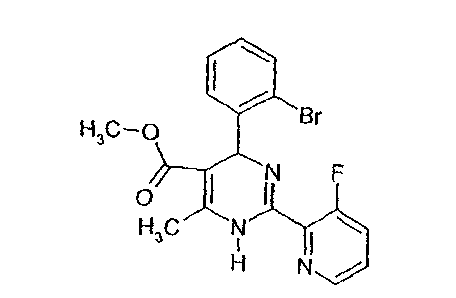

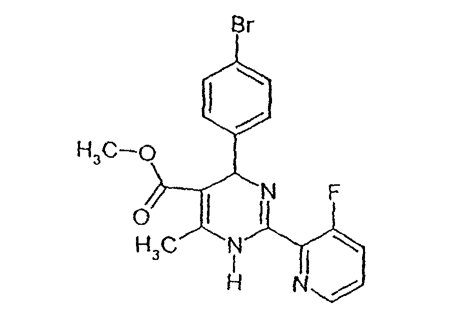

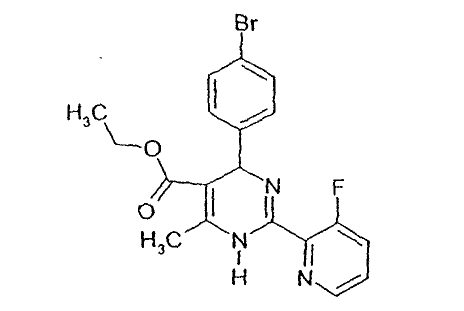

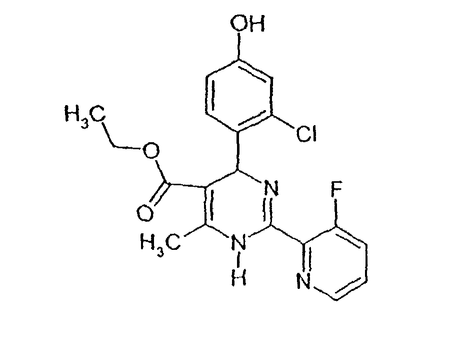

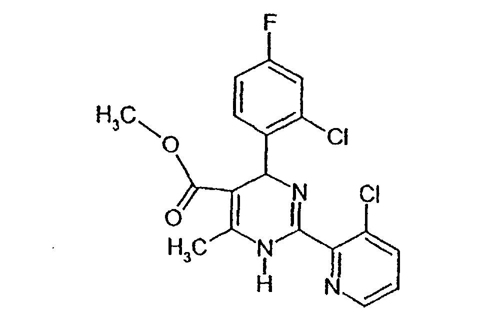

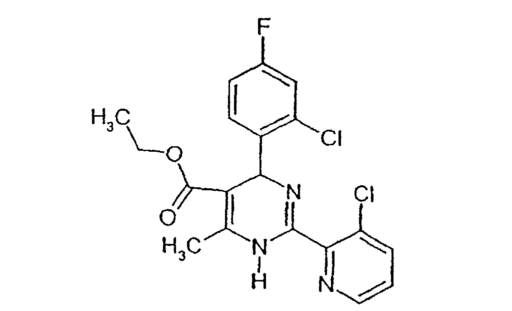

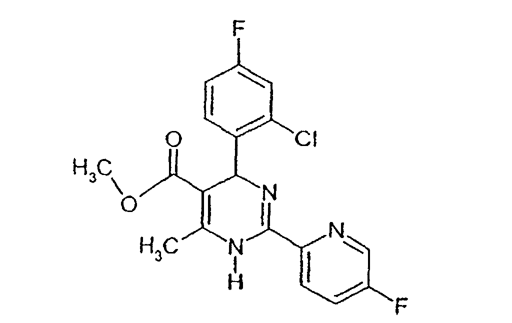

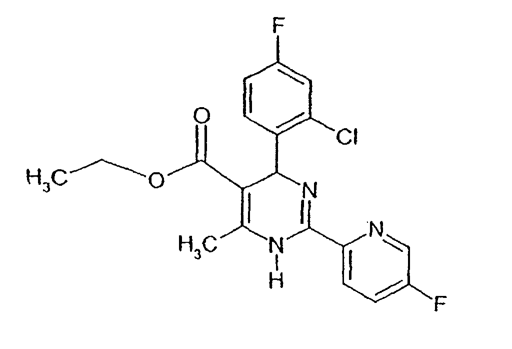

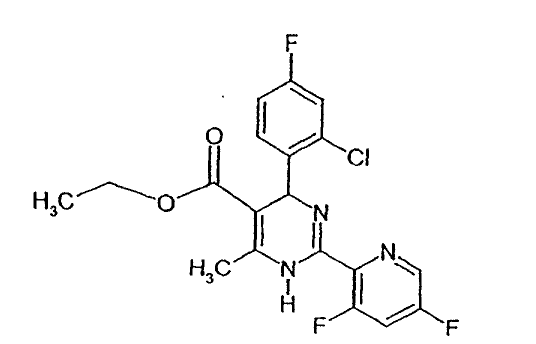

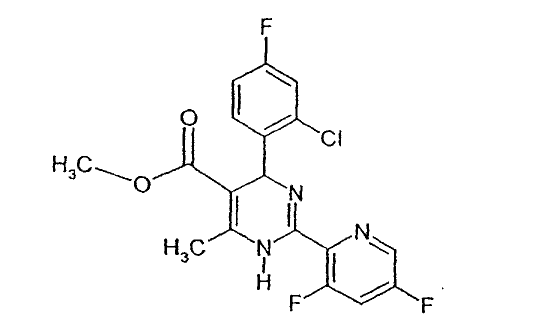

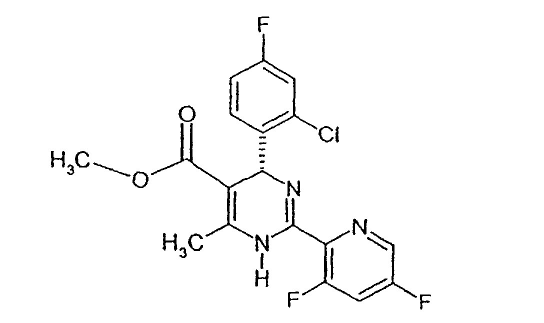

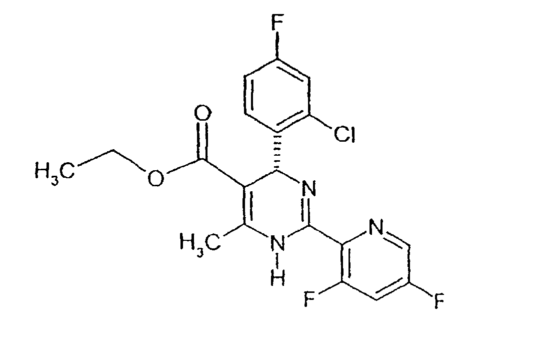

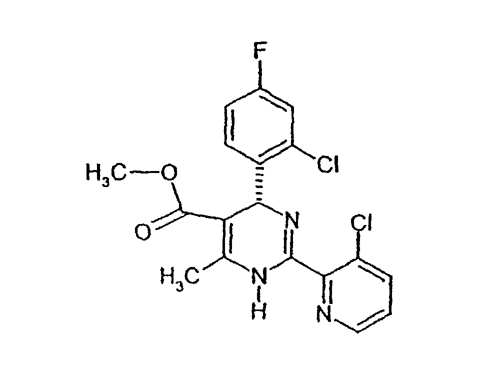

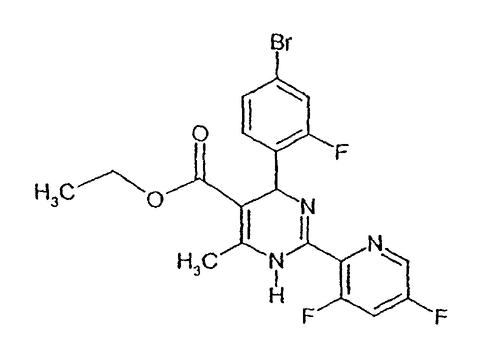

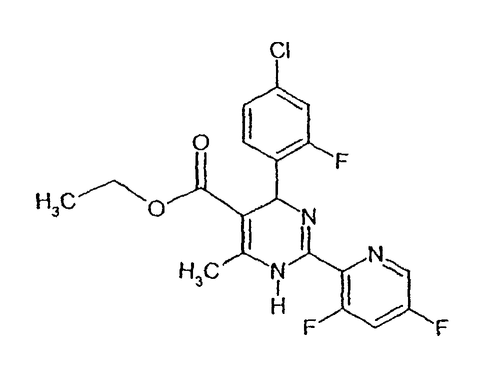

7. Composés suivant la revendication 1, de structures suivantes : 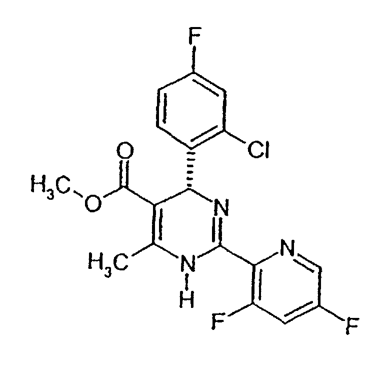 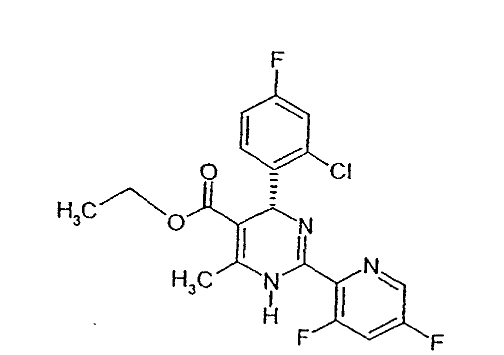 ou des sels de ces composés.

8. Procédé de production des composés suivant les revendications 1 à 7, **caractérisé en ce que** :
[A] on fait réagir des aldéhydes de formule générale (II)
R¹-CHO (II)
dans laquelle
R¹ a la définition indiquée ci-dessus,
avec des amidines ou leurs chlorhydrates de formule (III) 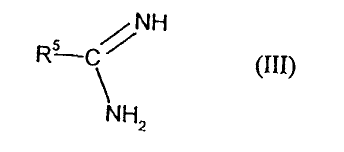 dans laquelle
R⁵ a la définition indiquée ci-dessus,
et des composés de formule générale (IV)
R³-CO-CH₂-CO-R² (IV)
dans laquelle
R² et R³ ont la définition indiquée ci-dessus,
le cas échéant en présence de solvants organiques inertes avec ou sans addition de bases ou d'acides,
ou bien
[B] on fait réagir des composés de formule générale (V) 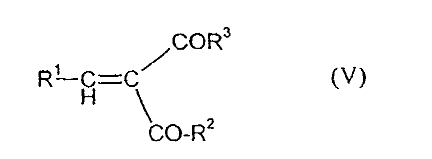 dans laquelle
R¹, R² et R³ ont la définition indiquée ci-dessus,
avec des amidines de formule générale (III) 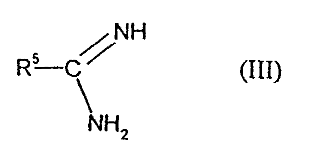 dans laquelle
R⁵ a la définition indiquée ci-dessus,
le cas échéant en présence de solvants organiques inertes, à des températures comprises entre 20°C et 150°C, avec ou sans addition de bases ou d'acides,
ou bien
[C] on fait réagir des aldéhydes de formule générale (II)
R¹-CHO (II)
dans laquelle
R¹ a la définition indiquée ci-dessus,
avec des composés de formule générale (VI) 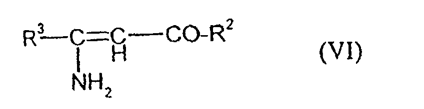 dans laquelle
R² et R³ ont la définition indiquée ci-dessus,
et des amidines de formule générale (III) comme décrit ci-dessus,
ou bien
[D] on fait réagir des aldéhydes de formule générale (II) avec des composés de formule générale (IV) et un imino-éther de formule générale (VII) 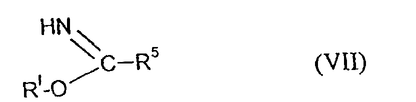 formule dans laquelle
R⁵ a la définition indiquée ci-dessus,
et
R¹ est un groupe alkyle en C₁ à C₄,
en présence de sels d'ammonium.

9. Composé de formule : 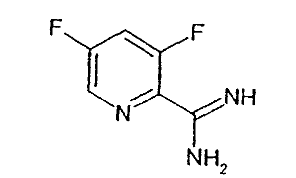 ainsi que des sels de ce composé.

10. Composé de formule

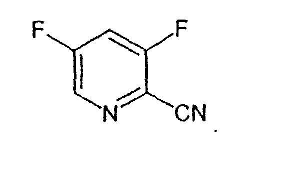

11. Médicaments contenant au moins un composé de formule générale (I) ou (Ia) suivant l'une des revendications 1 à 7 et contenant éventuellement d'autres substances pharmaceutiques actives.

12. Procédé de préparation de médicaments, **caractérisé en ce qu'**on fait prendre une forme d'application appropriée à au moins un composé de formule générale (I) ou (Ia) suivant l'une des revendications 1 à 7, en utilisant éventuellement des substances auxiliaires et des supports classiques.

13. Composés de formule générale (I) ou (Ia) suivant l'une des revendications 1 à 7, destinés à être utilisés comme médicament.

14. Utilisation de composés de formule générale (I) ou (Ia) suivant l'une des revendications 1 à 7 pour la préparation d'un médicament.

15. Utilisation de composés de formule générale (I) ou (Ia) suivant l'une des revendications 1 à 7 pour la préparation d'un médicament destiné au traitement de maladies virales aiguës ou chroniques.

16. Utilisation de composés de formule générale (I) ou (Ia) suivant l'une des revendications 1 à 7 pour la préparation d'un médicament destiné au traitement d'infections aiguës ou chroniques du type de l'hépatite B.
